# EUROPEAN PATENT APPLICATION

(11) **EP 3 686 182 A1**
(43) Date of publication of application: **29.07.2020**
(21) Application number: 18859459.2
(22) Date of filing: 21.09.2018
(51) Int. Cl.: C07C 229/12, A61K 31/265, A61K 31/27, A61K 31/352, A61K 31/36, A61K 31/381, A61K 31/453, A61P 43/00, C07C 271/52, C07C 271/54, C07D 307/92, C07D 311/92, C07D 317/64, C07D 405/04, C07D 495/04

(54) **CHEMICALLY ACTIVATED WATER-SOLUBLE PRODRUG**

(30) Priority: 22.09.2017 JP 2017182725
(71) Applicant: Sumitomo Dainippon Pharma Co., Ltd., Osaka-shi, Osaka 541-0045 (JP)
(72) Inventor: KAMIOKA, Seiji, Osaka-shi Osaka 554-0022 (JP); SAWAYAMA, Yusuke, Osaka-shi Osaka 554-0022 (JP); BAN, Hitoshi, Osaka-shi Osaka 554-0022 (JP); TAKANASHI, Yosuke, Osaka-shi Osaka 554-0022 (JP)
(74) Representative: Carridge, Andrew Edward
(86) International application number: PCT/JP2018/035022
(87) International publication number: WO 2019/059344

(57) **Abstract**

The present invention addresses the problem of providing a novel chemically activated water-soluble prodrug. The present invention provides a compound represented by formula (1), or a pharmacologically acceptable salt thereof (in the formula, A represents A-0; X¹ and X² are the same as or different from each other and each independently represent a hydroxyl group or -O-C(=O)-Y-(C(R^{1A})(R^{1B}))n-NH-R², where X¹ and X² are not simultaneously hydroxyl groups, n is 2, 3, or 4, Y represents an oxygen atom or -NR⁴, R^{1A} and R^{1B} are the same as or different from each other and each independently represent a hydrogen atom, etc., and R² represents a hydrogen atom, etc.; and R^{a} to R^{d} are optionally present, are the same as or different from each other, and each independently represent a hydrogen atom, etc.).

## Description

### [Technical Field]

The present invention relates to a novel chemically activated water-soluble prodrug, a pharmaceutically acceptable salt thereof, or a hydrate or solvate thereof. The present invention also relates to a pharmaceutical composition comprising them, or a solution-state formulation comprising them in an aqueous solution.

### [Background Art]

Aromatic derivatives such as polyphenols, ortho-quinones, and para-quinones have a wide range of pharmacological actions, and are used in clinical settings as agents, such as anti-malignant tumor agents, metabolic cardiotonic agents, thromboxane A₂ receptor antagonists, and hemostasis mechanism activating vitamin K₂. However, the series of derivatives had a problem in solubility such that the derivatives had poor absorbability through oral administration, and use in parenteral administration was limited in some cases due to the high crystallizability and high lipid solubility thereof. To allow such lipid-soluble agents to dissolve into water, studies on water-soluble prodrugs have been reported.
(1) Patent Literature 1 describes the following compounds as a water-soluble prodrug with a soluble side chain introduced to a phenolic hydroxyl group of a reduced vitamin K derivative via an ester linkage. 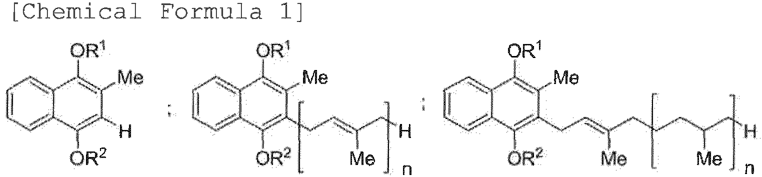 wherein R¹ and R² are the same or different, each independently a hydrogen atom, glycine, N-acylglycine, or the like, wherein at least one of R¹ and R² is a carboxylic acid group residue of glycine or a succinic acid residue, or n is an integer from 1 to 14.
(2) Patent Literature 2 describes the following compound as a water-soluble prodrug with a soluble side chain introduced into a phenolic hydroxyl group of 5-(2,4-dihydroxy-5-isopropylphenyl)-N-ethyl-4-(5-methyl-1,2,4-oxazol-3-yl)isoxazole-3-carboxamide via a phosphate group or an ester linkage. 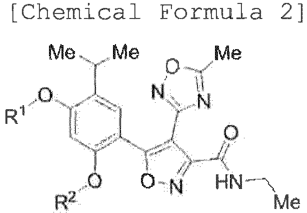 wherein R¹ and R² are the same or different, each independently PO(OH)₂ or PO(O⁻)₂(M)ₙ, wherein if one of R¹ and R² is a hydrogen atom, the other one of R¹ and R² is C(O)CH(NH₂) (CH₂)₄NH₂·2HCl, PO(OH)₂, or PO(O⁻)₂(M)ₙ, M is Na⁺, K⁺, Mg²⁺, or Ca²⁺, and n is 1 or 2.
(3) Patent Literature 3 describes the following compound as a transdermally administered prodrug of cannabidiol with high hydrophobicity. 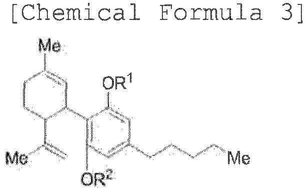 wherein R¹ and R² are the same or different, each independently a hydrogen atom, or oxidized ester, oxa-ester, pegylated ester, hydroxylated ester, alkyl ester, amino ester, alkyl amino acid ester, dialkyl amino acid ester, carbonate, alkyl carbonate, carbamate, alkyl carbamate, amino carbamate, alkyl amino carbamate, or dialkyl amino carbamate, wherein R¹ and R² are not simultaneously a hydrogen atom.
(4) Patent Literature 4 describes the following compound as a water-soluble prodrug with a soluble side chain introduced, via an ester group, into a tertiary hydroxyl group of camptothecins, which is a lipid-soluble agent. The compound is reported to have excellent stability as an intravenously administered agent. 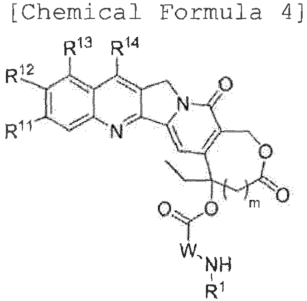 wherein R¹ is a hydrogen atom or an alkyl group with 1 to 6 carbons, W is a divalent group comprising a tertiary amino group or a divalent group comprising a sulfonyl group, m is 0 or 1, R¹¹ is a hydrogen atom, a halogen atom, or an alkyl group with 1 to 6 carbons, R¹² is a hydrogen atom, a halogen atom, a hydroxyl group or an alkyl group with 1 to 6 carbons, and R¹³ is a hydrogen atom, an amino group, a nitro group, or a (dimethylamino)methyl group, R¹⁴ is a hydrogen atom, an alkyl group with 1 to 6 carbons, a (4-methylpiperazinyl)methyl group, or a (tert-butoxyimino)methyl group, wherein R¹³ and R¹⁴, and R¹¹ and R¹² may each form a 5-membered ring or a 6-membered ring by binding to each other.

Patent Literature 5 describes the following compound as a water-soluble prodrug with a soluble side chain introduced into a phenolic hydroxyl group of reduced 2-acetylnaphtho[2,3-b]furan-4,9-dione via a carbamate group, a carbonate group, an ester group, or the like. 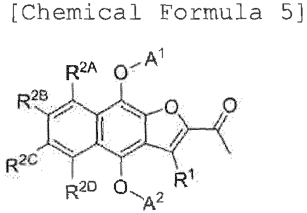 wherein A¹ and A² are the same or different, each independently -C(=O)B, -C(=O)CR^{3A}R^{3B}B, -CO₂B, -CONR^{3C}B, a hydrogen atom, or the like, wherein A¹ and A² are not simultaneously a hydrogen atom (wherein B is an optionally substituted 3- to 12-membered monocyclic or polycyclic heterocyclic group, an optionally substituted 3- to 12-membered cyclic amino group, or a group represented by the following formula: 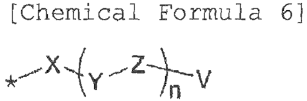
(wherein * represents the point of attachment, X is a single bond, alkylene, or the like, Y is a single bond, an oxygen atom, or the like, Z is a single bond, alkylene, or the like, n is 0, 1, or 2, V is -NHR⁵ or the like, and R⁵ is a hydrogen atom, an alkyl group, or the like),
wherein an optionally substituted 3- to 12-membered monocyclic or polycyclic heterocyclic group and an optionally substituted 3- to 12-membered cyclic amino group have at least one or more secondary nitrogen atoms in a ring, and R^{3A}, R^{3B}, and R^{3C} are the same or different, each independently a hydrogen atom, an alkyl group, or the like); R¹ is a hydrogen atom or the like, and R^{2A}, R^{2B}, R^{2C}, and R^{2D} are the same or different, each independently a hydrogen atom or the like.

Since the water-soluble prodrugs described in Patent Literatures 1, 2, and 3 are converted to activated forms by enzymatic conversion, there is an interspecies or individual difference in the conversion to an activated form, such that there is a risk of a problem in the drug concentration prediction in clinical settings (Non Patent Literature 1). On the other hand, the water-soluble prodrug described in Patent Literature 4 produces an active form by chemical decomposition under a neutral condition by having a soluble side chain comprising an amine structure linked by an ester group with a tertiary hydroxyl group of the active form. Accordingly, the effect of an interspecies or individual difference is reduced upon conversion to an activated form (Non Patent Literature 2). The series of derivatives described in Patent Literature 5 has a reduced effect of an interspecies or individual difference because an active form is produced through chemical decomposition by having a soluble side chain comprising an amine structure linked to a carbamate group, a carbonate group, or an ester group. However, a water-soluble chemically activated prodrug has been reported for a tricyclic para-quinone derivative naphtho[2,3-b]furan-4,9-dione, but not for other polyphenols, ortho-quinones, and para-quinones.

### [Citation List]

### [Patent Literature]

[PTL 1] International Publication No. WO 2006/080463
[PTL 2] International Publication No. WO 2013/015661
[PTL 3] International Publication No. WO 2009/018389
[PTL 4] International Publication No. WO 2005/097803
[PTL 5] International Publication No. WO 2016/157052

### [Non Patent Literature]

[NPL1] Kumpulainen et al. Nature Reviews Drug Discovery 7(3): 255-270. (2008)
[NPL 2] Anthoney et al. BMC Cancer 12: 536. (2012)

### [Summary of Invention]

### [Solution to Problem]

The present invention solves the problem associated with lipid-soluble agents such as polyphenols, ortho-quinones, and para-quinones having a wide range of pharmacological actions, but having poor absorbability through oral administration and limited use in parenteral administration due to the high crystallizability and high lipid solubility thereof. Since an orally administered prodrug is generally affected by the first pass effect in the small intestine or the liver that is the cause of interspecies differences after administration, there is a concern for the problem of interspecies and individual differences. Further, since a parenterally administered prodrug can be intravenously administered, such a prodrug is not affected by the first past effect in the small intestine or the liver. However, there is a concern for the problem of interspecies and individual differences because such a prodrug is metabolized *in vivo* by esterase and converted to an activated form.

The present invention provides a prodrug compound with low interspecies and individual differences by adding a step of conversion into an activated form by chemical conversion in addition to the normal enzymatic conversion.

The present invention relates to a water-soluble prodrug of polyphenols, ortho-quinones, para-quinones, and the like that can be orally or intravenously administered, which has excellent stability as an agent and is converted into an activated form *in vivo* by chemical conversion. When the prodrug of the invention is intravenously administered, the prodrug is not subjected to the first pass effect in the small intestine or liver, which causes interspecies or individual differences, and is pH-dependently converted into an activated form *in vivo.*

The inventors intensively studied to complete the invention by finding that a prodrug represented by formula (1) with a soluble side chain having an amine structure linked to an activated form via a carbamate group or a carbonate group has excellent stability as an agent, exhibits high water solubility that is suitable for intravenous administration, and is converted to an active form through a route including chemical conversion.

Specifically, the present invention is the following.
[Item 1]
   A compound represented by formula (1) 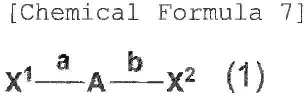 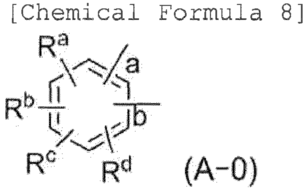 or a pharmaceutically acceptable salt thereof, wherein:
   A represents A-0, a and b in the compound of formula (1) are symbols indicating that two bonds binding to A correspond to two bonds in A-0, respectively;
   X¹, X², R^{a}, R^{b}, R^{c}, and R^{d} are present on any carbon atom in a random order on the aromatic ring of A-0;
   X¹ and X² are the same or different, each independently a hydroxyl group or -O-C(=O)-Y-(C(R^{1A})(R^{1B}))ₙ-NH-R², wherein X¹ and X² are not simultaneously a hydroxyl group;
   n is the same or different, each independently 2, 3, or 4;
   Y is an oxygen atom or NR⁴;
   R^{1A} and R^{1B} are the same or different, each independently a hydrogen atom, -CO₂R⁵, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
   R² is a hydrogen atom, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
   R^{a}, R^{b}, R^{c}, and R^{d} are the same or different, each independently a hydrogen atom, a hydroxyl group, a halogen atom, a cyano group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₃₋₁₀ cycloalkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 3- to 12-membered monocyclic or polycyclic heterocyclylcarbonyl group, a carboxyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 3- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 3-to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 3- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group, or
   2 to 4 of R^{a}, R^{b}, R^{c}, and R^{d}, when adjacent to one another on the aromatic ring, together with 2 to 4 carbon atoms on the aromatic ring to which they are attached, may form 1 or 2 optionally substituted C₃₋₁₈ monocyclic or polycyclic hydrocarbon rings or optionally substituted 3- to 18-membered monocyclic or polycyclic heterocycles, wherein the C₃₋₁₈ monocyclic or polycyclic hydrocarbon ring or the 3- to 18-membered monocyclic or polycyclic heterocycle is fused to the aromatic ring to form a fused ring;
   the C₃₋₁₈ monocyclic or polycyclic hydrocarbon ring or the 3- to 18-membered monocyclic or polycyclic heterocycle can have one or more substituents R^{e}, the one or more R^{e} being, each independently, a hydrogen atom, a hydroxyl group, a halogen atom, a cyano group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₃₋₁₀ cycloalkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 3- to 12-membered monocyclic or polycyclic heterocyclylcarbonyl group, a carboxyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 3- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 3-to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 3- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group;
   R⁴ is a hydrogen atom, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
   R⁵ and R⁶ are the same or different, each independently a hydrogen atom, or a C₁₋₆ alkyl group optionally substituted with 1 to 2 groups selected from the group consisting of a fluorine atom and a carboxyl group, or R⁵ and R⁶, together with the nitrogen atom to which they are attached, may form a 4- to 8-membered cyclic amino group;
   wherein A is not 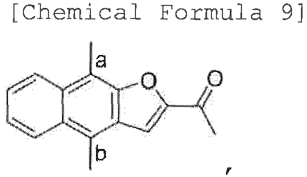 with the proviso that the compound is not the following compounds:
      3-hydroxy-2-[(1R,6R)-3-methyl-6-(prop-1-en-2-yl)cyclohex-2-en-1-yl]-5-pentylphenyl (4-aminobutyl)carbamate (1);
      2-[(1R,6R)-3-methyl-6-(prop-1-en-2-yl)cyclohex-2-en-1-yl]-5-pentylbenzene-1,3-diyl bis[(4-aminobutyl)carbamate] (2);
      (3R)-8-hydroxy-3-[(2R,3S)-3-hydroxy-4-{(2R,4R,6S)-6-[(S)-{[(2S,3S)-2-hydroxy-3-methoxy-5-methylhex-5-enoyl]amino}(methoxy)methyl]-3,3,4-trimethyltetrahydro-2H-pyran-2-yl}butan-2-yl]-5-methyl-1-methylidene-3,4-dihydro-1H-isochromen-6-yl methyl[2-(methylamino)ethyl]carbamate (3);
      3-hydroxy-2-methylphenyl (2-aminoethyl)carbamate (4);
      3-hydroxy-2-methylphenyl (1-aminopropan-2-yl)carbamate (5);
      3-hydroxyphenyl (2-aminoethyl)carbamate (6);
      3-hydroxy-2-methylphenyl (2-aminoethyl)methylcarbamate (7);
      3-hydroxyphenyl (1-aminopropan-2-yl)carbamate (8);
      3-hydroxyphenyl (2-amino-2-methylpropyl)carbamate (9);
      3-hydroxyphenyl [2-(methylamino)ethyl]carbamate (10);
      3-hydroxyphenyl (1-amino-2-methylpropan-2-yl)carbamate (11);
      3-hydroxyphenyl (3-aminopropyl)carbamate (12);
      3-hydroxy-2-methylphenyl (2-aminopropyl)carbamate (13);
      3-hydroxyphenyl (2-aminopropyl)carbamate (14);
      3-hydroxy-2-methylphenyl [2-(methylamino)ethyl]carbamate (15) ;
      3-hydroxyphenyl (2-aminoethyl)methylcarbamate (16); and 3-hydroxy-2-methylphenyl (3-aminopropyl)carbamate (17).
[Item 2]
   The compound or a pharmaceutically acceptable salt thereof according to item 1, wherein
   A represents A-1, A-2, A-3, A-4, A-5, A-6, A-7, A-8, A-9, A-10, A-11, A-12, A-13, A-14, A-15, A-16, or A-17: 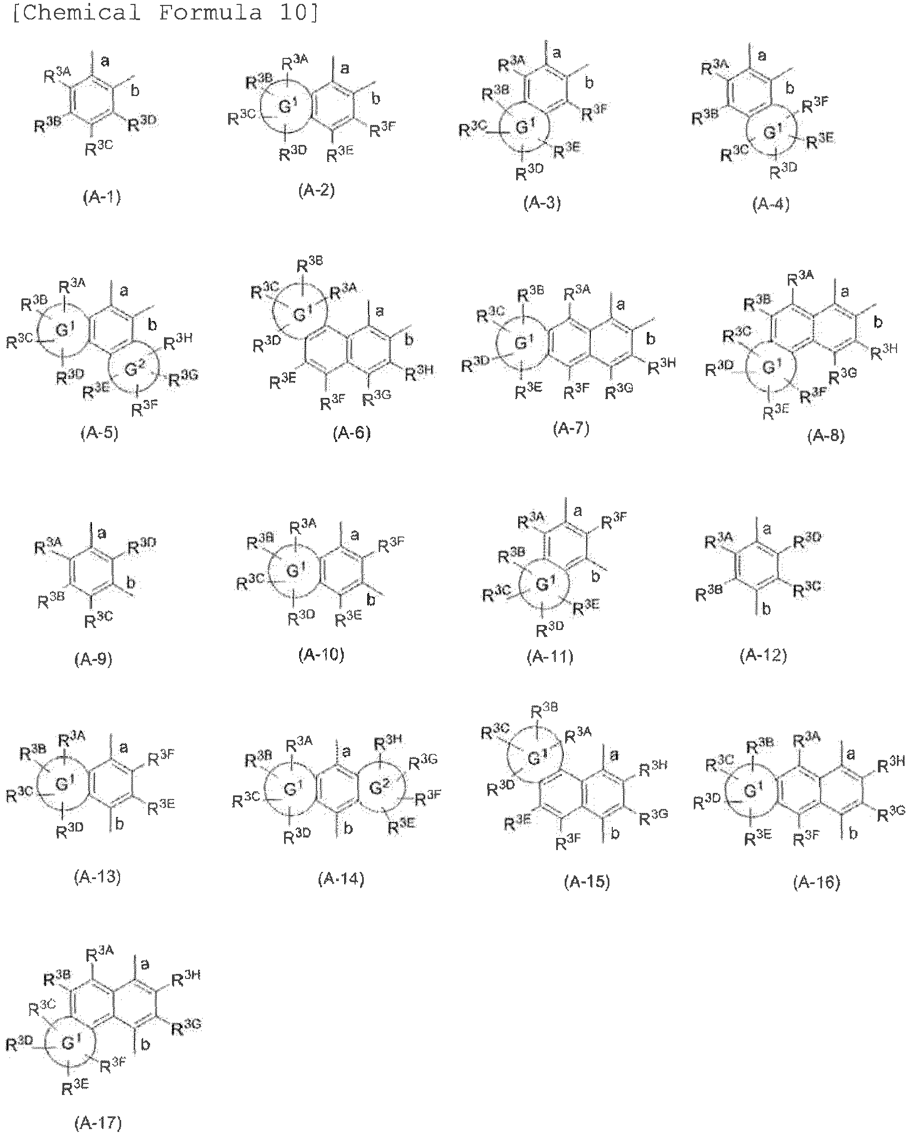
   a and b in the compound of formula (1) are symbols indicating that two bonds binding to A correspond to two bonds in A-1 to A-17, respectively;
   rings G¹ and G² are the same or different, each independently a benzene ring, or a 5- or 6-membered aromatic ring comprising, as a constituent atom, 1 to 3 heteroatoms consisting of O, S and N; and
   R^{3A}, R^{3B}, R^{3C}, R^{3D}, R^{3E}, R^{3F}, R^{3G}, and R^{3H} are null, or the same or different, each independently a hydrogen atom, a hydroxyl group, a fluorine atom, a chlorine atom, a cyano group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₃₋₁₀ cycloalkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 5- to 12-membered monocyclic or polycyclic heterocyclylcarbonyl group, a carboxyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 5- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 5-to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 5- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form an optionally substituted C₃₋₁₀ cycloalkane or an optionally substituted 3-to 12-membered monocyclic or polycyclic heterocycle, wherein the C₃₋₁₀ cycloalkane or the 3- to 12-membered monocyclic or polycyclic heterocycle is fused to the aromatic ring to form a fused ring.
[Item 3]
   The compound or a pharmaceutically acceptable salt thereof according to item 1 or 2, wherein an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 5- to 12-membered monocyclic or polycyclic heterocyclylcarbonyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 5- to 12-membered monocyclic or polycyclic heterocyclyloxycarbonyl group, an optionally substituted aminocarbonyl group, an optionally substituted C_{1- 20} alkylthio group, an optionally substituted C₃₋₁₀ cycloalkylthio group, an optionally substituted C₆₋₁₀ arylthio group, an optionally substituted 5- to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 5- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group in R^{3A}, R^{3B}, R^{3C}, R^{3D}, R^{3E}, R^{3F}, R^{3G}, and R^{3H} is each independently a group optionally substituted with the same or different 1 to 5 substituents selected from the group consisting of:
   (1) a fluorine atom;
   (2) a chlorine atom;
   (3) a hydroxyl group;
   (4) a carboxyl group;
   (5) a sulfinate group;
   (6) a sulfonate group;
   (7) a phosphate group;
   (8) an optionally substituted C₁₋₁₀ alkyl group;
   (9) an optionally substituted C₃₋₁₀ cycloalkyl group;
   (10) an optionally substituted C₆₋₁₀ aryl group;
   (11) an optionally substituted 5- to 10-membered heteroaryl group;
   (12) an optionally substituted C₁₋₁₀ alkoxy group;
   (13) an optionally substituted C₃₋₁₀ cycloalkoxy group;
   (14) an optionally substituted C₁₋₁₀ alkoxycarbonyl group;
   (15) an optionally substituted C₁₋₁₀ alkylcarbonyl group;
   (16) an optionally substituted 3- to 12-membered monocyclic or polycyclic heterocyclic group;
   (17) an optionally substituted 3- to 12-membered cyclic amino group;
   (18) -NR⁷R⁸;
   (19) -CO₂R⁷;
   (20) a guanidine group;
   (21) -CONR⁷R⁸;
   (22) -SO₂R⁷;
   (23) -SO₂NR⁷R⁸;
   (24) a cyano group;
   (25) -OCO₂R⁷;
   (26) -OCONR⁷R⁸;
   (27) -NR⁷CO₂R⁸; and
   (28) a triphenylphosphonium cation,
   wherein the substituents in (8), (9), (10), (11), (12), (13), (14), (15), (16), and (17) are each independently a group optionally substituted with the same or different 1 to 5 substituents selected from the group consisting of:
   (a) a fluorine atom;
   (b) a chlorine atom;
   (c) a hydroxyl group;
   (d) a C₁₋₆ alkyl group;
   (e) a C₁₋₆ alkoxy group;
   (f) a cyano group;
   (g) a carboxyl group;
   (h) a sulfinate group;
   (i) a sulfonate group;
   (j) a phosphate group;
   (k) a C₁₋₆ alkoxycarbonyl group;
   (l) a C₁₋₆ alkylcarbonyl group;
   (m) -NR⁷R⁸;
   (n) -CO₂R⁷;
   (o) a guanidine group;
   (p) -CONR⁷R⁸;
   (q) -SO₂R⁷;
   (r) -SO₂NR⁷R⁸;
   (s) a C₆₋₁₀ aryl group;
   (t) a 5- to 10-membered heteroaryl group;
   (u) a 3- to 12-membered cyclic amino group optionally substituted with 1 to 3 C₁₋₆ alkyl groups; and
   (v) a 3- to 12-membered monocyclic or polycyclic heterocyclic group optionally substituted with 1 to 3 C₁₋₆ alkyl groups,
   wherein R⁷ and R⁸ are the same or different, each independently a hydrogen atom, or a C₁₋₆ alkyl group optionally substituted with 1 to 2 groups selected from the group consisting of a fluorine atom and a carboxyl group, or R⁷ and R⁸, together with the nitrogen atom to which they are attached, may form a 4- to 8-membered cyclic amino group.
[Item 4]
   The compound or a pharmaceutically acceptable salt thereof according to any one of items 1 to 3, wherein
   R^{3A}, R^{3B}, R^{3C}, R^{3D}, R^{3E}, R^{3F}, R^{3G}, and R^{3H} are null, or the same or different, each independently a hydrogen atom, a hydroxyl group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₁₋₂₀ alkoxy group, an optionally substituted C₁₋₂₀ alkylcarbonyloxy group, an optionally substituted 3- to 12-membered monocyclic or polycyclic heterocyclic group, an optionally substituted C₆₋₁₀ aryl group, an optionally substituted C₁₋₂₀ alkylcarbonyl group, or a carboxyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form an optionally substituted C₃₋₁₀ cycloalkane or an optionally substituted 3-to 12-membered monocyclic or polycyclic heterocycle, wherein the C₃₋₁₀ cycloalkane or the 3- to 12-membered monocyclic or polycyclic heterocycle is fused to the aromatic ring to form a fused ring.
[Item 5]
   The compound or a pharmaceutically acceptable salt thereof according to any one of items 1 to 4, wherein an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₁₋₂₀ alkoxy group, an optionally substituted C₁₋₂₀ alkylcarbonyloxy group, an optionally substituted 3- to 12-membered monocyclic or polycyclic heterocyclic group, an optionally substituted C₆₋₁₀ aryl group, or a C₁₋₂₀ alkylcarbonyl group in R^{3A}, R^{3B}, R^{3C}, R^{3D}, R^{3E}, R^{3F}, R^{3G}, and R^{3H} is each independently a group optionally substituted with the same or different 1 to 2 substituents selected from the group consisting of:
   (1) a fluorine atom;
   (2) a chlorine atom;
   (3) a hydroxyl group;
   (4) a carboxyl group;
   (5) an optionally substituted C₁₋₁₀ alkyl group;
   (6) an optionally substituted C₃₋₁₀ cycloalkyl group;
   (7) an optionally substituted C₆₋₁₀ aryl group;
   (8) an optionally substituted 5- to 10-membered heteroaryl group;
   (9) an optionally substituted C₁₋₁₀ alkoxycarbonyl group; and
   (10) a triphenylphosphonium cation,
   wherein the substituents in (5), (6), (7), (8), and (9) are each independently a group optionally substituted with the same or different 1 to 2 substituents selected from the group consisting of:
   (a) a chlorine atom;
   (b) a hydroxyl group;
   (c) a C₁₋₆ alkyl group;
   (d) a carboxyl group; and
   (e) a C₁₋₆ alkoxycarbonyl group.
[Item 6]
   The compound or a pharmaceutically acceptable salt thereof according to any one of items 1 to 5, wherein R^{3A}, R^{3B}, R^{3C}, R^{3D}, R^{3E}, R^{3F}, R^{3G}, and R^{3H} are null, or the same or different, each independently:
   (1) a hydrogen atom;
   (2) a hydroxyl group;
   (3) an optionally substituted 3- to 12-membered cyclic amino group, wherein the cyclic amino group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, and a 5- to 10-membered heteroaryl group;
   (4) an optionally substituted C₁₋₃₀ alkyl group, wherein the alkyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (5) an optionally substituted C₃₋₁₀ cycloalkyl group, wherein the cycloalkyl group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (6) an optionally substituted C₂₋₃₀ alkenyl group, wherein the alkenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
   (7) an optionally substituted C₁₋₂₀ alkoxy group, wherein the alkoxy group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, and a C₁₋₁₀ alkoxycarbonyl group;
   (8) an optionally substituted C₁₋₂₀ alkylcarbonyloxy group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (9) an optionally substituted 3- to 12-membered monocyclic or polycyclic heterocyclic group, wherein the heterocyclic group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (10) an optionally substituted phenyl group, wherein the phenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (11) an optionally substituted C₁₋₂₀ alkylcarbonyl group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation; or
   (12) a carboxyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
   (1') an optionally substituted C₃₋₁₀ cycloalkane, wherein the cycloalkane is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
   (2') an optionally substituted 3- to 12-membered monocyclic or polycyclic heterocycle, wherein the heterocycle is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
   wherein the C₃₋₁₀ cycloalkane or the 3- to 12-membered monocyclic or polycyclic heterocycle is fused to the aromatic ring to form a fused ring.
[Item 7]
   The compound or a pharmaceutically acceptable salt thereof according to any one of items 1 to 6, wherein R^{3A}, R^{3B}, R^{3C}, R^{3D}, R^{3E}, R^{3F}, R^{3G}, and R^{3H} are null, or the same or different, each independently:
   (1) a hydrogen atom;
   (2) a hydroxyl group;
   (3) an optionally substituted C₁₋₃₀ alkyl group, wherein the alkyl group is optionally substituted with 1 to 3 groups selected from the group consisting of a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (4) an optionally substituted C₃₋₁₀ cycloalkyl group, wherein the cycloalkyl group is optionally substituted with 1 to 3 groups selected from the group consisting of a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (5) an optionally substituted C₂₋₃₀ alkenyl group, wherein the alkenyl group is optionally substituted with 1 to 3 groups selected from the group consisting of a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
   (6) a C₁₋₂₀ alkoxy group;
   (7) a C₁₋₂₀ alkylcarbonyloxy group;
   (8) an optionally substituted 3- to 12-membered monocyclic or polycyclic heterocyclic group, wherein the heterocyclic group is optionally substituted with 1 to 3 groups selected from the group consisting of a hydroxyl group, a carboxyl group, a C₁₋₁₀ alkyl group, a C₃₋₁₀ cycloalkyl group, and a C₁₋₆ alkoxycarbonyl group;
   (9) a phenyl group;
   (10) a C₁₋₂₀ alkylcarbonyl group; or
   (11) a carboxyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
   (1') an optionally substituted C₃₋₁₀ cycloalkane, wherein the cycloalkane is optionally substituted with 1 to 3 groups selected from the group consisting of a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
   (2') an optionally substituted 3- to 12-membered monocyclic or polycyclic heterocycle, wherein the heterocycle is optionally substituted with 1 to 3 groups selected from the group consisting of a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
   wherein the C₃₋₁₀ cycloalkane or the 3- to 12-membered monocyclic or polycyclic heterocycle is fused to the aromatic ring to form a fused ring.
[Item 8]
   The compound or a pharmaceutically acceptable salt thereof according to any one of items 1 to 7, wherein R^{3A}, R^{3B}, R^{3C}, R^{3D}, R^{3E}, R^{3F}, R^{3G}, and R^{3H} are null, or the same or different, each independently:
   (1) a hydrogen atom;
   (2) a hydroxyl group;
   (3) an optionally substituted C₁₋₃₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
   (4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
   (5) an optionally substituted C₂₋₃₀ alkenyl group, wherein the alkenyl group is optionally substituted with 1 to 3 groups selected from the group consisting of a carboxyl group and a pyridyl group;
   (6) a C₁₋₁₂ alkoxy group;
   (7) a C₁₋₁₂ alkylcarbonyloxy group;
   (8) a 5- to 8-membered monocyclic or polycyclic heterocyclic group, wherein the heterocyclic group is optionally substituted with 1 to 3 groups selected from the group consisting of a hydroxyl group and a C₁₋₁₀ alkyl group;
   (9) a phenyl group;
   (10) a C₁₋₁₂ alkylcarbonyl group; or
   (11) a carboxyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
   (1') an optionally substituted C₅₋₈ cycloalkane, wherein the cycloalkane is optionally substituted with 1 to 3 groups selected from the group consisting of a C₁₋₆ alkyl group and an optionally chloro-substituted C₆₋₁₀ aryl group; or
   (2') an optionally substituted 5- to 8-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group,
   wherein the C₃₋₁₀ cycloalkane or the 3- to 12-membered monocyclic or polycyclic heterocycle is fused to the aromatic ring to form a fused ring.
[Item 9]
   The compound or a pharmaceutically acceptable salt thereof according to any one of items 2 to 8, wherein A is A-1, A-2, A-3, A-4, A-5, A-6, A-7, A-8, A-9, A-12, A-13, A-14, A-15, A-16, or A-17.
[Item 10]
   The compound or a pharmaceutically acceptable salt thereof according to any one of items 2 to 9, wherein A is A-1, A-2, A-3, A-4, A-9, A-12, A-13, A-14, A-15, A-16, or A-17.
[Item 11]
   The compound or a pharmaceutically acceptable salt thereof according to any one of items 2 to 10, wherein A is A-1, A-2, A-4, A-9, A-12, A-13, or A-14.
[Item 12]
   The compound or a pharmaceutically acceptable salt thereof according to any one of items 1 to 11, wherein R^{1A} and R^{1B} are the same or different, each independently a hydrogen atom, -CO₂R⁵, or a C₁₋₆ alkyl group optionally substituted with 1 to 2 substituents selected from the group consisting of a carboxyl group, -CO₂R⁵, -CONR⁵R⁶, -SO₂R⁵, -SO₂NR⁵R⁶, -OCO₂R⁵, - OCONR⁵R⁶, and -NR⁵CO₂R⁶.
[Item 13]
   The compound or a pharmaceutically acceptable salt thereof according to any one of items 2 to 12, wherein rings G¹ and G² are the same or different, each independently a benzene ring, a furan ring, or a thiophene ring.
[Item 14]
   The compound or a pharmaceutically acceptable salt thereof according to any one of items 1 to 13, wherein R² is a hydrogen atom, or a C₁₋₆ alkyl group optionally substituted with 1 to 2 substituents selected from the group consisting of a carboxyl group, -CO₂R⁵, -CONR⁵R⁶, -SO₂R⁵, -SO₂NR⁵R⁶, -OCO₂R⁵, -OCONR⁵R⁶, and -NR⁵CO₂R⁶.
[Item 15]
   The compound or a pharmaceutically acceptable salt thereof according to any one of items 1 to 14, wherein R⁴ is a hydrogen atom, or a C₁₋₆ alkyl group optionally substituted with 1 to 2 substituents selected from the group consisting of a carboxyl group, a C₁₋₆ alkoxy group, -NR⁵R⁶, -CO₂R⁵, - CONR⁵R⁶, -OCO₂R⁵, -OCONR⁵R⁶, and -NR⁵CO₂R⁶.
[Item 16]
   The compound or a pharmaceutically acceptable salt thereof according to any one of items 1 to 15, wherein R⁴ is a hydrogen atom or an optionally carboxyl-substituted C₁₋₆ alkyl group.
[Item 17]
   The compound or a pharmaceutically acceptable salt thereof according to any one of items 1 to 16, wherein R⁴ is a hydrogen atom or a C₁₋₆ alkyl group.
[Item 18]
   The compound or a pharmaceutically acceptable salt thereof according to any one of items 1 to 17, wherein R⁵ and R⁶ are the same or different, each independently a hydrogen atom, or a C₁₋₆ alkyl group optionally substituted with 1 to 2 carboxyl groups, or R⁵ and R⁶, together with the nitrogen atom to which they are attached, may form a 4- to 8-membered cyclic amino group.
[Item 19]
   The compound or a pharmaceutically acceptable salt thereof according to item 2, wherein
   A is A-1, A-2, A-4, A-9, A-12, A-13, or A-14;
   X¹ and X² are the same or different, each independently a hydroxyl group, or -O-C(=O)-Y-(C(R^{1A})(R^{1B}))ₙ-NH-R², wherein X¹ and X² are not simultaneously a hydroxyl group;
   n is the same or different, each independently 2, 3, or 4;
   Y is an oxygen atom or NR⁴;
   rings G¹ and G² are the same or different, each independently a benzene ring, a furan ring, or a thiophene ring;
   R^{1A} and R^{1B} are the same or different, each independently a hydrogen atom, -CO₂R⁵, or a C₁₋₆ alkyl group optionally substituted with 1 to 2 substituents selected from the group consisting of a carboxyl group, -CO₂R⁵, -CONR⁵R⁶, and -SO₂NR⁵R⁶;
   R² is a hydrogen atom, or a C₁₋₆ alkyl group optionally substituted with 1 to 2 substituents selected from the group consisting of a carboxyl group, -CO₂R⁵, -CONR⁵R⁶, -SO₂R⁵, - SO₂NR⁵R⁶, -OCO₂R⁵, -OCONR⁵R⁶, and -NR⁵CO₂R⁶;
   R^{3A}, R^{3B}, R^{3C}, R^{3D}, R^{3E}, R^{3F}, R^{3G}, and R^{3H} are null, or the same or different, each independently:
   (1) a hydrogen atom,
   (2) a hydroxyl group,
   (3) an optionally substituted C₁₋₂₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
   (4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
   (5) an optionally substituted C₂₋₂₀ alkenyl group, wherein the alkenyl group is optionally substituted with 1 to 3 groups selected from the group consisting of a carboxyl group and a pyridyl group;
   (6) a C₁₋₁₂ alkoxy group;
   (7) a C₁₋₁₂ alkylcarbonyloxy group;
   (8) a 5- to 6-membered monocyclic or polycyclic heterocyclic group, wherein the heterocyclic group is optionally substituted with 1 to 3 groups selected from the group consisting of a hydroxyl group and a C₁₋₁₀ alkyl group;
   (9) a phenyl group;
   (10) a C₁₋₁₂ alkylcarbonyl group; or
   (11) a carboxyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
   (1') an optionally substituted C₅₋₆ cycloalkane, wherein the cycloalkane is optionally substituted with 1 to 3 groups selected from the group consisting of a C₁₋₆ alkyl group and an optionally chloro-substituted C₆₋₁₀ aryl group; or
   (2') an optionally substituted 5- to 6-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group,
   wherein the C₃₋₁₀ cycloalkane or the 3- to 12-membered monocyclic or polycyclic heterocycle is fused to the aromatic ring to form a fused ring;
   R⁴ is a hydrogen atom or a C₁₋₄ alkyl group; and
   R⁵ and R⁶ are the same or different, each independently a hydrogen atom or a C₁₋₆ alkyl group, or R⁵ and R⁶, together with the nitrogen atom to which they are attached, may form a 4- to 8-membered cyclic amino group.
[Item 20]
   The compound or a pharmaceutically acceptable salt thereof according to any one of items 1 to 19, wherein R^{1A} and R^{1B} are the same or different, each independently a hydrogen atom, -CO₂R⁵, or a C₁₋₆ alkyl group optionally substituted with 1 to 2 substituents selected from the group consisting of a carboxyl group and -CO₂R⁵.
[Item 21]
   The compound or a pharmaceutically acceptable salt thereof according to any one of items 1 to 20, wherein R^{1A} and R^{1B} are the same or different, each independently a hydrogen atom, -CO₂R⁵, or a C₁₋₆ alkyl group.
[Item 22]
   The compound or a pharmaceutically acceptable salt thereof according to any one of items 1 to 21, wherein R^{1A} and R^{1B} are hydrogen atoms.
[Item 23]
   The compound or a pharmaceutically acceptable salt thereof according to any one of items 1 to 22, wherein R² is a hydrogen atom, or a C₁₋₆ alkyl group optionally substituted with 1 to 2 substituents selected from the group consisting of a carboxyl group, -CO₂R⁵, -CONR⁵R⁶, and -SO₂NR⁵R⁶.
[Item 24]
   The compound or a pharmaceutically acceptable salt thereof according to any one of items 1 to 23, wherein R² is a hydrogen atom, or a C₁₋₆ alkyl group optionally substituted with 1 to 2 carboxyl groups.
[Item 25]
   The compound or a pharmaceutically acceptable salt thereof according to any one of items 1 to 24, wherein Y is an oxygen atom.
[Item 26]
   The compound or a pharmaceutically acceptable salt thereof according to any one of items 1 to 24, wherein Y is NR⁴.
[Item 27]
   The compound or a pharmaceutically acceptable salt thereof according to any one of items 1 to 26, wherein R⁴ is a C₁₋₄ alkyl group.
[Item 28]
   The compound or a pharmaceutically acceptable salt thereof according to any one of items 1 to 26, wherein R⁴ is a hydrogen atom.
[Item 29]
   The compound or a pharmaceutically acceptable salt thereof according to any one of items 1 to 28, wherein R⁵ and R⁶ are the same or different, each independently a hydrogen atom or a C₁₋₆ alkyl group.
[Item 30]
   The compound or a pharmaceutically acceptable salt thereof according to any one of items 1 to 29, wherein R⁵ and R⁶ are C₁₋₆ alkyl groups.
[Item 31]
   The compound or a pharmaceutically acceptable salt thereof according to any one of items 1 to 30, wherein X¹ and X² are -O-C(=O)-Y-(C(R^{1A})(R^{1B}))ₙ-NH-R².
[Item 32]
   The compound or a pharmaceutically acceptable salt thereof according to any one of items 1 to 30, wherein one of X¹ and X² is -O-C(=O)-Y-(C(R^{1A})(R^{1B}))ₙ-NH-R², and the other is a hydroxyl group.
[Item 33]
   The compound or a pharmaceutically acceptable salt thereof according to any one of items 1 to 32, wherein n is 2 or 3.
[Item 34]
   The compound or a pharmaceutically acceptable salt thereof according to any one of items 1 to 33, wherein n is 2.
[Item 35]
   The compound or a pharmaceutically acceptable salt thereof according to any one of items 1 to 33, wherein n is 3.
[Item 36]
   The compound or a pharmaceutically acceptable salt thereof according to any one of items 2 to 35, wherein
   A is A-13;
   ring G¹ is a benzene ring; and
   R^{3A}, R^{3B}, R^{3C}, R^{3D}, R^{3E}, and R^{3F} are the same or different, each independently:
   (1) a hydrogen atom;
   (2) a hydroxyl group;
   (3) a C₁₋₂₀ alkyl group;
   (4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group; or
   (5) a C₂₋₂₀ alkenyl group.
[Item 37]
   The compound or a pharmaceutically acceptable salt thereof according to any one of items 2 to 35, wherein
   A is A-12; and
   R^{3A}, R^{3B}, R^{3C}, and R^{3D} are the same or different, each independently:
   (1) a hydrogen atom;
   (2) an optionally substituted C₁₋₁₂ alkyl group, wherein the alkyl group is optionally substituted with 1 to 3 groups selected from the group consisting of a hydroxyl group, a carboxyl group, a phenyl group, a pyridyl group, a C₁₋₆ alkoxycarbonyl group, and a triphenylphosphonium cation;
   (3) an optionally substituted C₂₋₂₀ alkenyl group, wherein the alkenyl group is optionally substituted with 1 to 3 groups selected from the group consisting of a carboxyl group and a pyridyl group;
   (4) a C₁₋₆ alkoxy group; or
   (5) a phenyl group.
[Item 38]
   The compound or a pharmaceutically acceptable salt thereof according to any one of items 2 to 35, wherein
   A is A-14;
   rings G¹ and G² are the same or different, each independently a benzene ring, a furan ring, or a thiophene ring; and
   R^{3A}, R^{3B}, R^{3C}, R^{3D}, R^{3E}, R^{3F}, R^{3G}, and R^{3H} are null, or the same or different, each independently:
   (1) a hydrogen atom;
   (2) a hydroxyl group;
   (3) a C₁₋₆ alkylcarbonyloxy group;
   (4) a C₁₋₆ alkylcarbonyl group; or
   (5) a carboxyl group.
[Item 39]
   The compound or a pharmaceutically acceptable salt thereof according to any one of items 2 to 35, wherein
   A is A-2 or A-4;
   ring G¹ is a benzene ring; and
   R^{3A}, R^{3B}, R^{3C}, R^{3D}, R^{3E}, and R^{3F} are the same or different, each independently:
   (1) a hydrogen atom;
   (2) a C₁₋₆ alkyl group; or
   (3) a C₁₋₆ alkoxy group, or,
   R^{3A} and R^{3B}, R^{3B} and R^{3C}, R^{3C} and R^{3D}, R^{3D} and R^{3E}, or R^{3E} and R^{3F}, when adjacent to each other on an aromatic ring, together with 2 carbon atoms on the aromatic ring to which they are attached, may form:
   (1') cyclohexane optionally substituted with 1 to 3 C₁₋₆ alkyl groups;
   (2') tetrahydrofuran optionally substituted with 1 to 3 C₁₋₆ alkyl groups; or
   (3') tetrahydropyran optionally substituted with 1 to 3 C₁₋₆ alkyl groups,
   wherein the cyclohexane, the tetrahydrofuran, or the tetrahydropyran is fused to the aromatic ring to form a structure comprising 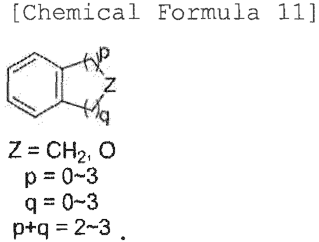
[Item 40]
   The compound or a pharmaceutically acceptable salt thereof according to any one of items 2 to 35, wherein
   A is A-9; and
   R^{3A}, R^{3B}, R^{3C}, and R^{3D} are the same or different, each independently:
   (1) a hydrogen atom; or
   (2) a 3- to 12-membered monocyclic or polycyclic heterocyclic group, wherein the heterocyclic group is optionally substituted with 1 to 3 groups selected from the group consisting of a hydroxyl group and a C₁₋₁₀ alkyl group, or
   R^{3A} and R^{3B} or R^{3B} and R^{3C}, together with 2 carbon atoms on the aromatic ring to which they are attached, may form a dihydropyranone ring optionally substituted with an optionally chloro-substituted C₆₋₁₀ phenyl group, wherein the dihydropyranone ring is fused to the aromatic ring to form a structure comprising 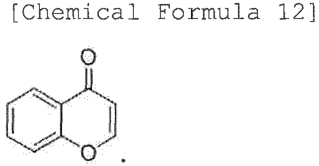
[Item 41] The compound or a pharmaceutically acceptable salt thereof according to any one of items 2 to 35, wherein
   A is A-1; and
   R^{3A}, R^{3B}, R^{3C}, and R^{3D} are the same or different, each independently an optionally substituted phenyl group, wherein the phenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation, or
   R^{3A} and R^{3B}, R^{3B} and R^{3C}, or R^{3C} and R^{3D}, together with 2 carbon atoms on the aromatic ring to which they are attached, may form an optionally substituted 1,3-dioxolane ring, wherein the 1,3-dioxolane ring is fused to the aromatic ring to form a structure comprising 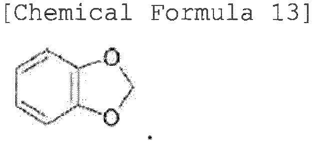
[Item 42]
   The compound or a pharmaceutically acceptable salt thereof according to item 1 or 2, wherein
   A is the following structure: 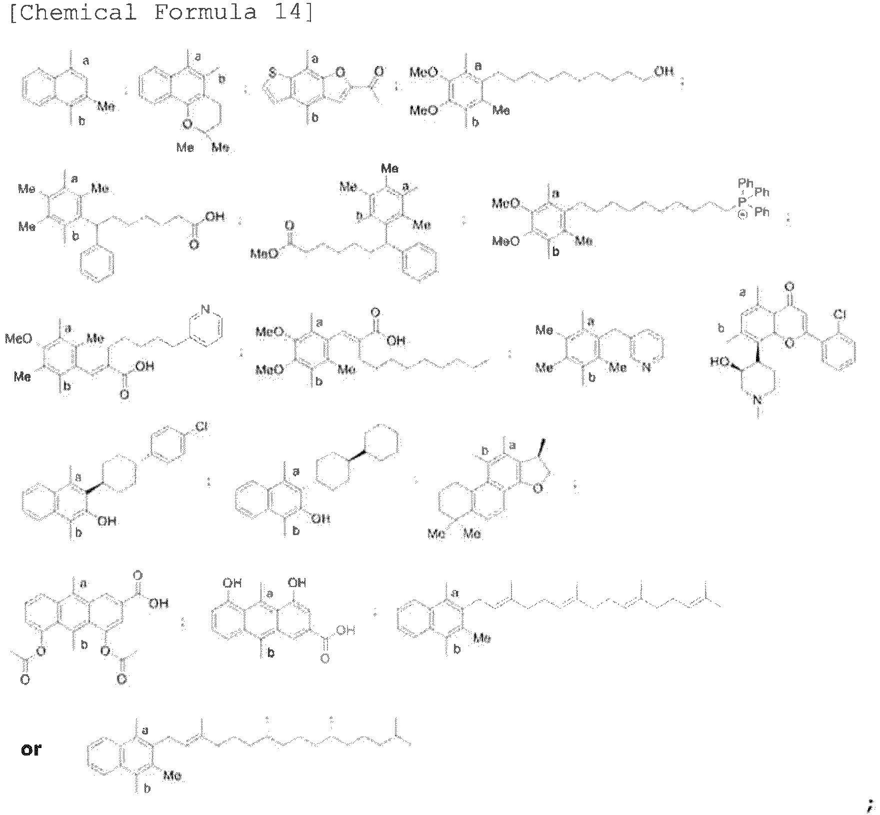
   X¹ and X² are the same or different, each independently a hydroxyl group or -O-C(=O)-Y-(C(R^{1A})(R^{1B}))ₙ-NH-R², wherein X¹ and X² are not simultaneously a hydroxyl group;
   n is the same or different, each independently 2, 3, or 4;
   Y is an oxygen atom or NR⁴;
   R^{1A} and R^{1B} are the same or different, each independently a hydrogen atom or -CO₂R⁵;
   R² is a hydrogen atom, or a C₁₋₆ alkyl group optionally substituted with 1 to 2 carboxyl groups;
   R⁴ is a hydrogen atom or a C₁₋₄ alkyl group; and
   R⁵ is a hydrogen atom, or a C₁₋₆ alkyl group optionally substituted with 1 to 2 groups selected from the group consisting of a fluorine atom and a carboxyl group.
[Item 43]
   The compound or a pharmaceutically acceptable salt thereof according to item 42, wherein A is the following structure: 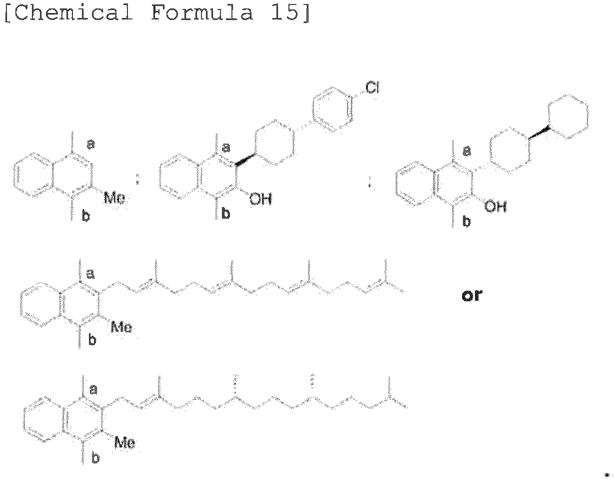
[Item 44]
   The compound or a pharmaceutically acceptable salt thereof according to item 42, wherein A is the following structure: 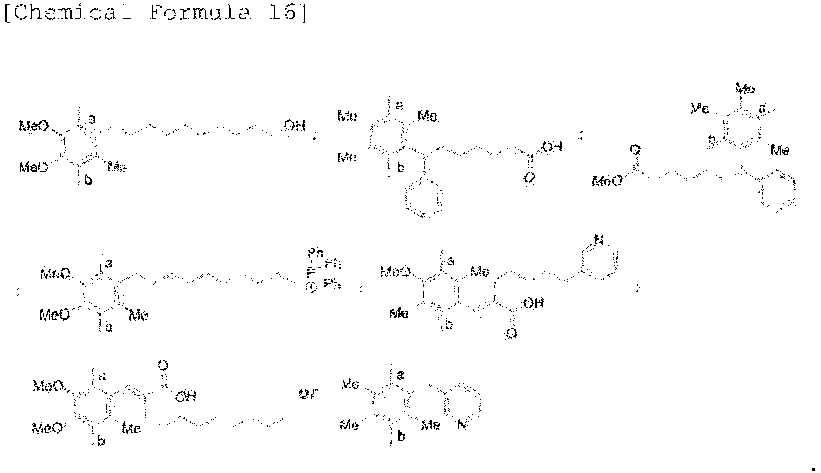
[Item 45]
   The compound or a pharmaceutically acceptable salt thereof according to item 42, wherein A is the following structure: 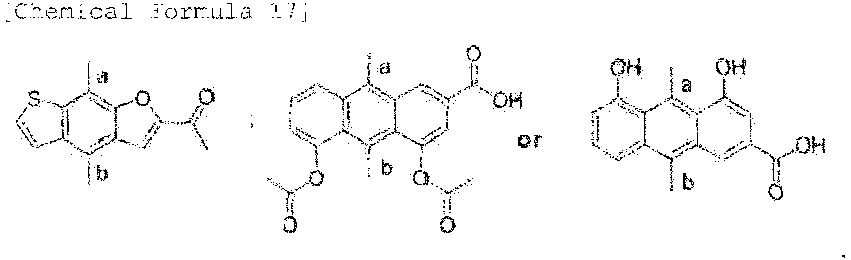
[Item 46]
   The compound or a pharmaceutically acceptable salt thereof according to item 42, wherein A is the following structure: 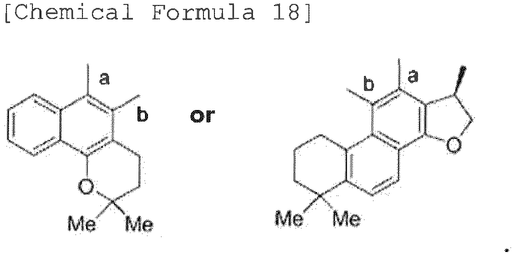
[Item 47]
   The compound or a pharmaceutically acceptable salt thereof according to item 42, wherein A is the following structure: 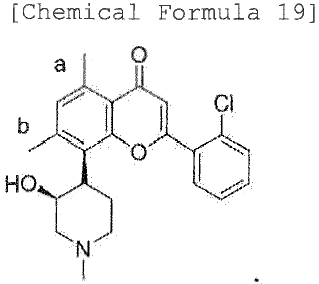
[Item 48]
   The compound or a pharmaceutically acceptable salt thereof of item 1 or 2, selected from the following compounds:
   2,2'-[(2-acetylthieno[3,2-f][1]benzofuran-4,8-diyl)bis(oxycarbonyliminoethane-2,1-diylimino)]diacetic acid;
   2,2'-[(2-methylnaphthalene-1,4-diyl)bis(oxycarbonyliminoethane-2,1-diylimino)]diacetic acid;
   2,2'-[(2-methylnaphthalene-1,4-diyl)bis(oxycarbonyloxybutane-4,1-diylimino)]diacetic acid;
   2,2'-[(2,2-dimethyl-3,4-dihydro-2H-benzo[H]chromene-5,6-diyl)bis(oxycarbonyliminoethane-2,1-diylimino)]diacetic acid;
   dimethyl(2S,2'S)-4,4'-[(2-{10-[(tert-butoxycarbonyl)oxy]decayl}-5,6-dimethoxy-3-methylbenzene-1,4-diyl)bis(oxycarbonylimino)]bis(2-aminobutanoate);
   4,7-diphenyl-1,3-benzodioxole-5,6-diyl bis[(3-aminopropyl)carbamate];
   2,2'-[1,1':4',1"-terphenyl-2',5'-diyl bis(oxycarbonyliminoethane-2,1-diylimino)]diacetic acid;
   2-(2-chlorophenyl)-5-hydroxy-8-[(3S,4R)-3-hydroxy-1-methylpiperidin-4-yl]-4-oxo-4H-chromen-7-yl (2-aminoethyl)methylcarbamate;
   2-(2-chlorophenyl)-7-hydroxy-8-[(3S,4R)-3-hydroxy-1-methylpiperidin-4-yl]-4-oxo-4H-chromen-5-yl (2-aminoethyl)methylcarbamate;
   2-(2-chlorophenyl)-5-hydroxy-8-[(3S,4R)-3-hydroxy-1-methylpiperidin-4-yl]-4-oxo-4H-chromen-7-yl methyl[2-(methylamino)ethyl]carbamate;
   2-(2-chlorophenyl)-7-hydroxy-8-[(3S,4R)-3-hydroxy-1-methylpiperidin-4-yl]-4-oxo-4H-chromen-5-yl methyl[2-(methylamino)ethyl]carbamate;
   2,2'-{[(1R)-1,6,6-trimethyl-1,2,6,7,8,9-hexahydrophenanthro[1,2-b]furan-10,11-diyl]bis(oxycarbonylazanediylethane-2,1-diylazanediyl)}diacetic acid; and
   2,2'-{[(1R)-1,6,6-trimethyl-1,2,6,7,8,9-hexahydrophenanthro[1,2-b]furan-10,11-diyl]bis(oxycarbonylazanediylpropane-3,1-diylazanediyl)}diacetic acid
[Item 49]
   The compound according to item 1 or 2, which is 2,2'-[(2-acetylthieno[3,2-f][1]benzofuran-4,8-diyl)bis(oxycarbonyliminoethane-2,1-diylimino)]diacetic acid dihydrochloride.
[Item 50]
   The compound according to item 1 or 2, which is 2-(2-chlorophenyl)-5-hydroxy-8-[(3S,4R)-3-hydroxy-1-methylpiperidin-4-yl]-4-oxo-4H-chromen-7-yl (2-aminoethyl)methylcarbamate ditrifluoroacetate.
[Item 51]
   The compound according to item 1 or 2, which is 2,2'-{[(1R)-1,6,6-trimethyl-1,2,6,7,8,9-hexahydrophenanthro[1,2-b]furan-10,11-diyl]bis(oxycarbonylazanediylethane-2,1-diylazanediyl)}diacetic acid dihydrochloride.
[Item 52]
   A pharmaceutical composition comprising the compound or a pharmaceutically acceptable salt thereof according to any one of items 1 to 51.
[Item 53]
   A therapeutic agent or a preventive agent for cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes, comprising the compound or a pharmaceutically acceptable salt thereof according to any one of items 1 to 51 as an active ingredient, or the pharmaceutical composition according to item 52.
[Item 54]
   A method for treating and/or preventing cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes, characterized by administering, to a patient in need thereof, a therapeutically and/or preventively effective amount of the compound or a pharmaceutically acceptable salt thereof according to any one of items 1 to 51 or the pharmaceutical composition according to item 52.
[Item 55]
   Use of the compound or a pharmaceutically acceptable salt thereof according to any one of items 1 to 51 or the pharmaceutical composition according to item 52 for the manufacture of a therapeutic agent and/or a preventive agent for cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes.
[Item 56]
   The compound or a pharmaceutically acceptable salt thereof according to any one of items 1 to 51 or the pharmaceutical composition according to item 52 for use in the treatment and/or prevention of cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes.

In addition to the above, the present invention provides a therapeutic method, preventive method, use, and the like using the compound, pharmaceutically acceptable salt thereof, pharmaceutical composition, therapeutic agent, or preventive agent of the invention. Those skilled in the art can understand more details and embodiments of the methods and use thereof from the descriptions herein.

It is understood that one or more of the features described above can be further combined and used. Additional embodiments and advantages of the inventions are recognized by those skilled in the art by reading and understanding the following detailed description as needed.

### [Advantageous Effects of Invention]

While lipid-soluble agents such as polyphenols, ortho-quinones, and para-quinones have a wide range of pharmacological actions, the agents have poor absorbability through oral administration and limited use in parenteral administration due to the high crystallizability and high lipid solubility thereof. A compound represented by formula (1) or a pharmaceutically acceptable salt thereof (also referred to as the compound of the invention) is a water-soluble prodrug of polyphenols, ortho-quinones, para-quicones, or the like. Such a prodrug can be intravenously administered in view of the high water solubility thereof, and is useful as a drug that can be successfully used for cancer prevention and/or therapy.

### [Description of Embodiments]

Since the compound of the invention can also be in a form of a hydrate and/or solvate, hydrates and/or solvates of a compound represented by formula (1) and a pharmaceutically acceptable salt thereof are also encompassed by the compound of the invention.

Since a compound of formula (1) can have one or optionally more asymmetric carbon atoms and can have geometric isomerism or axial chirality, the compound can be present as several types of stereoisomers. In the present invention, these stereoisomers, and mixtures and racemates thereof, are encompassed by the compound represented by formula (1) of the invention.

Deuterated forms with any one or two or more ¹H of a compound represented by general formula (1) converted to ²H(D) are also encompassed by the compound represented by general formula (1).

A compound represented by general formula (1), and a pharmaceutically acceptable salt thereof, obtained as a crystal can have crystal polymorphisms. The compound of the invention includes all crystalline forms.

The terms herein are now described hereinafter.

As used herein, the number of substituents in a group when defined as "optionally substituted" is not particularly limited and is one or more, as long as it is substitutable. The description for each group is also applied when the group is a part of or a substituent on another group, unless specifically noted otherwise.

Examples of substituents in the present invention include a hydrogen atom, hydroxyl group, carboxyl group, sulfinate group, sulfonate group, phosphate group, guanidine group, cyano group, halogen atom (fluorine atom, chlorine atom, and the like), alkyl group, alkylthio group, cycloalkylthio group, alkenyl group, alkynyl group, cycloalkyl group, cycloalkenyl group, cycloalkylcarbonyl group, alkylcarbonyloxy group, alkylsulfinyl group, cycloalkylsulfinyl group, alkoxy group, cycloalkoxy group, alkoxycarbonyl group, cycloalkyloxycarbonyl group, alkylcarbonyl group, aryl group, arylcarbonyl group, arylthio group, aryloxycarbonyl group, heteroaryl group, heterocyclic group, amino group, cyclic amino group, aminocarbonyl group, aminosulfinyl group, aminosulfonyl group, heterocyclyloxycarbonyl group, heterocyclylsulfinyl group, heterocyclylsulfonyl group, heterocyclylcarbonyl group, alkylsulfonyl group, cycloalkylsulfonyl group, arylsulfonyl group, arylsulfinyl group, and triphenylphosphonium cation group. The substituents described above can be further substituted with an aforementioned substituent.

Examples of a "halogen atom" as used herein include a fluorine atom, chlorine atom, bromine atom, and iodine atom. A halogen atom is preferably a fluorine atom or a chlorine atom.

An "alkyl group" refers to a straight or branched, saturated hydrocarbon group. For example, a "C₁₋₄ alkyl group" and a "C₆ alkyl group" refer to alkyl groups with 1 to 4 and 6 carbon atoms, respectively. The same applies to other numbers.

Preferred examples of "C₁₋₃₀ alkyl group" include a "C₁₋₂₀ alkyl group". Another preferred embodiment includes a "C₁₋₁₀ alkyl group". A still another preferred embodiment includes a "C₁₋₆ alkyl group".

Specific examples of "C₁₋₃₀ alkyl group" include a methyl group, ethyl group, propyl group, 1-methylethyl group, butyl group, 2-methylpropyl group, 1-methylpropyl group, 1,1-dimethylethyl group, pentyl group, 3-methylbutyl group, 2-methylbutyl group, 2,2-dimethylpropyl group, 1-ethylpropyl group, 1,1-dimethylpropyl group, hexyl group, 4-methylpentyl group, 3-methylpentyl group, 2-methylpentyl group, 1-methylpentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosanyl group, tricosanyl group, tetracosanyl group, pentacosanyl group, hexacosanyl group, heptacosanyl group, octacosanyl group, nonacosanyl group, triacontyl group, and the like.

Specific examples of "C₁₋₂₀ alkyl group" include the example of 1 to 20 carbons in the specific examples of "C₁₋₃₀ alkyl group".

Specific examples of "C₁₋₁₀ alkyl group" include the example of 1 to 10 carbons in the specific examples of "C₁₋₃₀ alkyl group".

Specific examples of "C₁₋₆ alkyl group" include the example of 1 to 6 carbons in the specific examples of "C₁₋₃₀ alkyl group".

The "C₁₋₂₀ alkyl" portion of a "C₁₋₂₀ alkylthio group", "C₁₋₂₀ alkylsufinyl group", and "C₁₋₂₀ alkylsulfonyl group" is defined the same as the "C₁₋₂₀ alkyl group" described above. Specific examples of "C₁₋₂₀ alkylthio group" include a methylthio group and the like. Specific examples of "C₁₋₂₀ alkylsulfinyl group" include a methylsulfinyl group and the like. Specific examples of "C₁₋₂₀ alkylsulfonyl group" include a methylsulfonyl group and the like".

A "C₂₋₃₀ alkenyl group" refers to a straight or branched, unsaturated hydrocarbon group having 2 to 30 carbons and comprising 1 to 10 double bonds. Preferred examples of "C₂₋₃₀ alkenyl group" include a "C₂₋₂₀ alkenyl group". Another preferred embodiment includes a "C₂₋₁₀ alkenyl group". A still another preferred embodiment includes a "C₂₋₆ alkenyl group".

Specific examples of "C₂₋₃₀ alkenyl group" include a vinyl group, propenyl group, methylpropenyl group, butenyl group, methylbutenyl group, pentenyl group, dimethylallyl group, hexenyl group, heptenyl group, octenyl group, nonenyl group, decenyl group, geranyl group, undecenyl group, dodecenyl group, tridecenyl group, tetradecenyl group, pentadecenyl group, farnesyl group, hexadecenyl group, heptadecenyl group, octadecenyl group, nonadecenyl group, icocenyl group, geranylgeranyl group, heneicocenyl group, dococenyl group, tricocenyl group, tetracocenyl group, pentacocenyl group, hexacocenyl group, heptacocenyl group, octacocenyl group, nonacosenyl group, triacontenyl group, and the like.

Specific examples of "C₂₋₂₀ alkenyl group" include the example of 1 to 20 carbons in the specific examples of "C₂₋₃₀ alkenyl group".

Specific examples of "C₂₋₁₀ alkenyl group" include the examples of 1 to 10 carbons in the specific examples of "C₂₋₃₀ alkenyl group".

Specific examples of "C₂₋₆ alkenyl group" include the example of 1 to 6 carbons in the specific examples of "C₂₋₃₀ alkenyl group".

A "C₂₋₂₀ alkynyl group" refers to a straight or branched, unsaturated hydrocarbon group having 2 to 20 carbons and comprising one triple bond. Preferred examples of "C₂₋₂₀ alkynyl group" include a "C₂₋₁₀ alkynyl group". Specific examples of "C₂₋₂₀ alkynyl group" include a propynyl group, methylpropynyl group, butynyl group, methylbutynyl group, pentynyl group, hexynyl group, heptynyl group, octynyl group, nonynyl group, decynyl group, undecynyl group, dodecynyl group, tridecynyl group, tetradecynyl group, pentadecynyl group, hexadecynyl group, heptadecynyl group, octadecynyl group, nonadecynyl group, eicosinyl group, and the like.

Specific examples of "C₂₋₁₀ alkynyl group" include the example of 2 to 10 carbons in the specific examples of "C₂₋₂₀ alkynyl group".

A "C₃₋₁₀ cycloalkyl group" refers to cyclic alkyl with 3 to 10 carbon atoms, including those having a partially crosslinked structure. Preferred examples of "C₃₋₁₀ cycloalkyl group" include a "C₃₋₇ cycloalkyl group", and more preferred examples include a "C₄₋₆ cycloalkyl group". Specific examples of "C₃₋₁₀ cycloalkyl group" include a cyclopropyl group, cyclobutyl group, cyclopentyl group, cyclohexyl group, cycloheptyl group, cyclooctyl group, adamantyl group, and the like. Specific examples of "C₃₋₇ cycloalkyl group" include a cyclopropyl group, cyclobutyl group, cyclopentyl group, cyclohexyl group, cycloheptyl group, and the like.

The "C₃₋₁₀ cycloalkyl group" described above also encompasses groups fused to an aromatic ring. Specific examples thereof include the groups represented by the following chemical formulas and the like.

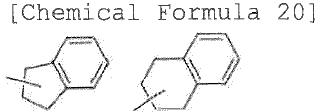

The "C₃₋₁₀ cycloalkyl group" described above also encompasses a saturated bicyclo ring group. Specific examples thereof include groups represented by the following groups and the like.

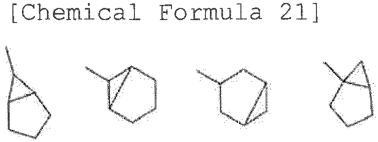

The "C₃₋₁₀ cycloalkyl" portion of a "C₃₋₁₀ cycloalkylcarbonyl group", "C₃₋₁₀ cycloalkylthio group", "C₃₋₁₀ cycloalkylsulfinyl group", and "C₃₋₁₀ cycloalkylsulfonyl group" is defined the same as the "C₃₋₁₀ cycloalkyl group" described above. Preferred examples of "C₃₋₁₀ cycloalkylcarbonyl group", "C₃₋₁₀ cycloalkylthio group", "C₃₋₁₀ cycloalkylsulfinyl group", and "C₃₋₁₀ cycloalkylsulfonyl group" include groups where the "C₃₋₁₀ cycloalkyl" portion is a "C₃₋₇ cycloalkyl group". Specific examples of "C₃₋₁₀ cycloalkylcarbonyl group" include a cyclopropylcarbonyl group and the like. Specific examples of "C₃₋₁₀ cycloalkylthio group" include a cyclopropylthio group and the like. Specific examples of "C₃₋₁₀ cycloalkylsulfinyl group" include a cyclopropylsulfinyl group, and the like. Specific examples of "C₃₋₁₀ cycloalkylsulfonyl group" include a cyclopropylsulfonyl group and the like.

"C₃₋₁₀ cycloalkane" refers to a cyclic alkane with 3 to 10 carbon atoms, including those having a partially crosslinked structure. "C₃₋₁₀ cycloalkane" is preferably "C₅₋₈ cycloalkane", and more preferably "C₅₋₆ cycloalkane". Specific examples of "C₃₋₁₀ cycloalkane" include cyclopropane, cyclobutane, cyclopentane, cyclohexane, cycloheptane, cyclooctane, adamantane, and the like. Specific examples of "C₅₋₈ cycloalkane" include cyclopentane, cyclohexane, cycloheptane, cyclooctane, and the like.

The "C₃₋₁₀ cycloalkane" described above also encompasses compounds fused to an aromatic ring. Specific examples thereof include rings represented by the following and the like.

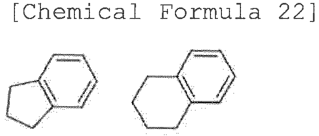

The "C₃₋₁₀ cycloalkane" described above also encompasses saturated bicyclo rings. Specific examples thereof include rings represented by the following group and the like.

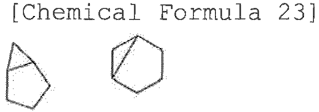

"R' and R", when adjacent to each other on an aromatic ring, together with 2 carbon atoms on the aromatic ring to which they are attached, may form C₃₋₁₀ cycloalkane" means that "C₃₋₁₀ cycloalkane" formed by R' and R" may form a fused ring with an aromatic ring. The "C₃₋₁₀ cycloalkane" portion of "R' and R", when adjacent to each other on an aromatic ring, together with 2 carbon atoms on the aromatic ring to which they are attached, may form C₃₋₁₀ cycloalkane" is defined the same as the "C₃₋₁₀ cycloalkane" described above. Specific examples of the fused ring include, when the aromatic ring is a benzene ring, rings represented by the following.

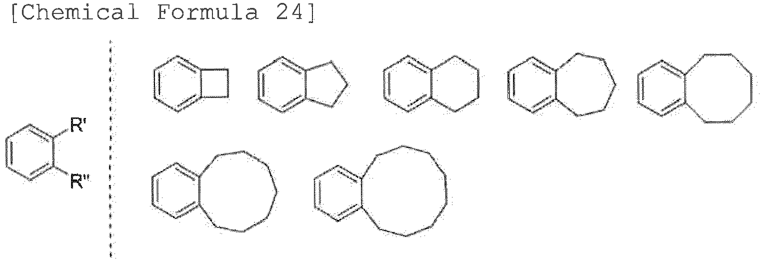

A "C₃₋₁₀ cycloalkenyl group" refers to a hydrocarbon ring with 3 to 10 carbon atoms that is an unsaturated hydrocarbon group with at least one of the carbon bonds forming a ring being a double bond, including those having a partially crosslinked structure. Specific examples of "C₃₋₁₀ cycloalkenyl group" include a cyclopropenyl group, cyclopentenyl group, cyclohexenyl group, cycloheptenyl group, cyclooctenyl group, and the like.

A "C₁₋₂₀ alkoxy group" is a "C₁₋₂₀ alkyloxy group". The "C₁₋₂₀ alkyl" portion is defined the same as the "C₁₋₂₀ alkyl group" described above. Preferred examples of "C₁₋₂₀ alkoxy group" include a "C₁₋₁₀ alkoxy group". More preferred examples of "C₁₋₂₀ alkoxy group" include a "C₁₋₆ alkoxy group".

Specific examples of "C₁₋₂₀ alkoxy group" include a methoxy group, ethoxy group, propoxy group, 1-methylethoxy group, butoxy group, 2-methylpropoxy group, 1-methylpropoxy group, 1,1-dimethylethoxy group, pentyloxy group, 3-methylbutoxy group, 2-methylbutoxy group, 2,2-dimethylpropoxy group, 1-ethylpropoxy group, 1,1-dimethylpropoxy group, hexyloxy group, 4-methylpentyloxy group, 3-methylpentyloxy group, 2-methylpentyloxy group, 1-methylpentyloxy group, 3,3-dimethylbutoxy group, 2,2-dimethylbutoxy group, 1,1-dimethylbutoxy group, 1,2-dimethylbutoxy group, heptyloxy group, octyloxy group, nonyloxy group, decyloxy group, undecyloxy group, dodecyloxy group, tridecyloxy group, tetradecyloxy group, pentadecyloxy group, hexadecyloxy group, heptadecyloxy group, octadecyloxy group, nonadecyloxy group, eicocyloxy group, and the like.

Specific examples of "C₁₋₁₀ alkoxy group" include the example of 1 to 10 carbons in the specific examples of "C₁₋₂₀ alkoxy group".

Specific examples of "C₁₋₆ alkoxy group" include the example of 1 to 6 carbons in the specific examples of "C₁₋₂₀ alkoxy group".

A "C₃₋₁₀ cycloalkoxy group" is a "C₃₋₁₀ cycloalkyloxy group". The "C₃₋₁₀ cycloalkyl" portion is defined the same as the "C₃₋₁₀ cycloalkyl group" described above. Preferred examples of "C₃₋₁₀ cycloalkoxy group" include a "C₃₋₇ cycloalkoxy group". Specific examples of "C₃₋₁₀ cycloalkoxy group" include a cyclopropoxy group, cyclobutoxy group, cyclopentyloxy group, cyclohexyloxy group, cycloheptyloxy group, cyclooctyloxy group, adamantyloxy group, and the like. Specific examples of "C₃₋₇ cycloalkoxy group" include a cyclopropoxy group, cyclobutoxy group, cyclopentyloxy group, cyclohexyloxy group, cycloheptyloxy group, and the like.

The "C₃₋₁₀ cycloalkyloxy" portion of a "C₃₋₁₀ cycloalkyloxycarbonyl group" is defined the same as the "C₃₋₁₀ cycloalkyloxy group" described above. Preferred examples of "C₃₋₁₀ cycloalkyloxycarbonyl group" include a "C₃₋₇ cycloalkyloxycarbonyl group". Specific examples of "C₃₋₁₀ cycloalkyloxycarbonyl group" include a cyclopropoxycarbonyl group and the like.

The "C₁₋₂₀ alkoxy" portion of a "C₁₋₂₀ alkoxycarbonyl group" is defined the same as the "C₁₋₂₀ alkoxy group" described above. Preferred examples of "C₁₋₂₀ alkoxycarbonyl group" include a "C₁₋₁₀ alkoxycarbonyl group". Specific examples of "C₁₋₂₀ alkoxycarbonyl group" include a methoxycarbonyl group, ethoxycarbonyl group, and the like.

The "C₁₋₂₀ alkyl" portion of a "C₁₋₂₀ alkylcarbonyl group" is defined the same as the "C₁₋₂₀ alkyl group" described above. Preferred examples of "C₁₋₂₀ alkylcarbonyl group" include a "C₁₋₁₀ alkylcarbonyl group". Specific examples of "C₁₋₂₀ alkylcarbonyl group" include a methylcarbonyl group, ethylcarbonyl group, propylcarbonyl group, 1-methylethylcarbonyl group, butylcarbonyl group, 2-methylpropylcarbonyl group, 1-methylpropylcarbonyl group, 1,1-dimethylethylcarbonyl group, and the like.

The "C₁₋₂₀ alkyl" portion of a "C₁₋₂₀ alkylcarbonyloxy group" is defined the same as the "C₁₋₂₀ alkyl group" described above. Preferred examples of "C₁₋₂₀ alkylcarbonyloxy group" include a "C₁₋₁₀ alkylcarbonyloxy group". Specific examples of "C₁₋₂₀ alkylcarbonyloxy group" include a methylcarbonyloxy group, ethylcarbonyloxy group, propylcarbonyloxy group, 1-methylethylcarbonyloxy group, butylcarbonyloxy group, 2-methylpropylcarbonyloxy group, 1-methylpropylcarbonyloxy group, 1,1-dimethylethylcarbonyloxy group, and the like.

A "C₆₋₁₀ aryl group" refers to an aromatic hydrocarbon group with 6 to 10 carbon atoms. Specific examples of "C₆₋₁₀ aryl group" include a phenyl group, 1-naphthyl group, 2-naphthyl group, and the like. Particularly preferred examples include a phenyl group.
The "C₆₋₁₀ aryl group" also encompasses 8- to 14-membered polycyclic groups with C₄₋₆ cycloalkane fused to an aromatic group and 9- to 14-membered polycyclic groups with a 5- to 6-membered heterocycle fused to an aromatic ring, wherein the heterocycle has 1 to 3 same or different atoms selected from, for example, a nitrogen atom, an oxygen atom, and a sulfur atom. Specific examples thereof include groups represented by the following and the like.

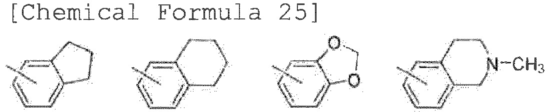

The "C₆₋₁₀ aryl" portion of a "C₆₋₁₀ arylcarbonyl group", "C₆₋₁₀ aryloxycarbonyl group", "C₆₋₁₀ arylthio group", "C₆₋₁₀ arylsulfinyl group", and "C₆₋₁₀ arylsulfonyl group" is defined the same as the "C₆₋₁₀ aryl group" described above. The "C₆₋₁₀ aryl" portion is preferably a phenyl group. Specific examples of "C₆₋₁₀ arylcarbonyl group" include a phenylcarbonyl group and the like. Specific examples of "C₆₋₁₀ aryloxycarbonyl group" include a phenyloxycarbonyl group and the like. Specific examples of "C₆₋₁₀ arylthio group" include a phenylthio group and the like. Specific examples of "C₆₋₁₀ arylsulfinyl group" include a phenylsufinyl group and the like. Specific examples of "C₆₋₁₀ arylsulfonyl group" include a phenylsufonyl group and the like.

A "5- to 10-membered heteroaryl group" comprises 1 to 4 atoms independently selected from the group consisting of a nitrogen atom, an oxygen atom, and a sulfur atom and includes a monocyclic 5- to 7-membered aromatic heterocyclic group ("5- to 7-membered heteroaryl group") and bicyclic 8- to 10-membered aromatic heterocyclic group ("8- to 10-membered heteroaryl group"). A "5- to 10-membered heteroaryl group" is preferably a monocyclic 5- to 7-membered aromatic heterocyclic group ("5- to 7-membered heteroaryl group"), and more preferably a 5- to 6-membered monocyclic aromatic heterocyclic group ("5- to 6-membered heteroaryl group").

Specific examples of "5- to 10-membered heteroaryl group" include a pyridyl group, pyridazinyl group, isothiazolyl group, pyrrolyl group, furyl group, thienyl group, thiazolyl group, imidazolyl group, pyrimidinyl group, thiadiazolyl group, pyrazolyl group, oxazolyl group, isoxazolyl group, pyrazinyl group, triazinyl group, triazolyl group, imidazolidinyl group, oxadiazolyl group, triazolyl group, tetrazolyl group, indolyl group, indazolyl group, quinolyl group, isoquinolyl group, benzofuranyl group, benzothienyl group, benzoxazolyl group, benzothiazolyl group, benzisoxazolyl group, benzisothiazolyl group benzotriazolyl group, benzimidazolyl group, 6,11-dihydrodibenzo[b,e]thiepinyl group, and the like. Preferably, the group is a pyridyl group, pyrimidinyl group, quinolyl group, or isoquinolyl group, and more preferably a pyridyl group.

Examples of "3- to 18-membered monocyclic or polycyclic heterocyclic group" include monocyclic or polycyclic heterocyclic groups comprising one or more atoms independently selected from the group consisting of a nitrogen atom, an oxygen atom, and a sulfur atom, and the like. The group is preferably the following "3- to 12-membered monocyclic or polycyclic heterocyclic group".

Examples of "3- to 12-membered monocyclic or polycyclic heterocyclic group" include monocyclic or polycyclic heterocyclic groups comprising 1 to 4 atoms independently selected from the group consisting of a nitrogen atom, an oxygen atom, and a sulfur atom, and the like. The group is preferably a 3- to 10-membered group, more preferably a 5- to 8- membered group, and still more preferably a 5- or 6-membered group. The nitrogen atom, oxygen atom, and sulfur atom are all ring-constituting atoms. The heterocyclic group may be saturated or partially unsaturated, and a saturated heterocyclic group is more preferable. The heterocyclic group also includes heterocyclic groups substituted with an oxo group and heterocyclic groups having a crosslinked structure. In the heterocyclic group, the "group" would never have the point of attachment at a ring-constituting nitrogen atom, however, the heterocyclic group may have a substituent on a nitrogen atom if the heterocyclic group is an optionally substituted "3- to 12-membered monocyclic or polycyclic heterocyclic group".

Specific examples of "3- to 12-membered monocyclic or polycyclic heterocyclic group" include the following oxiranyl group, oxetanyl group, tetrahydrofuranyl group, dihydrofuranyl group, dioxolanyl group, tetrahydropyranyl group, tetrahydropyranyl group, dioxanyl group, oxathianyl group, tetrahydropyranyl group, dihydropyranyl group, pyranyl group, oxathiane dioxanyl group, dihydropyranyl group, oxadinanyl group, morpholinyl group, morpholinonyl group, oxepanyl group, dioxepanyl group, oxathiepanyl group, oxathiepanonyl group, oxazepanyl group, oxazepanonyl group, oxabicyclooctanyl group, oxabicycloheptanyl group, oxaazabicyclooctanyl group, dioxaspirononanyl group, octahydropyranopyridinyl group, aziridinyl group, azetidinyl group, pyrrolidinyl group, pyrrolidinolyl group, imidazolidinonyl group, oxazolidinonyl group, piperidinyl group, piperidinonyl group, thiomorpholinyl group, dihydropyridinonyl group, pyridinonyl group, thiomorpholinedioxinyl group, dihydropyridinyl group, dihydropyrimidinonyl group, piperazinyl group, piperazinonyl group, azepanyl group, oxazepanyl group, thiazepanyl group, thiazepanonyl group, diazepanyl group, diazepanonyl group, azabicyclooctanyl group, azabicycloheptanyl group, diazabicyclooctanyl group, azaspironononyl group, octahydronaphthylidinyl group, and the like.

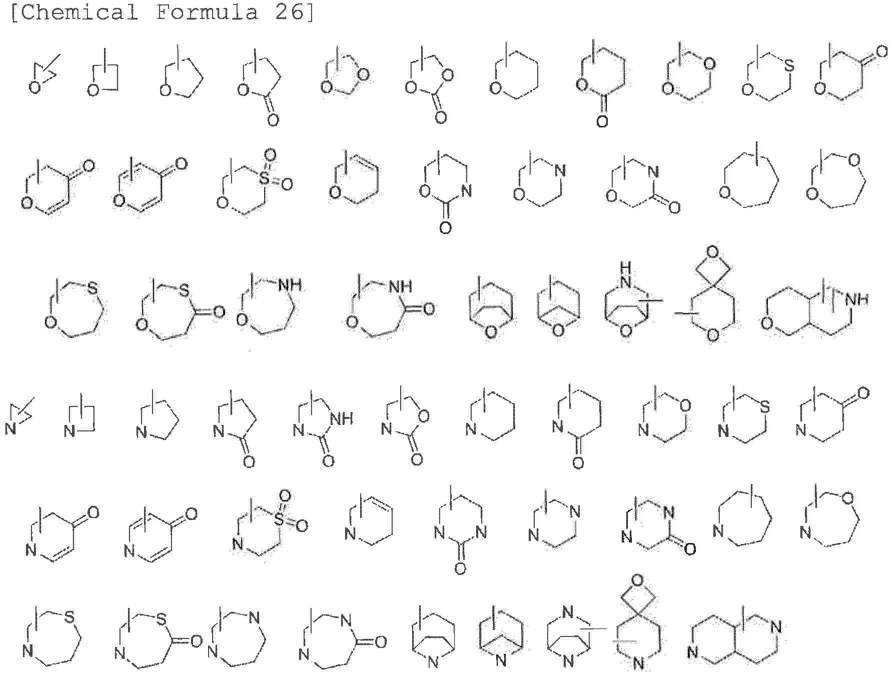

Specific examples of "3- to 12-membered monocyclic or polycyclic heterocyclic group" optionally substituted with 1 to 2 substituents selected from the group consisting of a C₁₋₁₀ alkyl group and a hydroxy group include the following 3-hydroxy-N-methyl-piperidinyl group and the like.

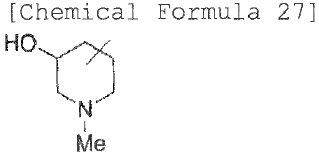

The "3- to 12-membered monocyclic or polycyclic heterocyclic group" described above may form a fused ring with a 6-membered aromatic hydrocarbon or 6-membered heteroaryl. Examples thereof include a bicyclic "heterocyclic group" having 9 or 10 ring-constituting atoms, with the aforementioned 5- to 6-membered "heterocyclic group" fused to a 6-membered aromatic hydrocarbon or 6-membered heteroaryl. Examples of 6-membered aromatic hydrocarbon include benzene and the like. Examples of 6-membered heteroaryl include pyridine, pyrimidine, pyridazine, and the like. Specific examples of the fused ring group include the following chromanyl group, chromenyl group, dihydrobenzofuranyl group, dihydroisobenzofuranyl group, benzodioxanyl group, tetrahydroquinolinyl group, dihydroquinolinyl group, indolinyl group, isoindolinyl group, dihydrobenzoxazoyl group, chromanonyl group, chromenonyl group, benzofuranoyl group, isobenzofuranoyl group, dihydroquinolinoyl group, quinolinoyl group, indolinoyl group, isoindolinoyl group, purinyl group, and the like.

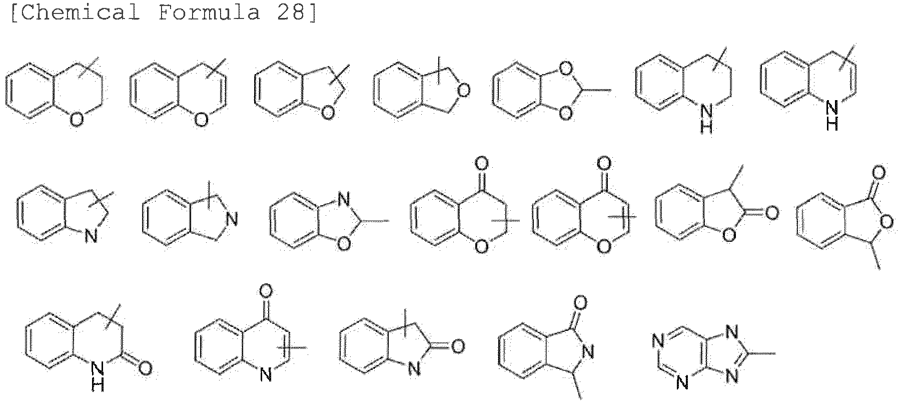

Examples of "3- to 12-membered monocyclic or polycyclic heterocycle" include monocyclic or polycyclic heterocycles comprising 1 to 4 atoms independently selected from the group consisting of a nitrogen atom, an oxygen atom, and a sulfur atom, and the like. It is preferably a 3- to 10-membered ring, more preferably 5- to 8-membered ring, and still more preferably 5- or 6-membered ring. The nitrogen atom, oxygen atom, and sulfur atom are all ring-constituting atoms. The heterocycle may be saturated or partially unsaturated, and a partially unsaturated saturated heterocycle is more preferable. The heterocycle also includes heterocycles substituted with an oxo group and heterocycles having a crosslinked structure. Specific examples of "3- to 12-membered monocyclic or polycyclic heterocycle" include the following oxirane, oxetane, tetrahydrofuran, dihydrofuranone, dioxolane, dioxolanone, tetrahydropyran, tetrahydropyranone, dioxane, oxathiane, tetrahydropyranone, dihydropyranone, pyranone, oxathiane dioxide, dihydropyran, oxazinanone, morpholine, morpholinone, oxepane, dioxepane, oxathiepane, oxathiepanone, oxazepane, oxazepanone, oxabicyclooctane, oxabicycloheptane, oxazabicyclooctane, dioxaspirononane, octahydropyranopyridine, aziridine, azetidine, pyrrolidine, pyrrolidinone, imidazolidinone, oxazolidinone, piperidine, piperidinone, thiomorpholine, dihydropyridinone, pyridinone, thiomorpholine dioxide, dihydropyridine, dihydropyrimidinone, piperazine, piperazinone, azepane, oxazepane, thiazepane, thiazepanone, diazepane, diazepanone, azabicyclooctane, azabicycloheptane, diazabicyclooctane, azaspirononane, octahydronaphthyridine, and the like.

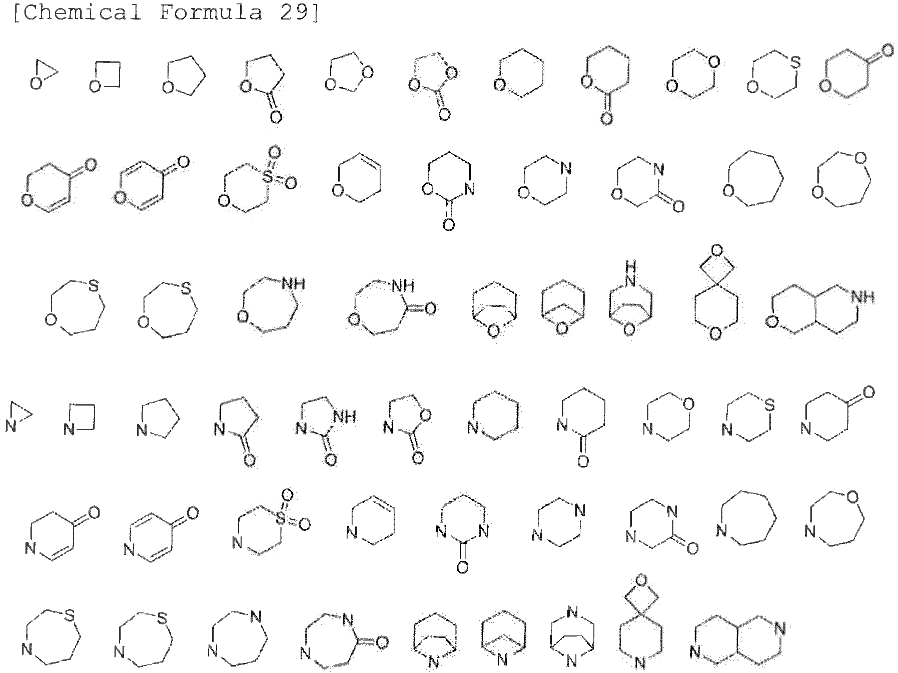

The "3- to 12-membered monocyclic or polycyclic heterocycle" described above may form a fused ring with a 6-membered aromatic hydrocarbon or 6-membered heteroaryl. Examples thereof include a bicyclic "heterocycle" having 9 or 10 ring-constituting atoms, wherein the aforementioned 5- to 6-membered "heterocyclic group" is fused to a 6-membered aromatic hydrocarbon or 6-membered heteroaryl. Examples of 6-membered aromatic hydrocarbon include benzene and the like. Examples of 6-membered unsaturated heteroaryl include pyridine, pyrimidine, pyridazine, and the like. Specific examples of the fused ring include the following chromane, chromene, dihydrobenzofuran, dihydroisobenzofuran, benzodioxane, tetrahydroquinoline, dihydroquinoline, indoline, isoindoline, dihydrobenzoxazole, chromanone, chromenone, benzofuranone, isobenzofuranone, benzodioxanone, dihydroquinolinone, quinolinone, indolinone, isoindolinone, benzoxazolinone, purine, and the like.

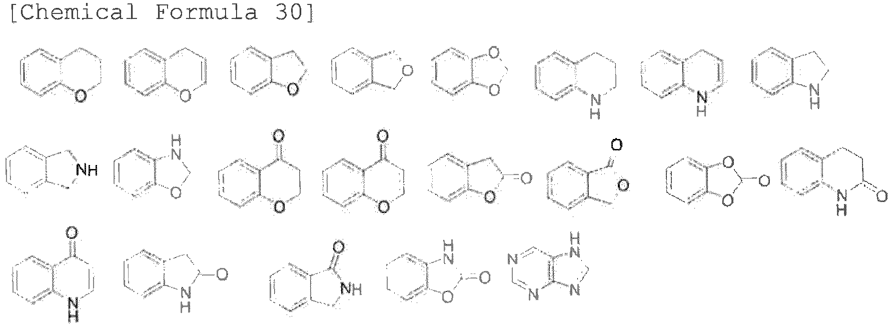

"R' and R", when adjacent to each other on an aromatic ring, together with 2 carbon atoms on the aromatic ring to which they are attached, may form a 3- to 12-membered monocyclic or polycyclic heterocycle" described above means that a "3- to 12-membered monocyclic or polycyclic heterocycle" formed by R' and R" may form a fused ring with an aromatic ring. The "3- to 12-membered monocyclic or polycyclic heterocycle" portion of "R' and R", when adjacent to each other on an aromatic ring, together with 2 carbon atoms on the aromatic ring to which they are attached, may form a 3- to 12-membered monocyclic or polycyclic heterocycle" is defined the same as the "3- to 12-membered monocyclic or polycyclic heterocycle" described above. Specific examples of the fused ring include, for example, when an aromatic ring is a benzene ring, the following chromane, chromene, dihydrobenzofuran, dihydroisobenzofuran, benzodioxane, tetrahydroquinoline, dihydroquinoline, indoline, isoindoline, dihydrobenzoxazole, chromanone, chromenone, benzofuranone, isobenzofuranone, benzodioxanone, dihydroquinolinone, quinolinone, indolinone, isoindolinone, benzoxazolinone, and the like.

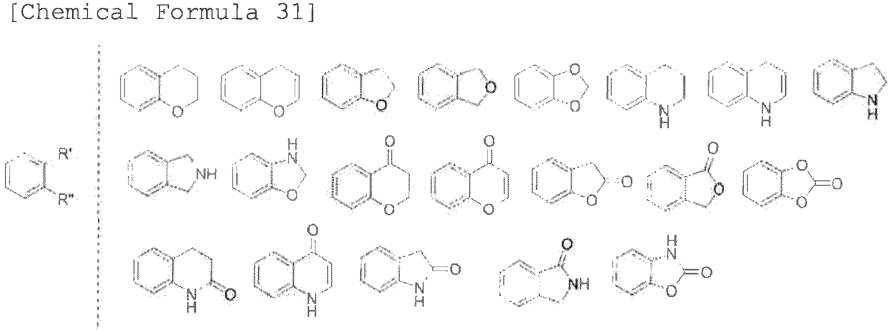

A "hydrocarbon ring" is a monocyclic or polycyclic compound comprised of three or more carbon atoms. The "C₃₋₁₀ cycloalkyl group", "C₃₋₁₀ cycloalkenyl group", and the like described above are encompassed by "hydrocarbon group".

A "fused ring" is a structure of two or more rings sharing and binding two or more atoms in a polycyclic hydrocarbon ring and heterocycle. Fused ring is used as a collective term for compounds having said structure herein.

Examples of "optionally substituted amino group" include unsubstituted amino and mono- or di-substituted amino.

Examples of substituents of "mono- or di-substituted amino" include "C₁₋₆ alkyl", "C₃₋₁₀ cycloalkyl", "C₃₋₁₀ cycloalkyl C₁₋₄ alkyl", "C₃₋₇ cycloalkyl C₁₋₄ alkoxycarbonyl", "C₁₋₄ alkylcarbonyl", "C₁₋₄ alkyloxycarbonyl", "3- to 8-membered heterocycle", "3- to 8-membered heterocyclyl C₁₋₄ alkyl", "3- to 8-membered heterocyclylcarbonyl", "3- to 8-membered heterocyclyloxycarbonyl", "3- to 8-membered heterocyclyl C₁₋₄ alkylcarbonyl", "C₆₋₁₀ aryl", "C₇₋₁₄ aralkyl", "C₆₋₁₀ arylcarbonyl", "C₆₋₁₀ aryloxycarbonyl", "5- or 6-membered heteroaryl", "5- or 6-membered heteroaryl C₁₋₄ alkyl", and the like.

In this regard, a "3- to 8-membered heterocyclyl C₁₋₄ alkyl" refers to a "C₁₋₄ alkyl group" that is substituted with a "3- to 8-membered heterocycle". A "3- to 8-membered heterocyclyl C₁₋₄ alkylcarbonyl" refers to a "C₁₋₄ alkylcarbonyl group" that is substituted with "3- to 8-membered heterocycle". A "C₇₋₁₄ aralkyl" refers to a "C₁₋₄ alkyl group" that is substituted with "C₆₋₁₀ aryl group". A "5- or 6-membered heteroaryl C₁₋₄ alkyl" refers to a "C₁₋₄ alkyl group" that is substituted with a "5- or 6-membered heteroaryl".

Specific examples of "mono-substituted amino" include:
"C₁₋₆ alkylamino" (e.g., methylamino, ethylamino, propylamino, 1-methylethylamino, butylamino, 2-methylpropylamino, 1-methylpropylamino, 1,1-dimethylethylamino, and the like);
"C₃₋₈ cycloalkylamino" (e.g., cyclopropylamino, cyclobutylamino, cyclopentylamino, cyclohexylamino, cycloheptylamino, and the like);
" (C₃₋₈ cycloalkyl C₁₋₄ alkyl) amino" (e.g., cyclopropylmethylamino, cyclobutylmethylamino, cyclopentylmethylamino, cyclohexylmethylamino, cycloheptylmethylamino, and the like);
"(C₃₋₈ cycloalkoxycarbonyl)amino" (e.g., cyclopropoxycarbonylamino, cyclobutoxycarbonylamino, cyclopentoxycarbonylamino, cyclohexyloxycarbonylamino, cycloheptyloxycarbonylamino, and the like);
"(C₁₋₄ alkylcarbonyl)amino" (e.g., methylcarbonylamino, ethylcarbonylamino, propylcarbonylamino, 1-methylpropylcarbonylamino, 2-methylpropylcarbonylamino, butylcarbonylamino, 2,2-dimethylethylcarbonylamino, and the like);
"(C₁₋₄ alkyloxycarbonyl)amino" (e.g., methoxycarbonylamino, ethoxycarbonylamino, propoxycarbonylamino, 1-methylpropoxycarbonylamino, 2-methylpropoxycarbonylamino, butoxycarbonylamino, 2,2-dimethylethoxycarbonylamino, and the like);
"C₅₋₁₀ arylamino" (e.g., phenylamino, 1-naphthylamino, 2-naphthylamino, and the like);
"C₇₋₁₄ aralkylamino" (e.g., benzylamino, 1-naphthylmethylamino, 2-naphthylmethylamino, and the like);
"C₆₋₁₀ arylcarbonylamino" (e.g., phenylcarbonylamino, 1-naphthylcarbonylamino, 2-naphthylcarbonylamino, and the like);
"C₆₋₁₀ aryloxycarbonylamino" (e.g., phenoxycarbonylamino, 1-naphthoxycarbonylamino, 2-naphthoxycarbonylamino, and the like;
"3- to 8-membered heterocyclylamino" (e.g., tetrahydropyranylamino, tetrahydropyridinylamino, pyrrolidinylamino, oxopyrrolidinylamino, tetrahydrofuranylamino, piperidinylamino, and the like);
"(3- to 8-membered heterocyclyl C₁₋₄ alkyl)amino" (e.g., tetrahydropyranylmethylamino, tetrahydropyridinylmethylamino, pyrrolidinylmethylamino, oxopyrrolidinylmethylamino, tetrahydrofuranylmethylamino, piperidinylmethylamino, piperazinylmethylamino, morpholinylmethylamino, and the like);
"3- to 8-membered heterocyclylcarbonylamino" (e.g., tetrahydropyranylcarbonylamino, tetrahydropyridinylcarbonylamino, pyrrolidinylcarbonylamino, oxopyrrolidinylcarbonylamino, tetrahydrofuranylcarbonylamino, piperidinylcarbonylamino, and the like);
"3- to 8-membered heterocyclyloxycarbonylamino" (e.g., tetrahydropyranyloxycarbonylamino, tetrahydropyridinyloxycarbonylamino, pyrrolidinyloxycarbonylamino, oxopyrrolidinyloxycarbonylamino, tetrahydrofuranyloxycarbonylamino, piperidinyloxycarbonylamino, and the like);
"(5- or 6-membered heteroaryl)amino" (e.g., pyrrolylamino, thienylamino, furylamino, oxazolylamino, thiazolylamino, isoxazolylamino, isothiazolylamino, imidazolylamino, pyrazolylamino, triazolylamino, oxadiazolylamino, thiadiazolylamino, tetrazolylamino, pyridylamino, pyrazylamino, pyrimidylamino, pyridazylamino, triazylamino, and the like);
"(5- or 6-membered heteroaryl C₁₋₄ alkyl) amino" (e.g., pyrrolylmethylamino, thienylmethylamino, furylmethylamino, oxazolylmethylamino, thiazolylmethylamino, isoxazolylmethylamino, isothiazolylmethylamino, imidazolylmethylamino, pyrazolylmethylamino, triazolylmethylamino, oxadiazolylmethylamino, thiadiazolylmethylamino, tetrazolylmethylamino, pyridylmethylamino, pyrazylmethylamino, pyrimidylmethylamino, pyridazylmethylamino, triazylmethylamino, and the like); and the like.

Specific examples of "di-substituted amino" include:
"di-C₁₋₆ alkylamino" (e.g., dimethylamino, diethylamino, dipropylamino, di-1-methylethylamino, dibutylamino, di-2-methylpropylamino, di-1-methylpropylamino, di-1,1-dimethylethylamino, and the like);
"N-(C₁₋₆ alkyl)-N-(C₃₋₁₀ cycloalkyl) amino" (e.g., methylcyclopropylamino, methylcyclobutylamino, methylcyclopentylamino, methylcyclohexylamino, methylcycloheptylamino, and the like);
"N-(C₁₋₆ alkyl)-N-(3- to 8-membered heterocyclyl)amino" (e.g., methyltetrahydropyranylamino, methyltetrahydropyridinylamino, methylpyrrolidinylamino, methyloxopyrrolidinylamino, methyltetrahydrofuranylamino, methylpiperidinylamino, and the like); and the like.

An "aminocarbonyl group" refers to a carbonyl group to which the "amino group" described above is bound. In this regard, "amino" refers to unsubstituted amino, mono-substituted amino, di-substituted amino, or 3- to 12-membered cyclic amino. Specific examples thereof include a methylaminocarbonyl group, cyclopropylaminocarbonyl group, dimethylaminocarbonyl group, dicyclopropylaminocarbonyl group, and the like.

An "amino sulfinyl group" refers to a sulfinyl group to which the "amino group" described above is bound. In this regard, "amino" refers to unsubstituted amino, mono-substituted amino, di-substituted amino, or 3- to 12-membered cyclic amino. Specific examples thereof include a methylaminosulfinyl group, cyclopropylaminosulfinyl group, dimethylaminosulfinyl group, dicyclopropylaminosulfinyl group, and the like.

An "aminosulfonyl group" refers to a sulfonyl group to which the "amino group" described above is bound. In this regard, "amino" refers to unsubstituted amino, mono-substituted amino, di-substituted amino, or 3- to 12-membered cyclic amino. Specific examples thereof include a methylaminosulfonyl group, cyclopropylaminosulfonyl group, dimethylaminosulfonyl group, dicyclopropylaminosulfonyl group, and the like.

The "5- to 12-membered monocyclic or polycyclic heterocyclyl" portion of a "5- to 12-membered monocyclic or polycyclic heterocyclylthio group", "5- to 12-membered monocyclic or polycyclic heterocyclyloxycarbonyl group", "5-to 12-membered monocyclic or polycyclic heterocyclylsulfinyl group", "5- to 12-membered monocyclic or polycyclic heterocyclylsulfonyl group", and "5- to 12-membered monocyclic or polycyclic heterocyclylcarbonyl group" is defined the same as the "5- to 12-membered monocyclic or polycyclic heterocyclic group" described above. It is preferably a 3- to 10-membered group, more preferably a 3- to 8-membered group, and still more preferably a 3- to 6-membered group. Specific examples of "5- to 12-membered monocyclic or polycyclic heterocyclylthio group" include a pyridylthio group and the like. Specific examples of "5- to 12-membered monocyclic or polycyclic heterocyclyloxycarbonyl group" include a pyridyloxycarbonyl group and the like. Specific examples of "5- to 12-membered monocyclic or polycyclic heterocyclylsulfinyl group" include a pyridylsufinyl group and the like. Specific examples of "5-to 12-membered monocyclic or polycyclic heterocyclylsulfonyl group" include a pyridylsulfonyl group and the like. Specific examples of "5- to 12-membered monocyclic or polycyclic heterocyclylcarbonyl group" include a pyridylcarbonyl group and the like.

A "3- to 12-membered cyclic amino group" refers to a 3-to 12-membered cyclic amino group where the "group" has the point of attachment directly at a nitrogen atom on the ring, including those having a partially crosslinked structure. The group is preferably 3- to 8-membered, still more preferably 3- to 7-membered, and most preferably 3- to 6-membered. Specific examples thereof include the groups set forth below. The group also includes a cyclic amino group comprising a partially unsaturated ring.

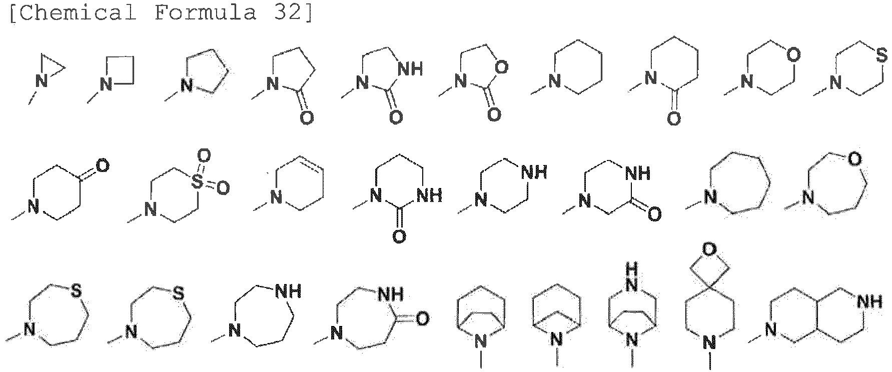

A "3- to 12-membered cyclic amino group" may form a fused ring with a 6-membered aromatic hydrocarbon or 5- or 6-membered heteroaryl. Specific examples thereof include the "groups" set forth below and the like.

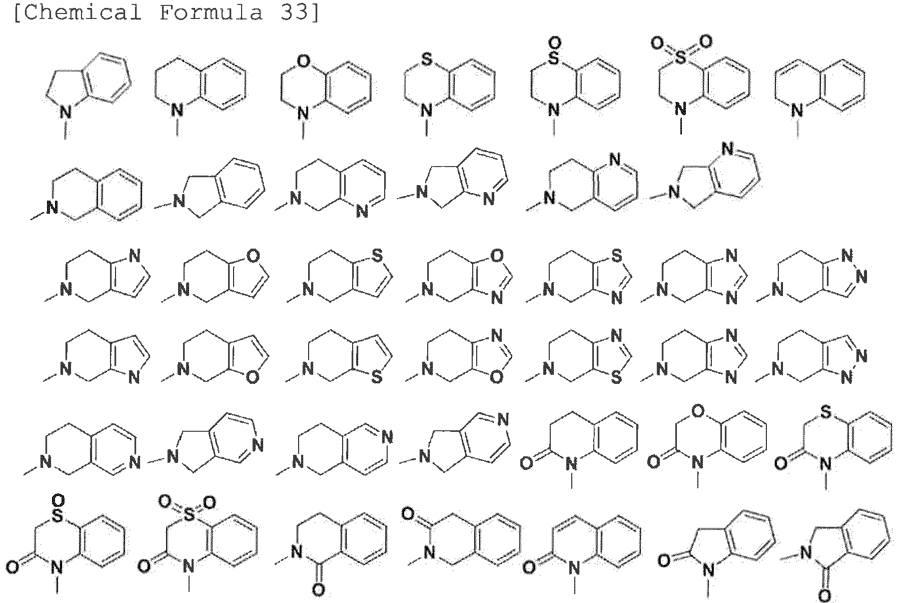

In the compounds of the invention represented by formula (1), preferred A, n, Y, G¹, G², X¹, X², R^{1A}, R^{1B}, R², R^{3A}, R^{3B}, R^{3C}, R^{3D}, R^{3E}, R^{3F}, R^{3G}, R^{3H}, R⁴, R⁵, R⁶, R⁷, and R⁸ are the following, but the technical scope of the invention is not limited to the scope of the compounds listed below. Preferred R^{3A}, R^{3B}, R^{3C}, R^{3D}, R^{3E}, R^{3F}, R^{3G}, and R^{3H} can also be each of preferred R^{a}, R^{b}, R^{c}, and R^{d}.

A is preferably A-1, A-2, A-3, A-4, A-5, A-6, A-7, A-8, A-9, A-10, A-11, A-12, A-13, A-14, A-15, A-16, or A-17.

A is more preferably A-1, A-2, A-3, A-4, A-5, A-6, A-7, A-8, A-9, A-12, A-13, A-14, A-15, A-16, or A-17.

A is still more preferably A-1, A-2, A-3, A-4, A-9, A-12, A-13, A-14, A-15, A-16, or A-17.

A is most preferably A-1, A-2, A-4, A-9, A-12, A-13, or A-14.

X¹ and X² are preferably the same or different, each independently a hydroxyl group, or -O-C(=O)-Y-(C(R^{1A})(R^{1B}))ₙ-NH-R², wherein X¹ and X² are not simultaneously a hydroxyl group.

X¹ and X² are more preferably -O-C(=O)-Y-(C(R^{1A})(R^{1B}))ₙ-NH-R²,

In another preferred embodiment of X¹ and X², one of X¹ and X² is -O-C(=O)-Y-(C(R^{1A})(R^{1B}))ₙ-NH-R², and the other is a hydroxyl group.

n is preferably 2, 3, or 4, more preferably 2 or 3. n is still more preferably 2. In another more preferred embodiment, n is 3.

Y is preferably an oxygen atom or NR⁴. Y is more preferably NR⁴. In another preferred embodiment, Y is an oxygen atom.

G¹ and G² are preferably the same or different, each independently a benzene ring, or a 5- or 6-membered aromatic ring comprising, as a constituent atom, 1 to 3 heteroatoms consisting of O, S and N. G¹ and G² are more preferably the same or different, each independently a benzene ring, a furan ring, or a thiophene group. G¹ and G² are still more preferably a benzene ring.

R^{1A} and R^{1B} are preferably the same or different, each independently a hydrogen atom, -CO₂R⁵, or a C₁₋₆ alkyl group optionally substituted with 1 to 2 substituents selected from the group consisting of a carboxyl group, -CO₂R⁵, -CONR⁵R⁶, - SO₂R⁵, -SO₂NR⁵R⁶, -OCO₂R⁵, -OCONR⁵R⁶, and -NR⁵CO₂R⁶.

R^{1A} and R^{1B} are more preferably the same or different, each independently a hydrogen atom, -CO₂R⁵, or a C₁₋₆ alkyl group optionally substituted with 1 to 2 substituents selected from the group consisting of a carboxyl group, -CO₂R⁵, -CONR⁵R⁶, and -SO₂NR⁵R⁶.

R^{1A} and R^{1B} are still more preferably the same or different, each independently a hydrogen atom, -CO₂R⁵, or a C₁₋₆ alkyl group.

R^{1A} and R^{1B} are most preferably hydrogen atoms.

R² is preferably a hydrogen atom, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, - NR⁵R⁶, -CO₂R⁵, -CONR⁵R⁶, -SO₂R⁵, -SO₂NR⁵R⁶, -OCO₂R⁵, -OCONR⁵R⁶, and -NR⁵CO₂R⁶.

R² is more preferably a hydrogen atom, or a C₁₋₆ alkyl group optionally substituted with 1 to 2 substituents selected from the group consisting of a carboxyl group, -CO₂R⁵, -CONR⁵R⁶, -SO₂R⁵, -SO₂NR⁵R⁶, -OCO₂R⁵, -OCONR⁵R⁶, and -NR⁵CO₂R⁶.

R² is still more preferably a hydrogen atom, or a C₁₋₆ alkyl group optionally substituted with 1 to 2 substituents selected from the group consisting of a carboxyl group, -CO₂R⁵, -CONR⁵R⁶, and -SO₂NR⁵R⁶.

R² is most preferably a hydrogen atom, or a C₁₋₆ alkyl group optionally substituted with 1 to 2 carboxyl groups.

R^{3A}, R^{3B}, R^{3C}, R^{3D}, R^{3E}, R^{3F}, R^{3G}, and R^{3H} are preferably null, or the same or different, each independently:
(1) a hydrogen atom;
(2) a hydroxyl group;
(3) an optionally substituted 3- to 12-membered cyclic amino group, wherein the cyclic amino group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, and a 5- to 10-membered heteroaryl group;
(4) an optionally substituted C₁₋₃₀ alkyl group, wherein the alkyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(5) an optionally substituted C₃₋₁₀ cycloalkyl group, wherein the cycloalkyl group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(6) an optionally substituted C₂₋₃₀ alkenyl group, wherein the alkenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
(7) an optionally substituted C₁₋₂₀ alkoxy group, wherein the alkoxy group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, and a C₁₋₁₀ alkoxycarbonyl group;
(8) an optionally substituted C₁₋₂₀ alkylcarbonyloxy group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(9) an optionally substituted 5- to 12-membered monocyclic or polycyclic heterocyclic group, wherein the heterocyclic group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(10) an optionally substituted phenyl group, wherein the phenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(11) an optionally substituted C₁₋₂₀ alkylcarbonyl group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation; or
(12) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₃₋₁₀ cycloalkane, wherein the cycloalkane is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
(2') an optionally substituted 3- to 12-membered monocyclic or polycyclic heterocycle, wherein the heterocycle is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
wherein the C₃₋₁₀ cycloalkane or the 3- to 12-membered monocyclic or polycyclic heterocycle is fused to the aromatic ring to form a fused ring.

R^{3A}, R^{3B}, R^{3C}, R^{3D}, R^{3E}, R^{3F}, R^{3G}, and R^{3H} are more preferably null, or the same or different, each independently:
(1) a hydrogen atom;
(2) a hydroxyl group;
(3) an optionally substituted C₁₋₃₀ alkyl group, wherein the alkyl group is optionally substituted with 1 to 3 groups selected from the group consisting of a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(5) an optionally substituted C₂₋₃₀ alkenyl group, wherein the alkenyl group is optionally substituted with 1 to 3 groups selected from the group consisting of a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
(6) a C₁₋₂₀ alkoxy group;
(7) a C₁₋₂₀ alkylcarbonyloxy group;
(8) an optionally substituted 3- to 12-membered monocyclic or polycyclic heterocyclic group, wherein the heterocyclic group is optionally substituted with 1 to 3 groups selected from the group consisting of a hydroxyl group, a carboxyl group, a C₁₋₁₀ alkyl group, a C₃₋₁₀ cycloalkyl group, and a C₁₋₆ alkoxycarbonyl group;
(9) a phenyl group;
(10) a C₁₋₂₀ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₃₋₁₀ cycloalkane, wherein the cycloalkane is optionally substituted with 1 to 3 groups selected from the group consisting of a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
(2') an optionally substituted 3- to 12-membered monocyclic or polycyclic heterocycle, wherein the heterocycle is optionally substituted with 1 to 3 groups selected from the group consisting of a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
wherein the C₃₋₁₀ cycloalkane or the 3- to 12-membered monocyclic or polycyclic heterocycle is fused to the aromatic ring to form a fused ring.

R^{3A}, R^{3B}, R^{3C}, R^{3D}, R^{3E}, R^{3F}, R^{3G}, and R^{3H} are still more preferably null, or the same or different, each independently:
(1) a hydrogen atom;
(2) a hydroxyl group;
(3) an optionally substituted C₁₋₃₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
(5) an optionally substituted C₂₋₃₀ alkenyl group, wherein the alkenyl group is optionally substituted with 1 to 3 groups selected from the group consisting of a carboxyl group and a pyridyl group;
(6) a C₁₋₁₂ alkoxy group;
(7) a C₁₋₁₂ alkylcarbonyloxy group;
(8) a 5- to 8-membered monocyclic or polycyclic heterocyclic group, wherein the heterocyclic group is optionally substituted with 1 to 3 groups selected from the group consisting of a hydroxyl group and a C₁₋₁₀ alkyl group;
(9) a phenyl group;
(10) a C₁₋₁₂ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₅₋₈ cycloalkane, wherein the cycloalkane is optionally substituted with 1 to 3 groups selected from the group consisting of a C₁₋₆ alkyl group and an optionally chloro-substituted C₆₋₁₀ aryl group; or
(2') an optionally substituted 5- to 8-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group,
wherein the C₅₋₈ cycloalkane or the 5- to 8-membered monocyclic or polycyclic heterocycle is fused to the aromatic ring to form a fused ring.

R^{3A}, R^{3B}, R^{3C}, R^{3D}, R^{3E}, R^{3F}, R^{3G}, and R^{3H} are most preferably null, or the same or different, each independently:
(1) a hydrogen atom;
(2) a hydroxyl group;
(3) an optionally substituted C₁₋₂₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
(5) an optionally substituted C₂₋₂₀ alkenyl group, wherein the alkenyl group is optionally substituted with 1 to 3 groups selected from the group consisting of a carboxyl group and a pyridyl group;
(6) a C₁₋₁₂ alkoxy group;
(7) a C₁₋₁₂ alkylcarbonyloxy group;
(8) a 5- to 6-membered monocyclic or polycyclic heterocyclic group, wherein the heterocyclic group is optionally substituted with 1 to 3 groups selected from the group consisting of a hydroxyl group and a C₁₋₁₀ alkyl group;
(9) a phenyl group;
(10) a C₁₋₁₂ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₅₋₆ cycloalkane, wherein the cycloalkane is optionally substituted with 1 to 3 groups selected from the group consisting of a C₁₋₆ alkyl group and an optionally chloro-substituted C₆₋₁₀ aryl group; or
(2') an optionally substituted 5- to 6-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group,
wherein the C₅₋₆ cycloalkane or the 5- to 6-membered monocyclic or polycyclic heterocycle is fused to the aromatic ring to form a fused ring.

R⁴ is preferably a hydrogen atom, or a C₁₋₆ alkyl group optionally substituted with 1 to 2 substituents selected from the group consisting of a carboxyl group, a C₁₋₆ alkoxy group, -NR⁵R⁶, -CO₂R⁵, -CONR⁵R⁶, -OCO₂R⁵, -OCONR⁵R⁶, and -NR⁵CO₂R⁶.

R⁴ is more preferably a hydrogen atom, or a C₁₋₆ alkyl group optionally substituted with a carboxyl group.

R⁴ is still more preferably a hydrogen atom, or a C₁₋₆ alkyl group.

R⁴ is the most preferably a hydrogen atom, or a C₁₋₄ alkyl group.

R⁵ and R⁶ are preferably the same or different, each independently a hydrogen atom, or a C₁₋₆ alkyl group optionally substituted with 1 to 2 groups selected from the group consisting of a fluorine atom and a carboxyl group, or R⁵ and R⁶, together with the nitrogen atom to which they are attached, may form a 4- to 8-membered cyclic amino group.

R⁵ and R⁶ are more preferably the same or different, each independently a hydrogen atom, or a C₁₋₆ alkyl group optionally substituted with 1 to 2 carboxyl groups, or R⁵ and R⁶, together with the nitrogen atom to which they are attached, may form a 4- to 8-membered cyclic amino group.

R⁵ and R⁶ are still more preferably the same or different, each independently a hydrogen atom, or a C₁₋₆ alkyl group, or R⁵ and R⁶, together with the nitrogen atom to which they are attached, may form a 5- to 6-membered cyclic amino group.

R⁵ and R⁶ are most preferably the same or different, each independently a hydrogen atom, or a C₁₋₆ alkyl group.

In another preferred embodiment, R⁵ and R⁶ are hydrogen atoms.

In still another preferred embodiment, R⁵ and R⁶ are C₁₋₆ alkyl groups.

R⁷ and R⁸ are preferably the same or different, each independently a hydrogen atom, or a C₁₋₆ alkyl group optionally substituted with 1 to 2 groups selected from the group consisting of a fluorine atom and a carboxyl group, or R⁷ and R⁸, together with the nitrogen atom to which they are attached, may form a 4- to 8-membered cyclic amino group.

R⁷ and R⁸ are more preferably the same or different, each independently a hydrogen atom, or a C₁₋₆ alkyl group optionally substituted with 1 to 2 groups selected from the group consisting of a carboxyl group, or R⁷ and R⁸, together with the nitrogen atom to which they are attached, may form a 4- to 8-membered cyclic amino group.

R⁷ and R⁸ are still preferably the same or different, each independently a hydrogen atom or a C₁₋₆ alkyl group.

R⁷ and R⁸ are most preferably C₁₋₆ alkyl groups.

Among the compounds represented by formula (1), preferred compounds are the following compounds or pharmaceutically acceptable salt thereof.

Among the compounds represented by formula (1), preferred embodiments include the following (A):
(A) a compound or a pharmaceutically acceptable salt thereof, wherein
   A is A-1, A-2, A-3, A-4, A-5, A-6, A-7, A-8, A-9, A-10, A-11, A-12, A-13, A-14, A-15, A-16, or A-17;
   X¹ and X² are the same or different, each independently a hydroxyl group, or -O-C(=O)-Y-(C(R^{1A})(R^{1B}))ₙ-NH-R², wherein X¹ and X² are not simultaneously a hydroxyl group;
   n is the same or different, each independently 2, 3, or 4;
   Y is an oxygen atom or NR⁴;
   G¹ and G² are the same or different, each independently a benzene ring, or a 5- or 6-membered aromatic ring comprising, as a constituent atom, 1 to 3 heteroatoms consisting of O, S and N;
   R^{1A} and R^{1B} are the same or different, each independently a hydrogen atom, -CO₂R⁵, or a C₁₋₆ alkyl group optionally substituted with 1 to 2 substituents selected from the group consisting of a carboxyl group -CO₂R⁵, -CONR⁵R⁶, -SO₂R⁵, - SO₂NR⁵R⁶, -OCO₂R⁵, -OCONR⁵R⁶, and -NR⁵CO₂R⁶;
   R² is a hydrogen atom, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
   R^{3A}, R^{3B}, R^{3C}, R^{3D}, R^{3E}, R^{3F}, R^{3G}, and R^{3H} are null, or the same or different, each independently:
   (1) a hydrogen atom;
   (2) a hydroxyl group;
   (3) an optionally substituted 3- to 12-membered cyclic amino group, wherein the cyclic amino group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, and a 5- to 10-membered heteroaryl group;
   (4) an optionally substituted C₁₋₃₀ alkyl group, wherein the alkyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (5) an optionally substituted C₃₋₁₀ cycloalkyl group, wherein the cycloalkyl group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (6) an optionally substituted C₂₋₃₀ alkenyl group, wherein the alkenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
   (7) an optionally substituted C₁₋₂₀ alkoxy group, wherein the alkoxy group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, and a C₁₋₁₀ alkoxycarbonyl group;
   (8) an optionally substituted C₁₋₂₀ alkylcarbonyloxy group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (9) an optionally substituted 3- to 12-membered monocyclic or polycyclic heterocyclic group, wherein the heterocyclic group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (10) an optionally substituted phenyl group, wherein the phenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
   (11) an optionally substituted C₁₋₂₀ alkylcarbonyl group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation; or
   (12) a carboxyl group, or
   R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
   (1') an optionally substituted C₃₋₁₀ cycloalkane, wherein the cycloalkane is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
   (2') an optionally substituted 3- to 12-membered monocyclic or polycyclic heterocycle, wherein the heterocycle is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation, wherein the C₃₋₁₀ cycloalkane or the 3- to 12-membered monocyclic or polycyclic heterocycle is fused to the aromatic ring to form a fused ring;
   R⁴ is a hydrogen atom, or a C₁₋₆ alkyl group optionally substituted with 1 to 2 substituents selected from the group consisting of a carboxyl group, a C₁₋₆ alkoxy group, -NR⁵R⁶, - CO₂R⁵, -CONR⁵R⁶, -OCO₂R⁵, -OCONR⁵R⁶, and -NR⁵CO₂R⁶; and
   R⁵ and R⁶ are the same or different, each independently a hydrogen atom, or a C₁₋₆ alkyl group optionally substituted with 1 to 2 groups selected from the group consisting of a fluorine atom and a carboxyl group, or R⁵ and R⁶, together with the nitrogen atom to which they are attached, may form a 4- to 8-membered cyclic amino group.

Among the compounds represented by formula (1), more preferred embodiments include the following (B):
(B) a compound or a pharmaceutically acceptable salt thereof, wherein
A is A-1, A-2, A-3, A-4, A-5, A-6, A-7, A-8, A-9, A-12, A-13, A-14, A-15, A-16, or A-17;
X¹ and X² are the same or different, each independently a hydroxyl group, or -O-C(=O)-Y-(C(R^{1A})(R^{1B}))ₙ-NH-R², wherein X¹ and X² are not simultaneously a hydroxyl group;
n is the same or different, each independently 2, 3, or 4;
Y is an oxygen atom or NR⁴;
G¹ and G² are the same or different, each independently a benzene ring, a furan ring, or a thiophene group;
R^{1A} and R^{1B} are the same or different, each independently a hydrogen atom, -CO₂R⁵, or a C₁₋₆ alkyl group optionally substituted with 1 to 2 substituents selected from the group consisting of a carboxyl group -CO₂R⁵, -CONR⁵R⁶, and -SO₂NR⁵R⁶;
R² is a hydrogen atom, or a C₁₋₆ alkyl group optionally substituted with 1 to 2 substituents selected from the group consisting of a carboxyl group, -CO₂R⁵, -CONR⁵R⁶, -SO₂R⁵, - SO₂NR⁵R⁶, -OCO₂R⁵, -OCONR⁵R⁶, and -NR⁵CO₂R⁶;
R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) an optionally substituted C₁₋₃₀ alkyl group, wherein the alkyl group is optionally substituted with 1 to 3 groups selected from the group consisting of a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(5) an optionally substituted C₂₋₃₀ alkenyl group, wherein the alkenyl group is optionally substituted with 1 to 3 groups selected from the group consisting of a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
(6) a C₁₋₂₀ alkoxy group;
(7) a C₁₋₂₀ alkylcarbonyloxy group;
(8) an optionally substituted 3- to 12-membered monocyclic or polycyclic heterocyclic group, wherein the heterocyclic group is optionally substituted with 1 to 3 groups selected from the group consisting of a hydroxyl group, a carboxyl group, a C₁₋₁₀ alkyl group, a C₃₋₁₀ cycloalkyl group, and a C₁₋₆ alkoxycarbonyl group;
(9) a phenyl group;
(10) a C₁₋₂₀ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₃₋₁₀ cycloalkane, wherein the cycloalkane is optionally substituted with 1 to 3 groups selected from the group consisting of a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
(2') an optionally substituted 3- to 12-membered monocyclic or polycyclic heterocycle, wherein the heterocycle is optionally substituted with 1 to 3 groups selected from the group consisting of a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation, wherein the C₃₋₁₀ cycloalkane or the 3- to 12-membered monocyclic or polycyclic heterocycle is fused to the aromatic ring to form a fused ring;
R⁴ is a hydrogen atom, or a C₁₋₆ alkyl group optionally substituted with a carboxyl group; and
R⁵ and R⁶ are the same or different, each independently a hydrogen atom, or a C₁₋₆ alkyl group optionally substituted with 1 to 2 carboxyl groups, or R⁵ and R⁶, together with the nitrogen atom to which they are attached, may form a 4- to 8-membered cyclic amino group.

Among the compounds represented by formula (1), still more preferred embodiments include the following (C):
(C) a compound or a pharmaceutically acceptable salt thereof, wherein
A is A-1, A-2, A-3, A-4, A-9, A-12, A-13, A-14, A-15, A-16, or A-17;
X¹ and X² are the same or different, each independently a hydroxyl group, or -O-C(=O)-Y-(C(R^{1A})(R^{1B}))ₙ-NH-R², wherein X¹ and X² are not simultaneously a hydroxyl group;
n is the same or different, each independently 2, 3, or 4;
Y is NR⁴;
G¹ and G² are the same or different, each independently a benzene ring, a furan ring, or a thiophene group;
R^{1A} and R^{1B} are the same or different, each independently a hydrogen atom, -CO₂R⁵, or a C₁₋₆ alkyl group;
R² is a hydrogen atom, or a C₁₋₆ alkyl group optionally substituted with 1 to 2 substituents selected from the group consisting of a carboxyl group, -CO₂R⁵, -CONR⁵R⁶, and -SO₂NR⁵R⁶;
R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) an optionally substituted C₁₋₃₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
(5) an optionally substituted C₂₋₃₀ alkenyl group, wherein the alkenyl group is optionally substituted with 1 to 3 groups selected from the group consisting of a carboxyl group and a pyridyl group;
(6) a C₁₋₁₂ alkoxy group;
(7) a C₁₋₁₂ alkylcarbonyloxy group;
(8) a 5- to 8-membered monocyclic or polycyclic heterocyclic group, wherein the heterocyclic group is optionally substituted with 1 to 3 groups selected from the group consisting of a hydroxyl group and a C₁₋₁₀ alkyl group;
(9) a phenyl group;
(10) a C₁₋₁₂ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₅₋₈ cycloalkane, wherein the cycloalkane is optionally substituted with 1 to 3 groups selected from the group consisting of a C₁₋₆ alkyl group and an optionally chloro-substituted C₆₋₁₀ aryl group; or
(2') an optionally substituted 5- to 8-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group, wherein the C₅₋₈ cycloalkane or the 5- to 8-membered monocyclic or polycyclic heterocycle is fused to the aromatic ring to form a fused ring;
R⁴ is a hydrogen atom, or a C₁₋₆ alkyl group; and
R⁵ and R⁶ are the same or different, each independently a hydrogen atom, or a C₁₋₆ alkyl group, or R⁵ and R⁶, together with the nitrogen atom to which they are attached, may form a 5- to 6-membered cyclic amino group.

Among the compounds represented by formula (1), the most preferred embodiments include the following (D):
(D) a compound or a pharmaceutically acceptable salt thereof, wherein
A is A-1, A-2, A-4, A-9, A-12, A-13, or A-14;
X¹ and X² are the same or different, each independently a hydroxyl group, or -O-C(=O)-Y-(C(R^{1A})(R^{1B}))ₙ-NH-R², wherein X¹ and X² are not simultaneously a hydroxyl group;
n is the same or different, each independently 2 or 3;
Y is NR⁴;
G¹ and G² are benzene rings;
R^{1A} and R^{1B} are a hydrogen atom or -CO₂R⁵;
R² is a hydrogen atom, or a C₁₋₆ alkyl group optionally substituted with 1 to 2 carboxyl groups;
R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) an optionally substituted C₁₋₂₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
(5) an optionally substituted C₂₋₂₀ alkenyl group, wherein the alkenyl group is optionally substituted with 1 to 3 groups selected from the group consisting of a carboxyl group and a pyridyl group;
(6) a C₁₋₁₂ alkoxy group;
(7) a C₁₋₁₂ alkylcarbonyloxy group;
(8) a 5- to 6-membered monocyclic or polycyclic heterocyclic group, wherein the heterocyclic group is optionally substituted with 1 to 3 groups selected from the group consisting of a hydroxyl group and a C₁₋₁₀ alkyl group;
(9) a phenyl group;
(10) a C₁₋₁₂ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1'.) an optionally substituted C₅₋₆ cycloalkane, wherein the cycloalkane is optionally substituted with 1 to 3 groups selected from the group consisting of a C₁₋₆ alkyl group and an optionally chloro-substituted C₆₋₁₀ aryl group; or
(2') an optionally substituted 5- to 6-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group, wherein the C₅₋₆ cycloalkane or the 5- to 6-membered monocyclic or polycyclic heterocycle is fused to the aromatic ring to form a fused ring;
R⁴ is a hydrogen atom, or a C₁₋₄ alkyl group; and
R⁵ is a hydrogen atom, or a C₁₋₆ alkyl group optionally substituted with 1 to 2 groups selected from the group consisting of a fluorine atom and a carboxyl group.

Among the compounds represented by formula (1), another preferred embodiment includes the following (E):
(E) a compound or a pharmaceutically acceptable salt thereof, wherein
A is A-13;
X¹ and X² are -O-C(=O)-Y-(C(R^{1A})(R^{1B}))ₙ-NH-R²;
n is the same or different, each independently 2, 3, or 4;
Y is an oxygen atom or NR⁴;
ring G¹ is a benzene ring;
R^{1A} and R^{1B} are hydrogen atoms;
R² is a hydrogen atom, or a C₁₋₆ alkyl group optionally substituted with 1 to 2 carboxyl groups;
R^{3A}, R^{3B}, R^{3C}, R^{3D}, R^{3E}, and R^{3F} are the same or different, each independently:
(1) a hydrogen atom;
(2) a hydroxyl group;
(3) a C₁₋₂₀ alkyl group;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group; or
(5) a C₂₋₂₀ alkenyl group; and
R⁴ is a hydrogen atom.

Among the compounds represented by formula (1), still another more preferred embodiment includes the following (F):
(F) a compound or a pharmaceutically acceptable salt thereof, wherein
A is A-12;
X¹ and X² are -O-C(=O)-Y-(C(R^{1A})(R^{1B}))ₙ-NH-R²;
n is the same or different, each independently 2, 3, or 4;
Y is an oxygen atom or NR⁴;
R^{1A} and R^{1B} are a hydrogen atom or -CO₂R⁵;
R² is a hydrogen atom, or a C₁₋₆ alkyl group optionally substituted with 1 to 2 carboxyl groups;
R^{3A}, R^{3B}, R^{3C}, and R^{3D} are the same or different, each independently:
(1) a hydrogen atom;
(2) an optionally substituted C₁₋₁₂ alkyl group, wherein the alkyl group is optionally substituted with 1 to 3 groups selected from the group consisting of a hydroxyl group, a carboxyl group, a phenyl group, a pyridyl group, a C₁₋₆ alkoxycarbonyl group, and a triphenylphosphonium cation;
(3) an optionally substituted C₂₋₂₀ alkenyl group, wherein the alkenyl group is optionally substituted with 1 to 3 groups selected from the group consisting of a carboxyl group and a pyridyl group;
(4) a C₁₋₆ alkoxy group; or
(5) a phenyl group; and
R⁴ is a hydrogen atom.

Among the compounds represented by formula (1), still another more preferred embodiment includes the following (G):
(G) a compound or a pharmaceutically acceptable salt thereof, wherein
A is A-14;
X¹ and X² are -O-C(=O)-Y-(C(R^{1A})(R^{1B}))ₙ-NH-R²;
n is the same or different, each independently 2 or 3;
Y is NR⁴;
rings G¹ and G² are the same or different, each independently a benzene ring, a furan ring, or a thiophene group;
R^{1A} and R^{1B} are hydrogen atoms;
R² is a hydrogen atom, or a C₁₋₆ alkyl group optionally substituted with 1 to 2 carboxyl groups;
R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) a C₁₋₆ alkylcarbonyloxy group;
(4) a C₁₋₆ alkylcarbonyl group; or
(5) a carboxyl group; and
R⁴ is a hydrogen atom.

Among the compounds represented by formula (1), still another more preferred embodiment includes the following (H):
(H) a compound or a pharmaceutically acceptable salt thereof, wherein
A is A-2 or A-4;
X¹ and X² are -O-C(=O)-Y-(C(R^{1A})(R^{1B}))ₙ-NH-R²;
n is the same or different, each independently 2 or 3;
Y is NR⁴;
ring G¹ is a benzene ring;
R^{1A} and R^{1B} are hydrogen atoms;
R² is a hydrogen atom, or a C₁₋₆ alkyl group optionally substituted with 1 to 2 carboxyl groups;
R^{3A}, R^{3B}, R^{3C}, R^{3D}, R^{3E}, and R^{3F} are the same or different, each independently:
(1) a hydrogen atom;
(2) a C₁₋₆ alkyl group; or
(3) a C₁₋₆ alkoxy group; or
R^{3A} and R^{3B}, R^{3B} and R^{3C}, R^{3C} and R^{3D}, R^{3D} and R^{3E}, or R^{3E} and R^{3F}, when adjacent to each other on an aromatic ring, together with 2 carbon atoms on the aromatic ring to which they are attached, may form:
(1') cyclohexane optionally substituted with 1 to 3 C₁₋₆ alkyl groups;
(2') tetrahydrofuran optionally substituted with 1 to 3 C₁₋₆ alkyl groups; or
(3') tetrahydropyran optionally substituted with 1 to 3 C₁₋₆ alkyl groups; wherein the cyclohexane, the tetrahydrofuran, or the tetrahydropyran is fused to the aromatic ring to form a structure comprising and 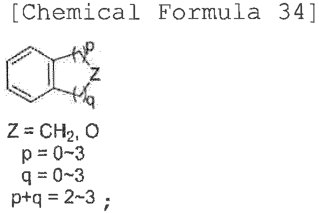 R⁴ is a hydrogen atom.

Among the compounds represented by formula (1), still another more preferred embodiment includes the following (I):
(I) a compound or a pharmaceutically acceptable salt thereof, wherein
A is A-9;
X¹ and X² are the same or different, each independently a hydroxyl group or -O-C(=O)-Y-(C(R^{1A})(R^{1B}))ₙ-NH-R², wherein X¹ and X² are not simultaneously a hydroxyl group;
n is the same or different, each independently 2 or 3;
Y is an oxygen atom or NR⁴;
R^{1A} and R^{1B} are hydrogen atoms;
R² is a hydrogen atom, or a C₁₋₆ alkyl group optionally substituted with 1 to 2 carboxyl groups;
R^{3A}, R^{3B}, R^{3C}, and R^{3D} are the same or different, each independently:
(1) a hydrogen atom; or
(2) a 3- to 12-membered monocyclic or polycyclic heterocyclic group, wherein the heterocyclic group is optionally substituted with 1 to 3 groups selected from the group consisting of a hydroxyl group and a C₁₋₁₀ alkyl group; or
R^{3A} and R^{3B} or R^{3B} and R^{3C}, together with 2 carbon atoms on the aromatic ring to which they are attached, may form a dihydropyranone ring optionally substituted with an optionally chloro-substituted C₆₋₁₀ phenyl group, wherein the dihydropyranone ring is fused to the aromatic ring to form a structure comprising and 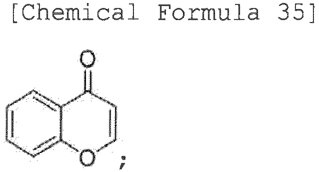 R⁴ is a hydrogen atom, or a C₁₋₄ alkyl group.

Preferred embodiments of the invention encompass the compounds represented by the following formulas (1-A-1a) to (1-A-17c) :
Compounds represented by the following formula (1-A-1a), (1-A-1b), (1-A-1c), (1-A-2a), (1-A-2b), (1-A-2c), (1-A-3a), (1-A-3b), (1-A-3c), (1-A-4a), (1-A-4b), or (1-A-4c), or a pharmaceutically acceptable salt thereof: 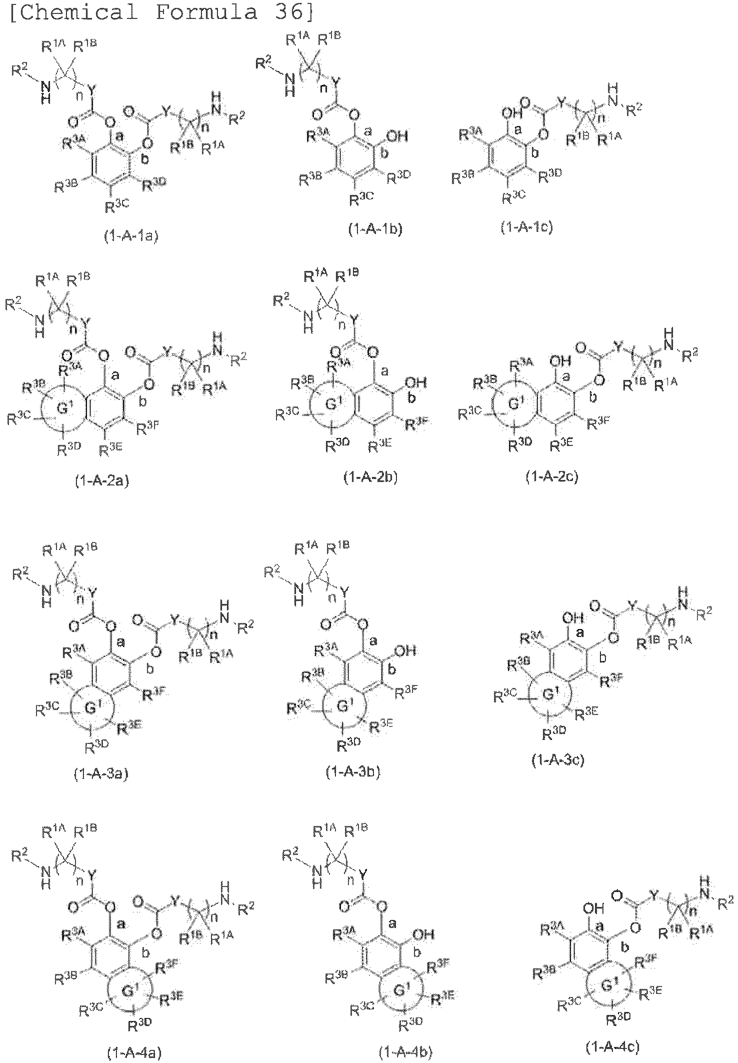 (wherein each symbol is defined the same as item 1.)

Preferred embodiments of each symbol in the compounds represented by formulas (1-A-1a), (1-A-1b), (1-A-1c), (1-A-2a), (1-A-2b), (1-A-2c), (1-A-3a), (1-A-3b), (1-A-3c), (1-A-4a), (1-A-4b), and (1-A-4c) are the same as the preferred embodiments in the compound represented by formula (1).

Compounds represented by the following formula (1-A-5a), (1-A-5b), (1-A-5c), (1-A-6a), (1-A-6b), (1-A-6c), (1-A-7a), (1-A-7b), (1-A-7c), (1-A-8a), (1-A-8b), or (1-A-8c), or a pharmaceutically acceptable salt thereof: 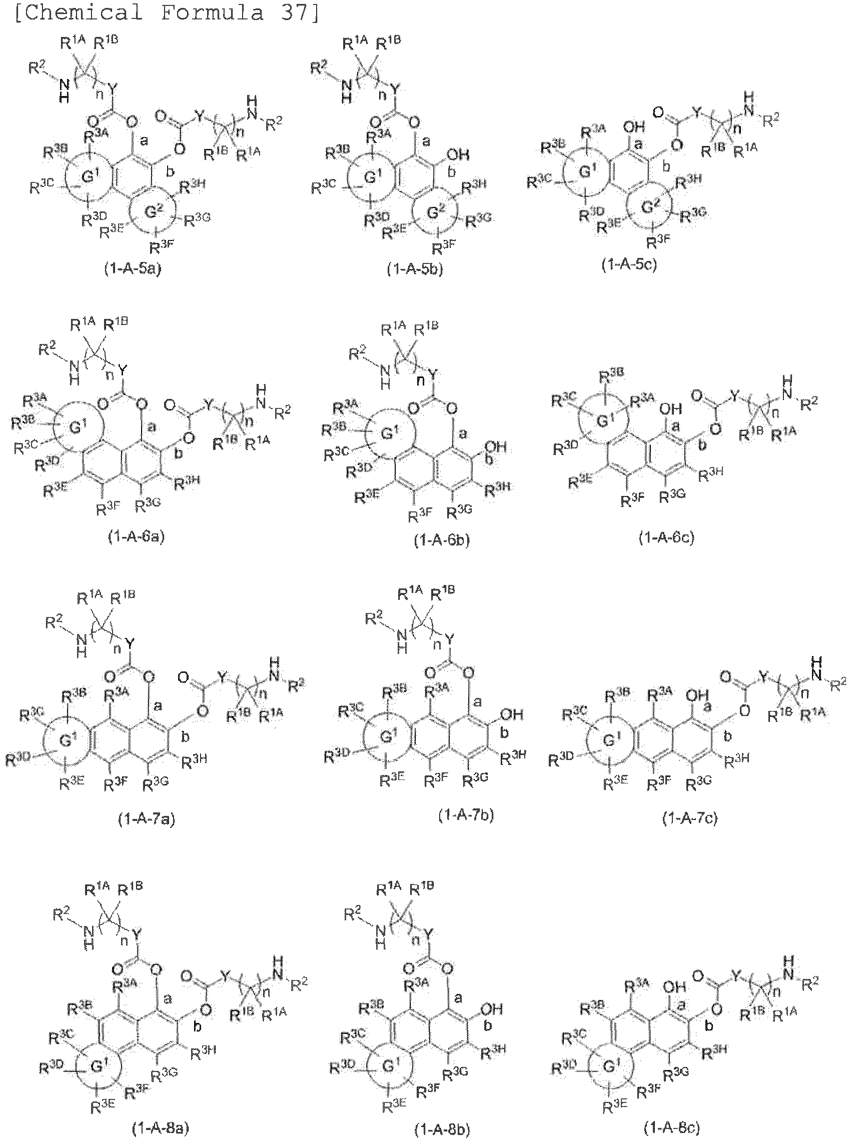 (wherein each symbol is defined the same as item 1.)

Preferred embodiments of each symbol in the compounds represented by formulas (1-A-5a), (1-A-5b), (1-A-5c), (1-A-6a), (1-A-6b), (1-A-6c), (1-A-7a), (1-A-7b), (1-A-7c), (1-A-8a), (1-A-8b), and (1-A-8c) are the same as the preferred embodiments in the compound represented by formula (1).

Compounds represented by the following formula (1-A-9a), (1-A-9b), (1-A-9c), (1-A-10a), (1-A-10b), (1-A-10c), (1-A-11a), (1-A-11b), (1-A-11c), (1-A-12a), (1-A-12b), or (1-A-12c), or a pharmaceutically acceptable salt thereof: 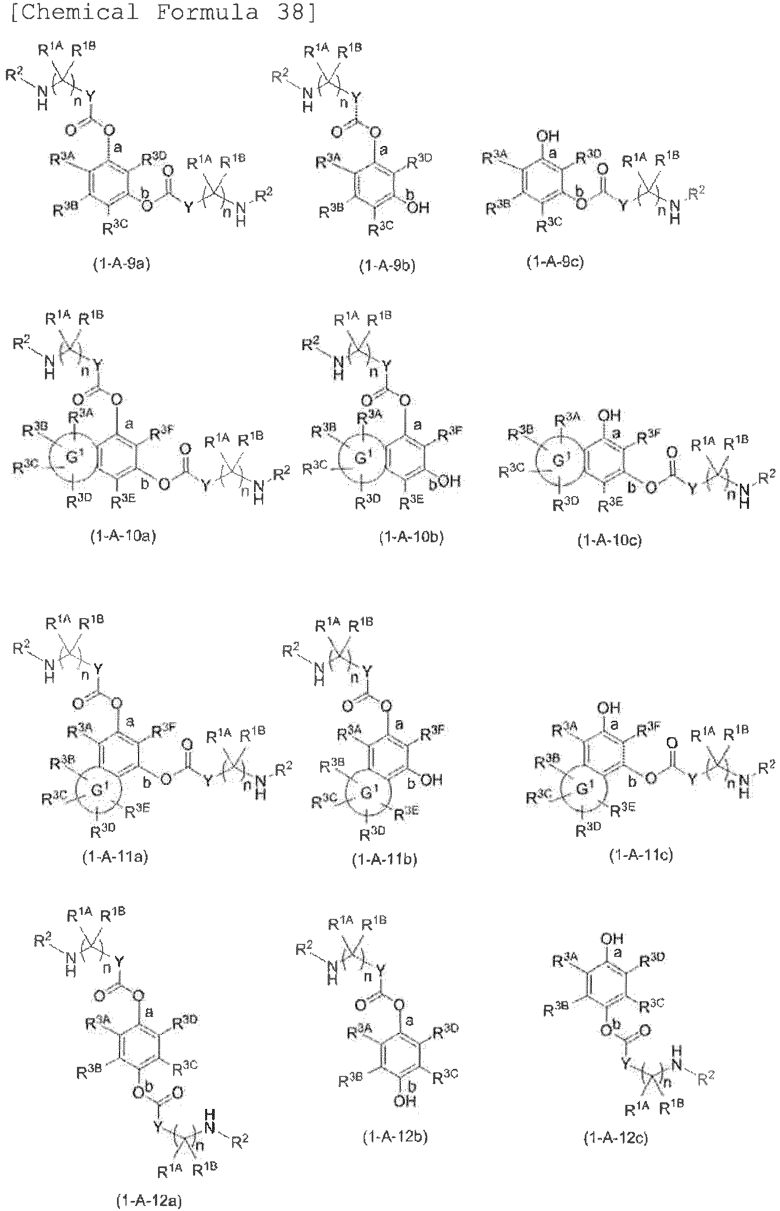 (wherein each symbol is defined the same as item 1.)

Preferred embodiments of each symbol in the compounds represented by formulas (1-A-9a), (1-A-9b), (1-A-9c), (1-A-10a), (1-A-10b), (1-A-10c), (1-A-11a), (1-A-11b), (1-A-11c), (1-A-12a), (1-A-12b), and (1-A-12c) are the same as the preferred embodiments in the compound represented by formula (1) .

Compounds represented by the following formula (1-A-13a), (1-A-13b), (1-A-13c), (1-A-14a), (1-A-14b), (1-A-14c), (1-A-15a), (1-A-15b), (1-A-15c), (1-A-16a), (1-A-16b), (1-A-16c), (1-A-17a), (1-A-17b), or (1-A-17c), or a pharmaceutically acceptable salt thereof: 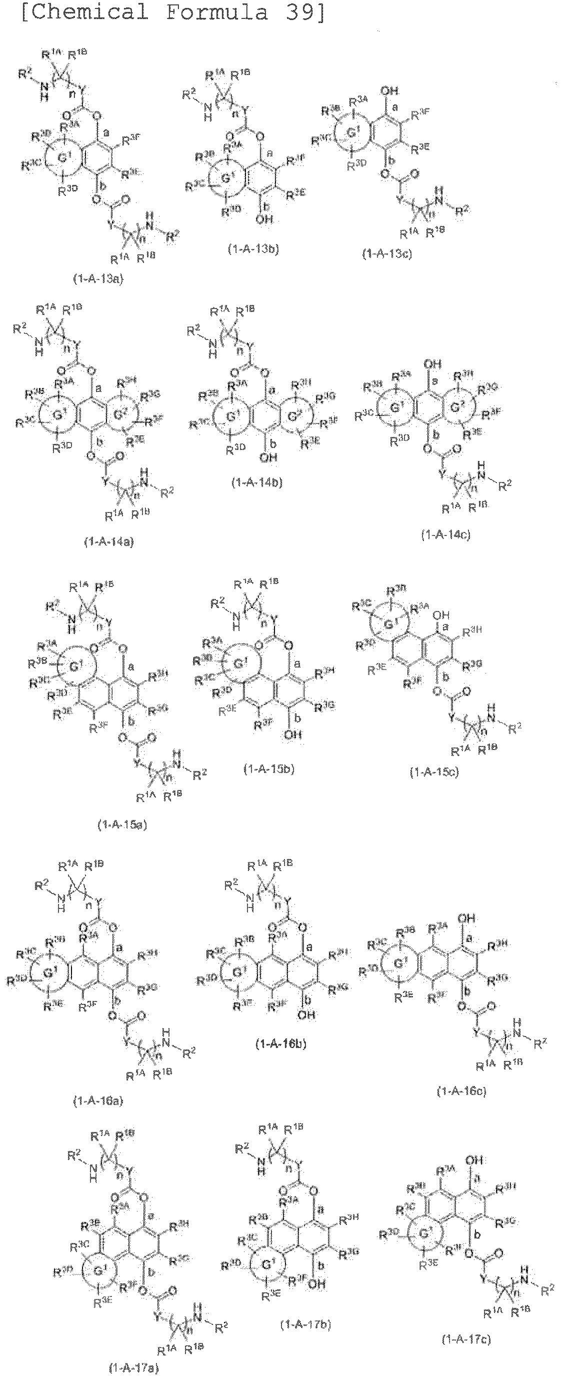 (wherein each symbol is defined the same as item 1.)

Preferred embodiments of each symbol in the compounds represented by formulas (1-A-13a), (1-A-13b), (1-A-13c), (1-A-14a), (1-A-14b), (1-A-14c), (1-A-15a), (1-A-15b), (1-A-15c), (1-A-16a), (1-A-16b), (1-A-16c), (1-A-17a), (1-A-17b), and (1-A-17c) are the same as the preferred embodiments in the compound represented by formula (1).

Examples of "pharmaceutically acceptable salt" include acid addition salts and base addition salts. Examples of acid addition salts include inorganic acid salts such as hydrochloride, hydrobromide, sulfate, hydroiodide, nitrate, and phosphate, and organic acid salts such as citrate, oxalate, phthalate, fumarate, maleate, succinate, malate, acetate, formate, propionate, benzoate, trifluoroacetate, methanesulfonate, benzenesulfonate, p-toluenesulfonate, and camphorsulfonate. Examples of base addition salts include inorganic base salts such as sodium salt, potassium salt, calcium salt, magnesium salt, barium salt, and aluminum salt, and organic base salts such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N-N-dibenzylethylamine. Furthermore, examples of pharmaceutically acceptable salt include salts of a basic or acidic amino acid such as arginine, lysine, ornithine, aspartic acid, and glutamic acid.

Suitable salts and pharmaceutically acceptable salts of starting compounds and target compounds are conventional nontoxic salts. Examples thereof include acid addition salts such as organic acid salts (e.g., acetate, trifluoroacetate, maleate, fumarate, citrate, tartrate, methanesulfonate, benzenesulfonate, formate, para-toluenesulfonate, and the like) and inorganic acid salts (e.g., hydrochloride, hydrobromide, hydroiodide, sulfate, nitrate, phosphate, and the like), salts with an amino acid (e.g., arginine, aspartic acid, glutamic acid, or the like), metal salts such as alkali metal salts (e.g., sodium salt, potassium salt, and the like), alkali earth metal salts (e.g., calcium salt, magnesium salt, and the like), ammonium salts, organic base salts (e.g., trimethylamine salts, triethylamine salts, pyridine salts, picoline salts, dicyclohexylamine salts, N,N'-dibenzylethylene diamine salts, and the like), and the like. Moreover, such conventional nontoxic salts can be appropriately selected by those skilled in the art.

When it is desirable to obtain a salt of the compound of the invention, the compound of the invention may be directly purified if the compound of the invention is obtained in a salt form, or the compound of the invention may be dissolved or suspended in a suitable organic solvent and an acid or a base is added to form a salt in accordance with a general method if the compound of the invention is obtained in a free form.

While the compound of the invention and pharmaceutically acceptable salt thereof may also be in a form of an adduct with water or various solvents, such an adduct is also encompassed by the present invention.

The present invention encompasses compounds represented by formula (1) or pharmaceutically acceptable salts thereof. The present invention also encompasses hydrates or solvates such as ethanol solvates thereof. Furthermore, the present invention encompasses all tautomers, all existing stereoisomers, and all modes of crystalline forms of the compound of the invention represented by formula (1).

Some of the compounds of the invention represented by formula (1) can be optical isomers based on an optically-active center, atropisomers based on axial or planar chirality resulting from restriction of intramolecular rotation, other stereoisomers, tautomers, geometric isomers, and the like. Meanwhile, all possible isomers and mixtures thereof, including the isomers mentioned, are encompassed within the scope of the present invention.

In particular, an optical isomer and an atropisomer can be obtained as either a racemate or an optically-active form if an optically-active starting material or intermediate is used. If necessary, a racemate of a corresponding starting material, an intermediate, or a final product can be physically or chemically resolved, during an appropriate step of the production method described below, into their optical enantiomers by a known separation method, such as a method using an optically active column, a fractional crystallization method, or the like. Specifically, a diastereomer method, for example, forms two types of diastereomers from a racemate by a reaction using an optically resolving agent. Since the different diastereomers generally have different physical properties, they can be resolved by a known method such as fractional crystallization or the like.

Manufacturing methods of compounds of the invention are described below. The compound of the invention represented by formula (1) or pharmaceutically acceptable salt thereof can be manufactured from known compounds, for example, by an appropriate combination of the following manufacturing methods A to K and methods in accordance therewith, or synthesis methods that are well known to those skilled in the art.

A compound in a reaction includes those where a salt is formed. For example, salts similar to salts of the compound represented by formula (1) or the like are used as such a salt.

While a compound obtained in each step can be used in a subsequent reaction directly as a reaction solution or as a composition, the compound can be isolated from a reaction mixture in accordance with a conventional method, and readily purified by separation means such as recrystallization, distillation, chromatography, or the like.

Each symbol of compounds in the following reactions is defined the same as above, unless specifically noted otherwise.

### Manufacturing method A

Among the compounds represented by formula (1), a compound represented by formula [A1] wherein A is A-13, X¹ and X² are -O-C(=O)-Y-(C(R^{1A})(R^{1B}))ₙ-NH-R², and Y is NR⁴ can be manufactured by, for example, the following manufacturing method. 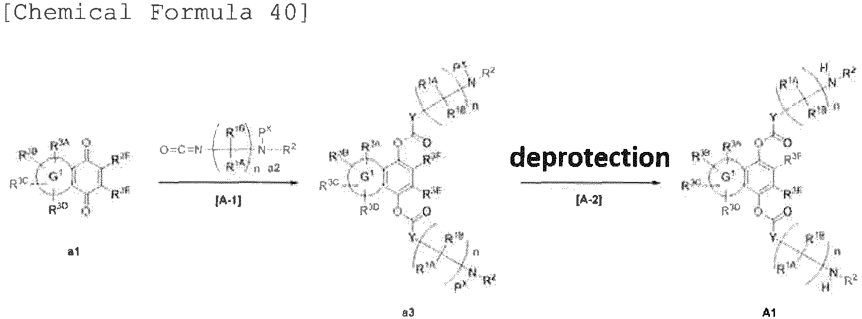 wherein R^{1A}, R^{1B}, R², n, Y, R^{3A}, R^{3B}, R^{3C}, R^{3D}, R^{3E}, and R^{3F} are defined in the same manner as item 1, and P^{x} indicates an amine protecting group.

Examples of protecting group P^{x} include the amine protecting group described in Protective Groups in Organic Synthesis (authored by Theodora W. Greene, Peter G. M. Wuts, published by John Wiley & Sons, Inc., 1999).

Compound a2 can be manufactured by the method described in a patent literature (e.g., WO 2009/036059 or the like).

### [Step A-1]

This is a step for obtaining compound a3 by reacting compound a2 obtained by the following manufacturing method with compound a1 in a suitable solvent in the presence of an additive, base, and reducing agent. Examples of additives include tetra-n-butylammonium chloride, tetra-n-butylammonium bromide, tetra-n-butylammonium iodide, and the like. The additive is preferably tetra-n-butylammonium bromide. Examples of base that can be used include basic salts such as sodium carbonate, potassium carbonate, cesium carbonate, sodium acetate, sodium hydride, and calcium hydride; aromatic amines such as pyridine, lutidine, 4-dimethylaminopyridine, and N,N-dimethylaniline; tertiary amines such as triethylamine, tripropylamine, tributylamine, cyclohexyldimethylamine, N,N-diisopropylethylamine, N-methylpiperidine, N-methylpyrrolidine, and N-methylmorpholine; and the like. In particular, triethylamine or N,N-diisopropylethylamine is preferable. Examples of reducing agent include iron (II) ion, tin (II) ion, sodium, zinc, formic acid, oxalic acid, sodium dithionite, and the like. A reducing agent is preferably a mixed reducing agent of zinc and sodium dithionite. The solvent used in this step is selected from the solvents described below. Examples thereof include aprotic solvents such as N, N-dimethylformamide, N-methyl-2-pyrrolidone, dimethyl sulfoxide, acetonitrile, and propionitrile; ether-based solvents such as tetrahydrofuran and 1,4-dioxane; halogenated hydrocarbons such as dichloromethane (methylene chloride), chloroform, 1,2-dichloroethane, and chlorobenzene; hydrocarbons such as toluene and benzene; water; mixed solvents thereof; and the like. Preferred examples thereof include N, N-dimethylformamide. The amount of a reducing agent used is typically 2 equivalents to 20 equivalents, preferably 4 equivalents to 8 equivalents, relative to one equivalent of compound a1. The reaction time is typically about 0.5 hours to about 48 hours, and preferably about 0.5 hours to about 2 hours. The reaction temperature is typically about -20°C to about 180°C, and preferably about 0°C to about 50°C.

### [Step A-2]

This is a step of obtaining compound A1 by deprotecting the amine protecting group P^{x} of compound a3 obtained in step A-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method B

Among compounds represented by formula (1), a compound represented by formula [A1] wherein A is A-13, and X¹ and X² are -O-C(=O)-Y-(C(R^{1A})(R^{1B}))ₙ-NH-R² can be manufactured by, for example, the following manufacturing method. 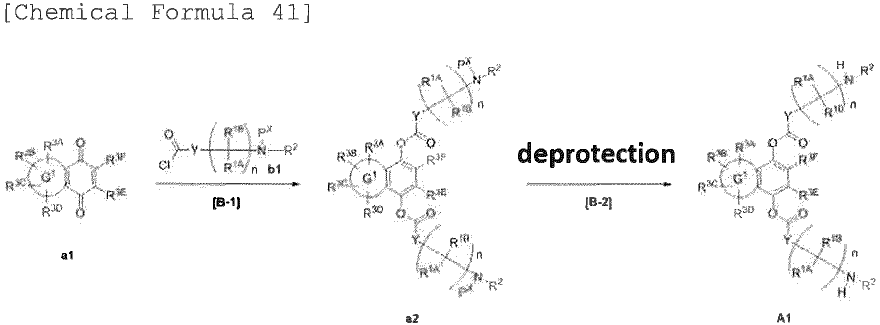 wherein R^{1A}, R^{1B}, R², n, Y, R^{3A}, R^{3B}, R^{3C}, R^{3D}, R^{3E}, and R^{3F} are defined in the same manner as item 1, and P^{x} indicates an amine protecting group.

Examples of protecting group P^{x} include the amine protecting group described in Protective Groups in Organic Synthesis (authored by Theodora W. Greene, Peter G. M. Wuts, published by John Wiley & Sons, Inc., 1999).

### [Step B-1]

This is a step for obtaining compound a3 by reacting compound b1 obtained by the following manufacturing method with compound a1 under the conditions in accordance with step A-1.

### [Step B-2]

This is a step of obtaining compound A1 by deprotecting the amine protecting group P^{x} of compound a3 obtained by manufacturing method B-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method C

Among compounds represented by formula (1), a compound represented by formula [C1] wherein A is A-13, X¹ and X² are a hydroxyl group and -O-C(=O)-Y-(C(R^{1A})(R^{1B}))ₙ-NH-R², respectively, and Y is NR⁴ can be manufactured by, for example, the following manufacturing method. 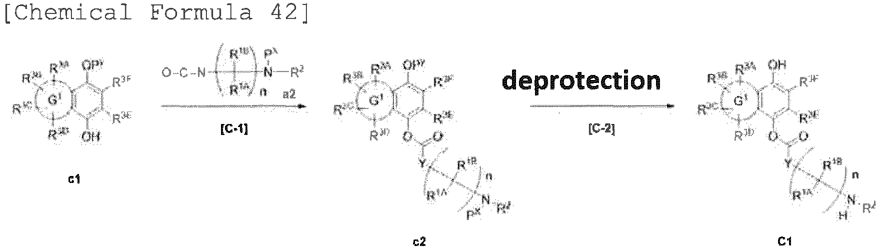 wherein R^{1A}, R^{1B}, R², n, Y, R^{3A}, R^{3B}, R^{3C}, R^{3D}, R^{3E}, and R^{3F} are defined in the same manner as item 1, and P^{x} indicates an amine protecting group and P^{Y} as a protecting group for a phenolic hydroxyl group.

Examples of protecting group P^{x} as an amine protecting group and P^{Y} as a protecting group for a phenolic hydroxyl group include those described in Protective Groups in Organic Synthesis (authored by Theodora W. Greene, Peter G. M. Wuts, published by John Wiley & Sons, Inc., 1999).

Compound c1 can be manufactured from compound a1 by a method similar to the method described in a patent literature (e.g., WO 2012/119265, WO 2013/120229, WO 2013/128037, etc.) or the like.

### [Step C-1]

This is a step for obtaining compound c2 by reacting compound a2 obtained by the following manufacturing method with compound c1 in a suitable solvent in the presence of a base. Solvent used in the reaction may be any solvent as long as it is inactive in the reaction, and is not particularly limited. For example, tetrahydrofuran, 1,4-dioxane, dimethoxyethane, benzene, toluene, xylene, N,N-dimethylformamide, N-methyl-2-pyrrolidone, dimethyl sulfoxide, and the like can be used alone or as a mixture thereof. In particular, tetrahydrofuran or N,N-dimethylformamide is preferable. Examples of base that can be used include basic salts such as sodium carbonate, potassium carbonate, cesium carbonate, sodium acetate, sodium hydride, and calcium hydride; aromatic amines such as pyridine, lutidine, 4-dimethylaminopyridine, and N,N-dimethylaniline; tertiary amines such as triethylamine, tripropylamine, tributylamine, cyclohexyldimethylamine, N,N-diisopropylethylamine, N-methylpiperidine, N-methylpyrrolidine, and N-methylmorpholine; and the like. In particular, triethylamine or N,N-diisopropylethylamine is preferable.

The reaction temperature is typically a temperature between 0°C and the boiling point of a solvent used, and preferably, 0 to 80°C. The reaction time is typically 0.5 hours to 24 hours, and preferably about 0.5 hours to about 2 hours.

### [Step C-2]

This is a step of obtaining compound C1 by deprotecting the amine protecting group P^{x} and the protecting group P^{Y} for a phenolic hydroxyl group of compound c2 obtained by manufacturing method C-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method D

Among compounds represented by formula (1), a compound represented by formula [C1] wherein A is A-13, and X¹ and X² are a hydroxyl group and -O-C(=O)-Y-(C(R^{1A})(R^{1B}))ₙ-NH-R², respectively, can be manufactured by, for example, the following manufacturing method. 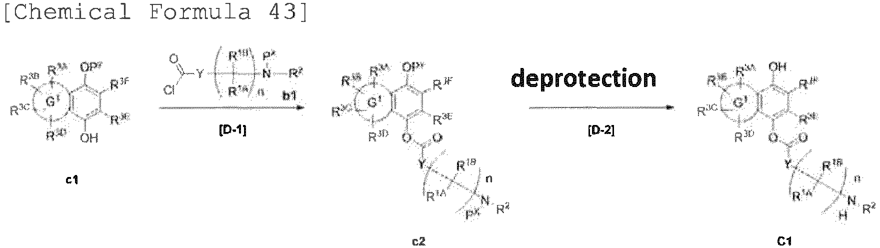 wherein R^{1A}, R^{1B}, R², n, Y, R^{3A}, R^{3B}, R^{3C}, R^{3D}, R^{3E}, and R^{3F} are defined in the same manner as item 1, P^{x} indicates an amine protecting group, and P^{Y} indicates a protecting group for a phenolic hydroxyl group.

Examples of protecting group P^{x} as an amine protecting group and P^{Y} as a protecting group for a phenolic hydroxyl group include those described in Protective Groups in Organic Synthesis (authored by Theodora W. Greene, Peter G. M. Wuts, published by John Wiley & Sons, Inc., 1999).

Compound c1 can be manufactured from compound a1 by a method similar to the method described in a patent literature (e.g., WO 2012/119265, WO 2013/120229, WO 2013/128037, etc.) or the like.

### [Step D-1]

This is a step for obtaining compound c2 by reacting compound b1 obtained by the following manufacturing method with compound c1 under the conditions in accordance with step C-1 described above.

### [Step D-2]

This is a step of obtaining compound C1 by deprotecting the amine protecting group P^{x} and protecting group P^{Y} for a phenolic hydroxyl group of compound c2 obtained by manufacturing method D-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method E (Manufacturing method of a synthetic intermediate)

A synthetic intermediate represented by a2 described above can be manufactured by, for example, the following manufacturing method. 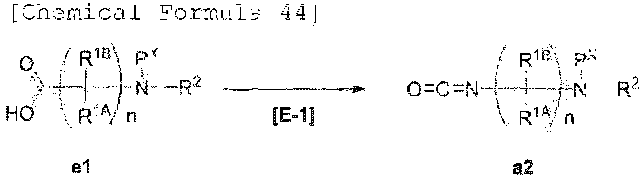 wherein R^{1A}, R^{1B}, R², and n are defined in the same manner as item 1, and P^{x} indicates an amine protecting group.

### [Step E-1]

This is a step of converting compound e1 to compound a2.

Compound a2 can be manufactured from compound e1 by a method similar to the method described in a Non Patent Literature {e.g., J. Am. Chem. Soc., 6203, vol.94, (1972), Tetrahedron, 2151, vol.30, (1974), etc.} or the like.

### Manufacturing method F (Manufacturing method of a synthetic intermediate)

A synthetic intermediate represented by b1 described above can be manufactured by, for example, the following manufacturing method. 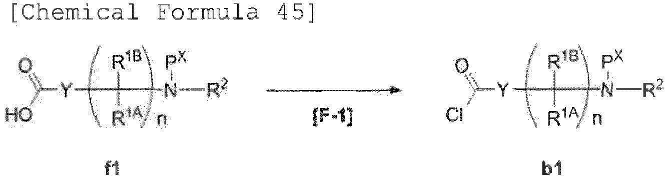 wherein R^{1A}, R^{1B}, R², n, and Y are defined in the same manner as item 1, and P^{x} indicates an amine protecting group.

### [Step F-1]

This is a step of converting compound f1 to compound b1.

Compound b1 can be manufactured from compound f1 by a method similar to the method described in a document {e.g., J. Am. Chem. Soc., 7442, vol.135, (2013), J. Org. Chem., 4506, vol. 68, (2003), J. Med. Chem., 3582, vol. 44, (2001), etc.} or the like.

### Manufacturing method G (Manufacturing method of a synthetic intermediate)

A synthetic intermediate represented by b1 described above can be manufactured by, for example, the following manufacturing method. 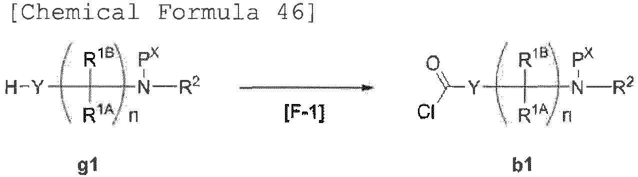 wherein R^{1A}, R^{1B}, R², n, and Y are defined in the same manner as item 1, and P^{x} indicates an amine protecting group.

### [Step G-1]

This is a step of converting compound g1 to compound b1.

Compound b1 can be manufactured from compound g1 by a method similar to the method described in a document {e.g., J. Am. Chem. Soc., 5505, vol. 106, (1984), J. Org. Chem., 5342, vol. 72, (2007), Tetrahedron, 9153, vol. 63, (2007), J. Org. Chem., 5325, vol. 45, (1980), J. Org. Chem., 3787, vol. 69, (2004), etc.} or the like.

### Manufacturing method H

Among compounds represented by formula (1), a compound represented by formula [H1] wherein A is A-9, and X¹ and X² are -O-C(=O)-Y-(C(R^{1A})(R^{1B}))ₙ-NH-R² can be manufactured by, for example, the following manufacturing method. 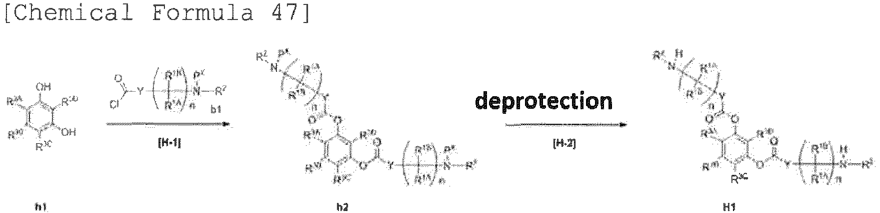 wherein R^{1A}, R^{1B}, R², n, Y, R^{3A}, R^{3B}, R^{3C}, and R^{3D} are defined in the same manner as item 1, and P^{x} indicates an amine protecting group.

Examples of protecting group P^{x} include the amine protecting group described in Protective Groups in Organic Synthesis (authored by Theodora W. Greene, Peter G. M. Wuts, published by John Wiley & Sons, Inc., 1999).

### [Step H-1]

This is a step for obtaining compound h2 by reacting compound b1 obtained by the manufacturing method described above with compound h1 under the conditions in accordance with step C-1 described above.

### [Step H-2]

This is a step of obtaining compound H1 by deprotecting the amine protecting group P^{x} of compound h2 obtained by manufacturing method H-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method I

Among compounds represented by formula (1), a compound represented by formula [H1] wherein A is A-9, and X¹ and X² are -O-C(=O)-Y-(C(R^{1A})(R^{1B}))ₙ-NH-R² can be manufactured by, for example, the following manufacturing method. 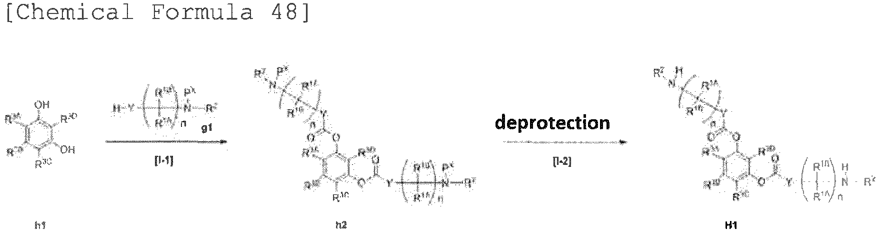 wherein R^{1A}, R^{1B}, R², n, Y, R^{3A}, R^{3B}, R^{3C}, and R^{3D} are defined in the same manner as item 1, and P^{x} indicates an amine protecting group.

Examples of protecting group P^{x} include the amine protecting group described in Protective Groups in Organic Synthesis (authored by Theodora W. Greene, Peter G. M. Wuts, published by John Wiley & Sons, Inc., 1999).

### [Step I-1]

This is a step for converting compound h1 to compound h2, which can be manufactured from compound h1 by a method similar to the method described in a document {e.g., Bioconjugate Chem., 1267, vol. 27, (2016), Mol. Pharmaceutics, 1813, vol. 12, (2015), etc.} or the like in this step.

### [Step I-2]

This is a step of obtaining compound H1 by deprotecting the amine protecting group P^{x} of compound h2 obtained by manufacturing method I-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method J

Among compounds represented by formula (1), a compound represented by formula [J1] wherein A is A-9, and X¹ and X² are a hydroxyl group and -O-C(=O)-Y-(C(R^{1A})(R^{1B}))ₙ-NH-R², respectively, can be manufactured by, for example, the following manufacturing method. 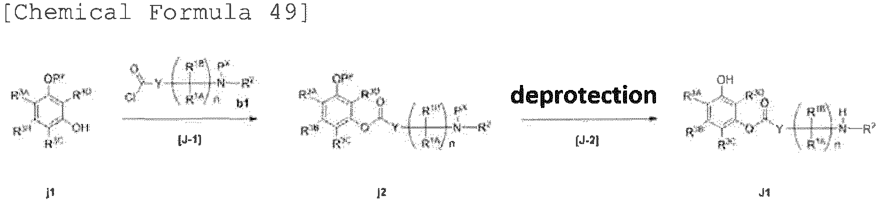 wherein R^{1A}, R^{1B}, R², n, Y, R^{3A}, R^{3B}, R^{3C}, and R^{3D} are defined in the same manner as item 1, and P^{x} indicates an amine protecting group, and P^{Y} indicates a protecting group for a phenolic hydroxyl group.

Examples of protecting group P^{x} as an amine protecting group and protecting group P^{Y} as a protecting group for a phenolic hydroxyl group include those described in Protective Groups in Organic Synthesis (authored by Theodora W. Greene, Peter G. M. Wuts, published by John Wiley & Sons, Inc., 1999).

### [Step J-1]

This is a step of obtaining compound j2 by reacting compound b1 obtained by the manufacturing method described above with compound j1 under the conditions in accordance with step C-1 described above.

### [Step J-2]

This is a step of obtaining compound J1 by deprotecting the amine protecting group P^{x} and the protecting group P^{Y} for a phenolic hydroxyl group of compound j2 obtained by manufacturing method J-1 described above. This step can be performed in accordance with the method described in Protective Groups in Organic Synthesis (authored by Theodora W. Greene, Peter G. M. Wuts, published by John Wiley & Sons, Inc., 1999) or the like.

### Manufacturing method K

Among compounds represented by formula (1), a compound represented by formula [J1] wherein A is A-9, and X¹ and X² are a hydroxyl group and -O-C(=O)-Y-(C(R^{1A})(R^{1B}))ₙ-NH-R², respectively, can be manufactured by, for example, the following manufacturing method. 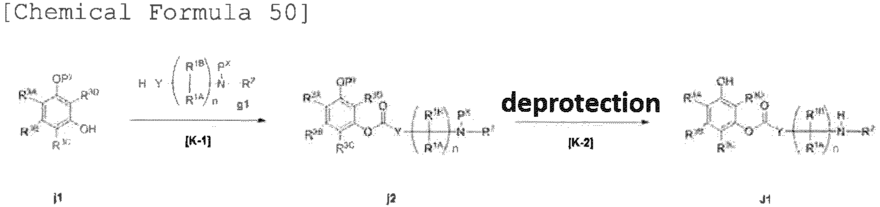 wherein R^{1A}, R^{1B}, R², n, Y, R^{3A}, R^{3B}, R^{3C}, and R^{3D} are defined in the same manner as item 1, P^{x} indicates an amine protecting group, and P^{Y} indicates a protecting group for a phenolic hydroxyl group.

Examples of protecting group P^{x} as an amine protecting group and protecting group P^{Y} as a protecting group for a phenolic hydroxyl group include those described in Protective Groups in Organic Synthesis (authored by Theodora W. Greene, Peter G. M. Wuts, published by John Wiley & Sons, Inc., 1999).

### [Step K-1]

This is a step of obtaining compound j2 by reacting compound j1 under the conditions in accordance with step I-1 described above.

### [Step K-2]

This is a step of obtaining compound J1 by deprotecting the amine protecting group P^{x} and the protecting group P^{Y} for a phenolic hydroxyl group of compound j2 obtained by manufacturing method K-1 described above. This step can be performed in accordance with the method described in Protective Groups in Organic Synthesis (authored by Theodora W. Greene, Peter G. M. Wuts, published by John Wiley & Sons, Inc., 1999) or the like.

In each reaction of the manufacturing methods described above, even when the use of a protecting group is not explicitly described, a compound of interest can be obtained by protecting a functional group other than the reaction point as needed and deprotecting after the completion of the reaction or after the completion of a series of reactions if any of the functional groups other than the reaction point changes under the described reaction conditions, or if it is unsuitable for performing the described method.

As a protecting group, general protecting groups described in a document (e.g., Protective Groups in Organic Synthesis, 3rd ed., T. W. Greene, John Wiley & Sons Inc. (1999) or the like) can be used. More specifically, examples of protecting groups of an amino group include benzyloxycarbonyl, tert-butoxycarbonyl, acetyl, benzyl, and the like, and examples of protecting groups of hydroxyl groups include trialkylsilyl groups such as trimethylsilyl and tert-butyldimethylsilyl, acetyl, benzyl, and the like.

Protecting groups can be introduced and removed according to a method that is generally used in synthetic organic chemistry (see, for example, the aforementioned Protective Groups in Organic Synthesis) or a method in accordance therewith.

The base used in each step described above should be appropriately selected depending on the type of raw material compound, reaction, or the like. Examples thereof include alkali bicarbonate such as sodium bicarbonate and potassium bicarbonate, alkali carbonate such as sodium carbonate and potassium carbonate, metal hydrides such as sodium hydride and potassium hydride, alkali metal hydroxides such as sodium hydroxide and potassium hydroxide, alkali metal alkoxides such as sodium methoxide and sodium tert-butoxide, organic metal bases such as butyl lithium and lithium diisopropylamide, and organic bases such as triethylamine, diisopropylethylamine, pyridine, 4-dimethylaminopyridine (DMAP), and 1,8-diazabicyclo[5.4.0]-7-undecene (DBU).

Solvents used in each step described above should be appropriately selected depending on the type of raw material compound, reaction or the like. Examples thereof include alcohols such as methanol, ethanol, and isopropanol, ketones such as acetone and ethyl methyl ketone, halogenated hydrocarbons such as methylene chloride and chloroform, ethers such as tetrahydrofuran (THF) and dioxane, aromatic hydrocarbons such as toluene and benzene, aliphatic hydrocarbons such as hexane and heptane, esters such as ethyl acetate and propyl acetate, amides such as N,N-dimethylformamide (DMF) and N-methyl-2-pyrrolidone, sulfoxides such as dimethyl sulfoxide (DMSO), and nitriles such as acetonitrile. Such solvents can be used alone or two or more thereof can be mixed and used. Depending on the type of reaction, an organic base can be used as a solvent.

The compound of the invention represented by formula (1) or an intermediate thereof can be separated and purified by a method that is known to those skilled in the art. Examples thereof include extraction, partition, reprecipitation, column chromatography (e.g., silica gel column chromatography, ion exchange column chromatography, or preparative liquid chromatography), recrystallization, and the like.

Examples of recrystallization solvents that can be used include alcohol-based solvents such as methanol, ethanol, and 2-propanol; ether-based solvents such as diethyl ether; ester-based solvents such as ethyl acetate; aromatic-hydrocarbon-based solvents such as benzene and toluene; ketone-based solvents such as acetone; halogen-based solvents such as dichloromethane and chloroform; hydrocarbon-based solvents such as hexane; aprotic solvents such as dimethylformamide and acetonitrile; water; mixed solvent thereof; and the like. Other purification methods can be used, such as the methods described in Jikken Kagaku Koza (The Chemical Society of Japan ed., Maruzen), vol. 1, or the like. The molecular structure of the compound of the invention can be readily determined by spectroscopic techniques such as the nuclear magnetic resonance, infrared absorption method, and circular dichroism spectroscopy, and mass spectrometry with reference to the structures originating from respective raw material compounds.

The intermediates or final products in the manufacturing methods described above can be led to other compounds encompassed in the present invention by appropriately converting a functional group thereof, and particularly by extending various side chains using an amino group, a hydroxyl group, a carbonyl group, a halogen group, or the like as the starting point, with the protection and deprotection described above as needed. A routine common method can be used for the conversion of a functional group and extension of a side chain (see, for example, Comprehensive Organic Transformations, R. C. Larock, John Wiley & Sons Inc. (1999), and the like).

The compound of the invention represented by formula (1) or pharmaceutically acceptable salts thereof can have asymmetry or a substituent having an asymmetric carbon. Such a compound has an optical isomer. The compound of the invention also encompasses mixtures of each isomer and isolated isomers, which can be manufactured according to a conventional method. Examples of the manufacturing method include a method using a raw material having an asymmetric point and a method of introducing asymmetry during the process. Optical isomers for example can be obtained by using an optically active raw material, performing optical resolution, or the like in a suitable stage of a production step. Examples of optical resolution methods include a diastereomer method of forming a salt, when the compound represented by formula (1) or intermediates thereof have a basic functional group, in an inactive solvent (e.g., an alcohol-based solvent such as methanol, ethanol, or 2-propanol; an ether-based solvent such as diethyl ether; an ester-based solvent such as ethyl acetate; a hydrocarbon-based solvent such as toluene; an aprotic solvent such as acetonitrile; or a mixed solvent thereof) using an optically active acid (e. g., monocarboxylic acid such as mandelic acid, N-benzyloxyalanine, or lactic acid, dicarboxylic acid such as tartaric acid, ortho-diisopropylidene tartaric acid, or malic acid, sulfonic acid such as camphorsulfonic acid or bromocamphorsulfonic acid).

When an intermediate for the compound of the invention represented by formula (1) has an acidic functional group such as a carboxyl group, optical resolution can be performed by forming a salt using an optically active amine (e.g., organic amines such as 1-phenylethylamine, quinine, quinidine, cinchonidine, cinchonine, or strychnine).

A temperature for the formation of a salt is selected from the range from room temperature to the boiling point of a solvent. To improve the optical purity, it is desirable to first increase the temperature to a temperature near the boiling point of a solvent. When collecting a precipitated salt by filtration, it can be cooled as needed to improve the yield. The amount of an optically active acid or amine used is suitable to be in the range from about 0.5 to about 2.0 equivalents and preferably approximately 1 equivalent relative to a substrate. A crystal can be recrystallized in an inactive solvent (e.g., an alcohol-based solvent such as methanol, ethanol, or 2-propanol; an ether-based solvent such as diethyl ether; an ester-based solvent such as ethyl acetate; a hydrocarbon-based solvent such as toluene; an aprotic solvent such as acetonitrile; or a mixture thereof) as needed to obtain an optically active salt with high purity. An optically resolved salt can be treated with an acid or a base by a conventional method to obtain its free form as needed.

Starting materials and intermediates in each manufacturing methods described above without a specific description of the manufacturing method are commercially available compounds, or compounds that can be synthesized from a commercially available compound by a method known to those skilled in the art or a method in accordance therewith.

Compounds represented by formulas (1) and (1-A-1a) to (1-A-17c) and pharmaceutically acceptable salts thereof exhibit high water-solubility that is suitable for oral and parenteral administration, and have at least one or more primary or secondary nitrogen atoms at the terminus. Due to such a structural feature, formulas (1) and (1-A-la) to (1-A-17c) act as prodrugs that are converted to an active form by chemical conversion. As used herein, chemical conversion means the conversion to an activated form *in vivo* via a route other than enzymatic conversion. For example, as represented by the following formula: 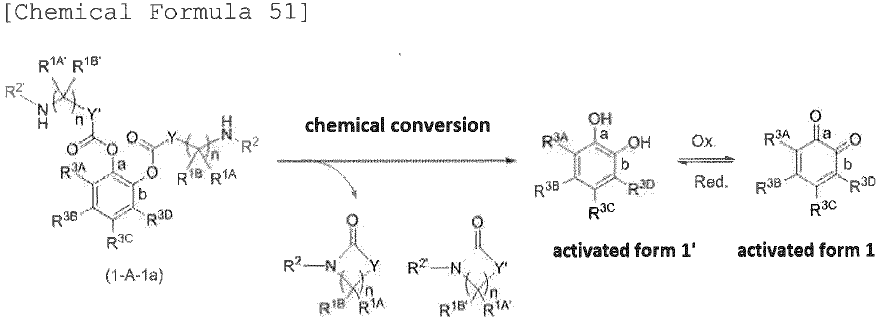 (wherein each symbol is defined the same as item 1, and R^{1A'}, R^{1B'}, R^{2'}, and Y' are defined the same as R^{1A}, R^{1B}, R², and Y, respectively), a typical compound of formula (1) represented by formula (1-A-1a), wherein A is A-1, X¹ and X² are -O-C(=O)-Y-(C(R^{1A})(R^{1B}))ₙ-NH-R² and -O-C(=O)-Y'-(C(R^{1A'})(R^{1B'}))n-NH-R^{2'}, respectively, is chemically converted *in vivo* to an active compound represented by activated form 1 by attack of nitrogen atoms binding to each of terminal R² and R^{2'} against the corresponding carbonyl carbon. Activated form 1 and activated form 1' are isomers having a relationship of a reductant and an oxidant that can be in equilibrium, and can be considered equivalent.

Likewise, a typical compound of formula (1) represented by formula (1-A-12a), wherein A is A-12, X¹ and X² are -O-C(=O)-Y-(C(R^{1A})(R^{1B}))ₙ-NH-R² and -O-C(=O)-Y'-(C(R^{1A'})(R^{1B'}))n-NH-R^{2'}, respectively, is chemically converted *in vivo* to an active compound represented by activated form 1 by attack of nitrogen atoms binding to each of terminal R² and R^{2'} against the corresponding carbonyl carbon, as described below. Activated form 1 and activated form 1' are isomers having a relationship of a reductant and an oxidant that can be in equilibrium, and can be considered equivalent. 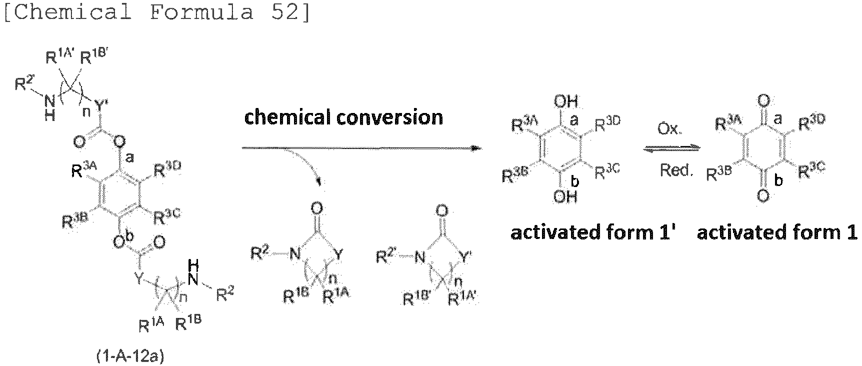 wherein each symbol is defined the same as item 1, and R^{1A'}, R^{1B'}, R^{2'}, and Y' are defined the same as R^{1A}, R^{1B}, R², and Y, respectively.

Likewise, a typical compound of formula (1) represented by formula (1-A-9a), wherein A is A-9, X¹ and X² are -O-C(=O)-Y-(C(R^{1A})(R^{1B}))ₙ-NH-R² and -O-C(=O)-Y'-(C(R^{1A'})(R^{1B'}))n-NH-R^{2'}, respectively, is chemically converted *in vivo* to an active compound represented by activated form 1 by attack of nitrogen atoms binding to each of terminal R² and R^{2'} against the corresponding carbonyl carbon, as described below. 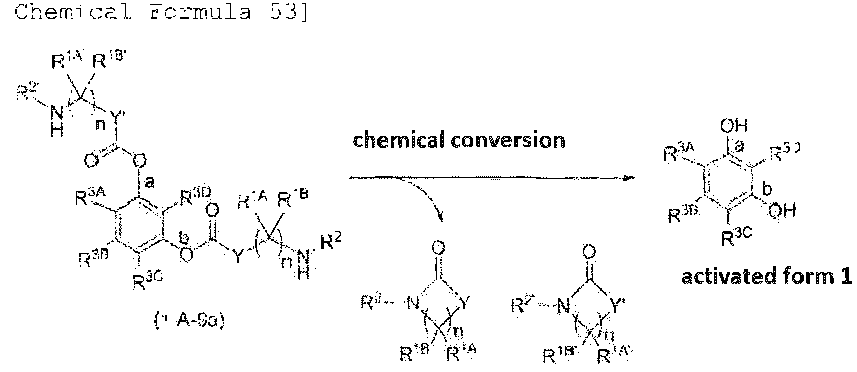 wherein each symbol is defined the same as item 1, and R^{1A'}, R^{1B'}, R^{2'}, and Y' are defined the same as R^{1A}, R^{1B}, R², and Y, respectively.

In the same manner as formulas (1-A-1a), (1-A-12a), and b(1-A-9a) described above, primary or secondary nitrogen atoms at the terminus attack the corresponding carbon atoms to generate activated form 1 via chemical conversion for formulas (1-A-1b), (1-A-1c), (1-A-2a), (1-A-2b), (1-A-2c), (1-A-3a), (1-A-3b), (1-A-3c), (1-A-4a), (1-A-4b), (1-A-4c), (1-A-5a), (1-A-5b), (1-A-5c), (1-A-6a), (1-A-6b), (1-A-6c), (1-A-7a), (1-A-7b), (1-A-7c), (1-A-8a), (1-A-8b), (1-A-8c), (1-A-9b), (1-A-9c), (1-A-10a), (1-A-10b), (1-A-10c), (1-A-11a), (1-A-11b), (1-A-11c), (1-A-12b), (1-A-12c), (1-A-13a), (1-A-13b), (1-A-13c), (1-A-14a), (1-A-14b), (1-A-14c), (1-A-15a), (1-A-15b), (1-A-15c), (1-A-16a), (1-A-16b), (1-A-16c), (1-A-17a), (1-A-17b), and (1-A-17c).

The compound of the invention is provided, for example, as a therapeutic agent and/or preventive agent for cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes.

The compound of the invention is provided, for example, as a therapeutic agent and/or preventive agent for cancer. Although the type of cancer is not limited, specific examples include acute leukemia, chronic lymphocytic leukemia, chronic myeloid leukemia, polycythemia vera, malignant lymphoma, brain tumor, head and neck cancer, esophageal cancer, thyroid cancer, small cell lung cancer, non-small-cell lung cancer, breast cancer, gastric cancer, gallbladder/bile duct cancer, liver cancer, pancreatic cancer, colon cancer, rectal cancer, chorioepithelioma, chorioblastoma, choriocarcinoma, endometrial cancer, cervical cancer, urothelial cancer, renal cell cancer, testicular tumor, Wilms' tumor, skin cancer, malignant melanoma, neuroblastoma, osteosarcoma, Ewing's sarcoma, and soft tissue sarcoma. Hematological cancer in the present invention is a concept encompassing lymphoma and leukemia. Therapeutic agents and/or preventive agents for cancer have an effect of reducing or eliminating carcinoma or preventing the growth of carcinoma in order to prevent and/or treat cancer.

The compound of the invention is provided, for example, as a therapeutic agent and/or preventive agent for allergy. Although the type of allergy is not limited, specific examples thereof include type I allergies, type II allergies, type III allergies, and type IV allergies. Examples of type I allergies include hives, PIE syndrome, food allergy, hay fever, allergic rhinitis, bronchial asthma, atopic dermatitis, and anaphylactic shock. Examples of types II allergies include autoimmune hemolytic anemia (AIHA), incompatible blood transfusion, idiopathic thrombocytopenic purpura (ITP), pernicious anemia, rheumatic fever, Goodpasture syndrome, myasthenia gravis, Hashimoto's disease, and alopecia areata. Examples of type III allergies include serum sickness, systemic lupus erythematosus (lupus nephritis), acute glomerulonephritis, rheumatoid arthritis, hypersensitivity pneumonitis, rheumatic pneumonia, polyarteritis, allergic vasculitis, and Sjogren's syndrome. Examples of type IV allergies include contact dermatitis (so-called "urushiol dermatitis" is one type of "allergic contact dermatitis"), tuberculin reaction, transplantation immunity, metal allergy, tumor immunity, Sjogren's syndrome, infection allergy, drug-induced pneumonia, and Guillain-Barre syndrome.

The compound of the invention is provided, for example, as a therapeutic agent and/or preventive agent for dementia (cognitive impairment). Although the type of dementia (cognitive impairment) is not limited, specific examples thereof include vascular dementia and degenerative dementia. Examples of vascular dementia include multi-infarct dementia, extensive ischemic dementia, multiple cerebral infarction dementia, localized cerebral infarction dementia, and inherited vascular dementia. Examples of degenerative dementia include Alzheimer's dementia, Lewy body dementia, Parkinson's disease with dementia, frontotemporal dementia, Huntington's disease, and cognitive impairment associated with schizophrenia.

The compound of the invention is provided, for example, as a therapeutic agent and/or preventive agent for muscular dystrophy. Although the type of muscular dystrophy is not limited, specific examples thereof include sex-linked recessive muscular dystrophy, congenital muscular dystrophy, limb-girdle muscular dystrophy, facioscapulohumeral muscular dystrophy, and myotonic dystrophy. Examples of sex-linked recessive muscular dystrophy include Duchenne muscular dystrophy and Becker muscular dystrophy. Examples of congenital muscular dystrophy include Fukuyama muscular dystrophy, Ullrich muscular dystrophy, merosin-deficient muscular dystrophy, integrin-deficient muscular dystrophy, and Walker-Warburg syndrome muscular dystrophy.

The compound of the invention is provided, for example, as a therapeutic agent and/or preventive agent for demyelinating disease. Although the type of demyelinating disease is not limited, specific examples thereof include demyelinating disease of the central nervous system and demyelinating disease of the peripheral nervous system. Examples of demyelinating disease of the central nervous system include multiple sclerosis, acute disseminated encephalomyelitis, inflammatory diffuse sclerosis, subacute sclerosing panencephalitis, progressive multifocal leukoencephalopathy, hypoxic encephalopathy, central pontine myelinolysis, vitamin B12 deficiency, and Binswanger disease. Examples of demyelinating disease of the peripheral nervous system include Guillain-Barre syndrome, Fisher syndrome, and chronic inflammatory demyelinating polyradiculoneuropathy.

The compound of the invention is provided, for example, as a therapeutic agent and/or preventive agent for protozoal infection. Although the type of protozoal infection is not limited, specific examples thereof include protozoal malaria infection, giardiasis, and trypanosomiasis.

The compound of the invention is provided, for example, as a therapeutic agent and/or preventive agent for heart failure. Although the type of heart failure is not limited, specific examples thereof include acute heart failure and chronic heart failure.

The compound of the invention is provided, for example, as a therapeutic agent and/or preventive agent for hypertension. Although the type of hypertension is not limited, specific examples thereof include essential hypertension and secondary hypertension. Examples of causes of secondary hypertension include aortic coarctation, renovascular hypertension, renal parenchymal hypertension, primary aldosteronism, pseudoaldosteronism, Apparent Mineralocorticoid Excess syndrome (AME syndrome), Liddle syndrome, Cushing's syndrome, pheochromocytoma, Takayasu arteritis, thyroid dysfunction, gestational hypertension, and hypercalcemia.

The compound of the invention is provided, for example, as a therapeutic agent and/or preventive agent for liver disease. Although the type of liver disease is not limited, specific examples thereof include fatty liver, cirrhosis, hepatitis, liver cancer, hemochromatosis, primary sclerosing cholangitis, and Wilson's disease. Examples of hepatitis include viral hepatitis, alcoholic hepatitis, nonalcoholic steatohepatitis, drug-induced hepatitis, autoimmune hepatitis, and primary biliary cholangitis.

The compound of the invention is provided, for example, as a therapeutic agent and/or preventive agent for bullous disease. Although the type of bullous disease is not limited, specific examples thereof include pemphigus, pemphigoid, dermatitis herpetiformis, linear IgA bullous dermatosis, congenital epidermolysis bullosa, and acquired epidermolysis bullosa. Examples of pemphigus include pemphigus vulgaris, pemphigus foliaceus, pemphigus vegetans, pemphigus erythematosus, paraneoplastic pemphigus, and IgA pemphigus. Examples of pemphigoid include bullous pemphigoid and mucous membrane pemphigoid. Examples of congenital epidermolysis bullosa include epidermolysis bullosa simplex, hemidesmosomal epidermolysis bullosa, junctional epidermolysis bullosa, and dystrophic epidermolysis bullosa.

The compound of the invention is provided, for example, as a therapeutic agent and/or preventive agent for thrombus. Although the type of antithrombotic drug is not limited, specific examples thereof include antiplatelet drugs, anticoagulants, and thrombolytic drugs.

The compound of the invention is provided, for example, as a therapeutic agent and/or preventive agent for hemorrhage. Although the type of antihemorrhagic drug is not limited, specific examples thereof include blood clotting drugs, blood coagulation factor deficient drugs, and antifibrinolytics.

The compound of the invention is provided, for example, as a therapeutic agent and/or preventive agent for vitamin deficiency. Although the type of vitamin deficiency is not limited, specific examples thereof include vitamin A deficiency, vitamin D deficiency, vitamin E deficiency, vitamin K deficiency, vitamin B1 deficiency, vitamin B2 deficiency, vitamin B6 deficiency, pantothenic acid deficiency, niacin deficiency, folate deficiency, vitamin B12 deficiency, biotin deficiency, and vitamin C deficiency.

The compound of the invention is provided, for example, as a therapeutic agent and/or preventive agent for osteoporosis. Although the type of osteoporosis is not limited, specific examples thereof primary osteoporosis and secondary osteoporosis.

The compound of the invention is provided, for example, as a therapeutic agent and/or preventive agent for obesity.

The compound of the invention is provided, for example, as a therapeutic agent and/or preventive agent for central nervous system disease. Although the type of central nervous system disease is not limited, specific examples thereof include schizophrenia, depression, bipolar disorder, anxiety disorder, and adjustment disorder.

The compound of the invention is provided, for example, as a therapeutic agent and/or preventive agent for arthritis. Although the type of arthritis is not limited, specific examples thereof include acute monoarthritis, acute polyarthritis, chronic monoarthritis, and chronic polyarthritis.

Examples of acute monoarthritis include bacterial arthritis, crystal-induced arthritis, and traumatic arthritis. Examples of crystal-induced arthritis include gout and pseudogout.

Examples of acute polyarthritis include viral polyarthritis, gonococcal arthritis, and bacterial endocarditis.

Examples of chronic monoarthritis include chronic non-inflammatory monoarthritis and chronic inflammatory monoarthritis. Examples of chronic non-inflammatory monoarthritis include osteoarthritis, indolent osteonecrosis, neurogenic arthropathy, and chronic traumatic monoarthritis.

Examples of chronic polyarthritis include rheumatoid arthritis, polymyalgia rheumatica, crystal-induced arthritis, chronic polyarthritis associated with collagen disease, and psoriatic arthritis. Examples of crystal-induced arthritis include gout and pseudogout.

The compound of the invention is provided, for example, as a therapeutic agent and/or preventive agent for kidney disease. Although the type of kidney disease is not limited, specific examples thereof include acute kidney failure, chronic kidney failure, pyelonephritis, acute glomerulonephritis, chronic glomerulonephritis, renal pelvis cancer, kidney stones, ureteral stones, nephrotic syndrome, and IgA nephropathy.

The compound of the invention is provided, for example, as a therapeutic agent and/or preventive agent for inflammations. Although the type of inflammation is not limited, specific examples thereof include acute inflammation and chronic inflammation. Examples of acute inflammation include degenerative inflammation (parenchymal inflammation) and exudative inflammation (effusive inflammation and vascular inflammation). Examples of exudative inflammation include serous inflammation, fibrinous inflammation, suppurative inflammation, catarrhal inflammation, hemorrhagic inflammation, and septic inflammation. Examples of chronic inflammation include proliferative inflammation. Examples of proliferative inflammation include specific inflammation. Examples of specific inflammation include tuberculosis, syphilis, leprosy, sarcoidosis, tularemia, brucellosis, typhoid fever, and other mycosis.

The compound of the invention is provided, for example, as a therapeutic agent and/or preventive agent for diabetes. Although the type of diabetes is not limited, specific examples thereof include type 1 diabetes, type 2 diabetes, and secondary diabetes.

As used herein, "prevention" is an act of administering an active ingredient of the present invention to a healthy individual who has not developed a disease in order to, for example, prevent the onset of the disease. "Treatment (therapy)" is an act of administering an active ingredient of the present invention to a person (patient) diagnosed as having a disease by a physician in order to, for example, alleviate the disease or symptom, inhibit the growth of carcinoma, or restore a condition prior to the onset of the disease. Administration to prevent exacerbation of a disease or symptom or growth of carcinoma, if administered to a patient, is a therapeutic act.

The dosage of the compound of the invention administered varies depending on the symptom, age, administration method, or the like. For intravenous injections, an effect is expected by administering the compound, divided into one or several daily doses, to an adult with 0.01 mg (preferably 0.1 mg) as the lower limit and 1000 mg (preferably 30 mg) as the upper limit, depending on the symptom. Examples of dosing schedule thereof include single-dose administration, once daily administration for three consecutive days, and the like. Each administration described above can also be repeated at intervals of about 7 days to about 60 days.

For oral administrations, it is desirable to administer the compound, divided into one or several daily doses, to an adult with 0.01 mg (preferably 1 mg) as the lower limit and 5000 mg (preferably 500 mg) as the upper limit, depending on the symptom.

The compound of the invention can be formulated in a suitable dosage form for parenteral or oral administration. Examples of the dosage form include, but are not limited to, a tablet, a capsule, powder, granules, a liquid agent, a suspension, an injection, a patch, a poultice, and the like. A formulation can be manufactured by a known method using a pharmaceutically acceptable additive.

An excipient, disintegrator, binder, fluidizer, lubricant, coating agent, solubilizing agent, solubilizing adjuvant, thickener, dispersant, stabilizing agent, sweetener, flavoring agent, and the like can be used as an additive in accordance with the objective. Specific examples thereof include lactose, mannitol, crystalline cellulose, low substituted hydroxypropyl cellulose, corn starch, partially pregelatinized starch, carmellose calcium, croscarmellose sodium, hydroxypropyl cellulose, hydroxypropylmethyl cellulose, polyvinyl alcohol, magnesium stearate, sodium stearyl fumarate, polyethylene glycol, propylene glycol, titanium oxide, talc, and the like.

Examples of injectable liquid agent includes a solution, suspension, emulsion, and the like, including, for example, aqueous solution, water-propylene glycol solution, and the like. A liquid agent can be manufactured in form of a solution of polyethylene glycol or/and propylene glycol that may contain water. A liquid agent suitable for oral administration can be manufactured by adding the compound of the invention to water, as well as a coloring agent, flavoring agent, stabilizing agent, sweetener, solubilizing agent, thickener, or the like as needed. A liquid agent suitable for oral administration can also be manufactured by adding the compound of the invention to water with a dispersant and thickening the mixture. Examples of thickener include pharmaceutically acceptable natural or synthetic gum, resin, methylcellulose, sodium carboxymethylcellulose, known suspending agent, and the like.

Hereinafter, the present invention is described more specifically with the Reference Examples, Examples, and Test Examples, but the present invention is not limited thereto. Compounds were identified by an elemental analysis value, a mass spectrum, a high performance liquid chromatography-mass spectrometer; LC-MS, IR spectra, NMR spectra, high performance liquid chromatography (HPLC), and the like.

### [Examples]

Hereinafter, the present invention is described more specifically with the Reference Examples, Examples, and Test Examples, but the present invention is not limited thereto. The compound names denoted in the following Reference Examples and Examples are not necessarily in accordance with the IUPAC nomenclature. While abbreviations are sometimes used to simplify a description, these abbreviations are defined the same as the above descriptions.

The following abbreviations are sometimes used herein.

NMR and MS data of Reference Examples and Examples use the following abbreviations.

- Me:: methyl group
- tert:: tertiary
- t-Bu:: tert-butyl group
- Boc:: tert-butoxycarbonyl group
- s:: singlet
- brs:: broad singlet
- d:: doublet
- dd:: double doublet
- t:: triplet
- q:: quartet
- m:: multiplet
- br:: broad
- J:: coupling constant
- Hz:: Hertz
- CDCl₃:: deuterated chloroform
- DMSO-d₆:: deuterated dimethyl sulfoxide
- hr:: hour
- min:: minute

High performance liquid chromatography-mass spectrometer; LC-MS measurement conditions are the following. The observed mass spectrometry value [MS(m/z)] is indicated by [M+H]⁺ or [M+Na]⁺, and the retention time is indicated by Rt (min).

### Measurement condition A

Detector: ACQUITY® SQ detector (Waters)
HPLC: ACQUITY UPLC® system
Column: Waters ACQUITY UPLC® BEH C18 (1.7 um, 2.1 mm X 30 mm)
Solvent: solution A: 0.06% formic acid/H₂O, solution B: 0.06% formic acid/MeCN
Gradient condition: 0.0-1.3 min Linear gradient from B 2% to 96%
Flow rate: 0.8 mL/min
UV: 220 nm and 254 nm

### Measurement condition B

Detector: Shimadzu LCMS-IT-TOF
Column: Phenomenex Kinetex C8 (1.7 um, 2.1 mm X 50 mm)
Solvent: solution A: 0.1% formic acid/MeCN, solution B: 0.1% formic acid/H₂O
Gradient condition:
0.0 min; A/B = 60:40
0.0-1.4 min; Linear gradient from A 40% to 90%
1.4-1.6 min; A/B = 90:10
1.6-2.0 min; A/B = 10:90
Flow rate: 1.2 mL/min
UV: 220nm and 254nm

### Reference Example 1

### tert-Butyl N-(tert-butoxycarbonyl)-N-(2-isocyanatoethyl)glycinate

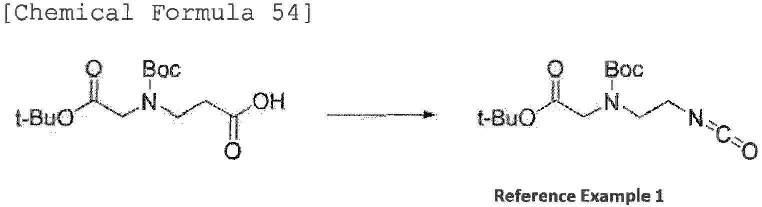

Diisopropylethylamine (1.76 mL) and diphenylphosphoryl azide (0.92 mL) were added to a toluene solution (20.0 mL) of N-(tert-butoxycarbonyl)-N-(2-tert-butoxy-2-oxoethyl)-β-alanine (1.00 g) and stirred for 15 minute at room temperature. After stirring for another 2 hours at 80°C, the mixture was cooled to room temperature to give the toluene solution in Reference Example 1. The resulting solution was directly used in the following reaction.

### Reference Example 2

### Di-tert-butyl 2,2'-[(2-acetylthieno[3,2-f][1]benzofuran-4,8-diyl)bis{oxycarbonyliminoethane-2,1-diyl[(tert-butoxycarbonyl)imino]}]diacetate

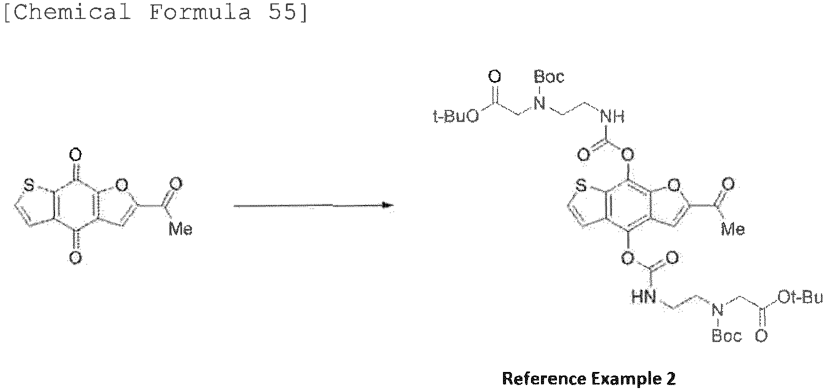

A suspension of 2-acetylthieno[3,2-f][1]benzofuran-4,8-dione (203 mg), zinc (270 mg), sodium dithionite (1.44 g), diisopropylethylamine (1.44 mL), and tetra-n-butylammonium bromide (26.6 mg) in N,N-dimethylformamide (20 mL) was stirred for 1 hour at room temperature under a nitrogen atmosphere. The reaction mixture was then cooled to 0°C, and a toluene solution of tert-butyl N- (tert-butoxycarbonyl) -N-(2-isocyanatoethyl)glycinate prepared in Reference Example 1 was added dropwise over 15 minutes. The mixture was stirred for another 1 hour at room temperature, and then ethyl acetate and aqueous saturated ammonium chloride solution were added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 1:1 to 1:4) to give Reference Example 2 (294 mg) .
(LC-MS: [M+H]⁺/Rt(min)) = 849/1.32 measurement condition A

### Example 1

### 2,2'-[(2-Acetylthieno[3,2-f][1]benzofuran-4,8-diyl)bis(oxycarbonyliminoethane-2,1-diylimino)]diacetic acid dihydrochloride

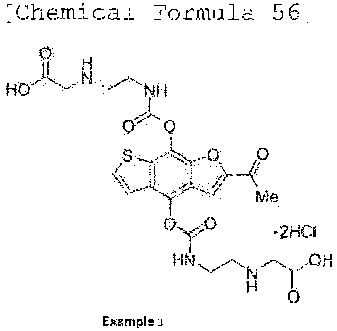

4 mol/L hydrochloric acid/dioxane solution (5.0 mL) was added to Reference Example 2 (294 mg), and the mixture was stirred for 2 hours at 50°C. The reaction solution was concentrated, and the residue was washed with ethyl acetate to give Example 1 (80 mg).
(LC-MS: [M+H]⁺/Rt(min) = 537/0.38 measurement condition A

### Reference Example 3

### Di-tert-butyl 2,2'-[(2-methylnaphthalene-1,4-diyl)bis{oxycarbonyliminoethane-2,1-diyl[(tert-butoxycarbonyl)imino]}]diacetate

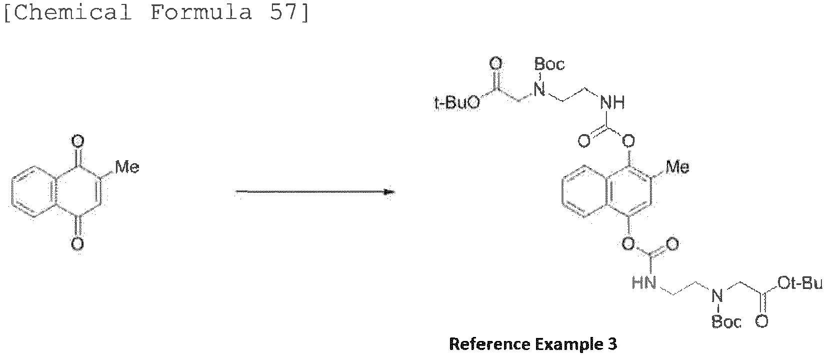

A suspension of 2-methylnaphthalen-1,4-dione (142 mg), zinc (539 mg), 4-dimethylaminopyridine (20 mg), and triethylamine (2.30 mL) in N,N-dimethylformamide (20 mL) was stirred for 1 hour at room temperature under a nitrogen atmosphere. The reaction mixture was then cooled to 0°C, and a toluene solution of tert-butyl N-(tert-butoxycarbonyl)-N-(2-isocyanatoethyl)glycinate prepared from N-(tert-butoxycarbonyl)-N-(2-tert-butoxy-2-oxoethyl)-β alanine (1.00 g) in the same manner as Reference Example 1 was added dropwise over 10 minutes. The mixture was stirred for 1 hour at 0°C, and then the reaction solution was filtered through celite, and the celite was washed with ethyl acetate. After adding an aqueous saturated ammonium chloride solution to the filtrate and separating the organic layer, the aqueous layer was further extracted once with ethyl acetate. The resulting organic layer was dried over anhydrous sodium sulfate, which was then filtered off, and the solvent was evaporated under reduced pressure. The residue was purified by silica gel column chromatography (hexane/ethyl acetate = 4:1 to 3:2) to give Reference Example 3 (120 mg).
(LC-MS: [M+Na]⁺/Rt(min)) = 797/1.33 measurement condition A

### Example 2

### 2,2'-[(2-Methylnaphthalene-1,4-diyl)bis(oxycarbonyliminoethane-2,1-diylimino)]diacetic acid dihydrochloride

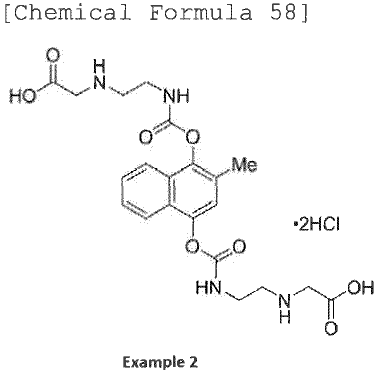

4 mol/L hydrochloric acid/dioxane solution (10.0 mL) was added to Reference Example 3 (120 mg) and stirred for 2 hours at 60°C. The reaction solution was concentrated, and the residue was washed with ethyl acetate to give Example 2 (40 mg) .
¹HNMR (400 MHz, DMSO-d6) δ2.29 (3H, s), 3.13 (4H, m), 3.31 (4H, m), 3.46 (4H, m), 7.25 (1H, s), 7.55-7.59 (2H, m), 7.83-7.91 (2H, m), 8.11-8.36 (2H, m).
(LC-MS: [M+H]⁺/Rt(min)) = 463/0.34 measurement condition A

### Reference Example 4

### tert-Butyl N-(tert-butoxycarbonyl)-N-{4-[(chlorocarbonyl)oxy]butyl}glycinate

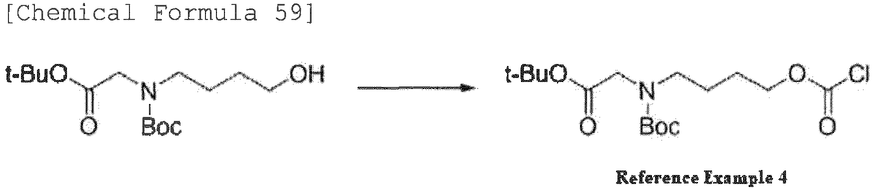

Diisopropylethylamine (5.74 mL) and triphosgene (342 mg) were added to a tetrahydrofuran (200.0 mL) solution of tert-butyl N-(tert-butoxycarbonyl)-N-{4-hydroxybutyl}glycinate (1.00 g) at 0°C and stirred for 1 hour at room temperature to give the tetrahydrofuran solution of Reference Example 4. The resulting solution was directly used in the following reaction.

### Reference Example 5

### Di-tert-butyl 2,2'-[(2-methylnaphthalene-1,4-diyl)bis{oxycarbonyloxybutane-4,1-diyl[(tert-butoxycarbonyl)imino] }]diacetate

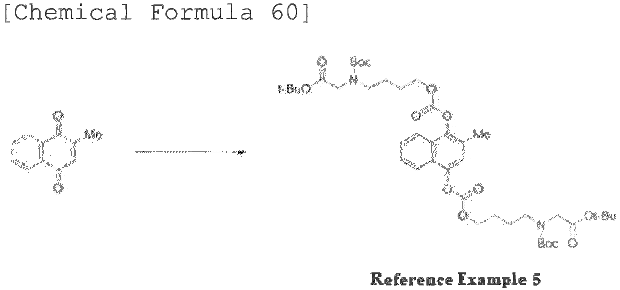

A suspension of 2-methylnaphthalen-1,4-dione (142 mg), zinc (539 mg), 4-dimethylaminopyridine (20 mg), and triethylamine (2.30 mL) in N,N-dimethylformamide (20 mL) was stirred for 1 hour at room temperature under a nitrogen atmosphere. The reaction mixture was then cooled to 0°C, and a toluene solution of tert-butyl N-(tert-butoxycarbonyl)-N-{4-[chlorocarbonyl)oxy]butyl}glycinate prepared in Reference Example 4 from tert-butyl N-(tert-butoxycarbonyl)-N-{4-hydroxybutyl}glycinate (1.00 g) was added dropwise over 10 minutes. The mixture was stirred for 1 hour at 0°C, and then the reaction solution was filtered through celite, and the celite was washed with ethyl acetate. An aqueous saturated ammonium chloride solution was added to the filtrate and the organic layer was separated. The aqueous layer was then further extracted once with ethyl acetate. The resulting organic layer was dried over anhydrous sodium sulfate, which was filtered off, and the solvent was evaporated under reduced pressure. The residue was purified by silica gel column chromatography (hexane/ethyl acetate = 4:1 to 1:1) to give Reference Example 5 (140 mg).
(LC-MS: [M+Na]⁺/Rt(min)) = 855/1.47 measurement condition A

### Example 3

### 2,2'-[(2-Methylnaphthalene-1,4-diyl)bis(oxycarbonyloxybutane-4,1-diylimino)]diacetic acid dihydrochloride

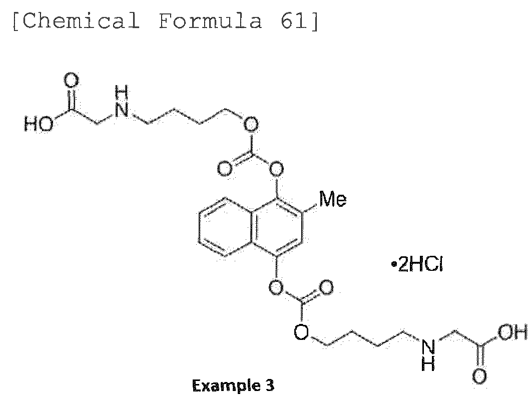

4 mol/L hydrochloric acid/dioxane solution (10.0 mL) was added to Reference Example 5 (140 mg) and stirred for 2 hours at 60°C. The reaction solution was concentrated, and the residue was washed with ethyl acetate to give Example 3 (40 mg) .
¹HNMR (400 MHz, DMSO-d6) δ1.70-1.90 (8H, m), 2.32 (3H, s), 2.90-2.10 (4H, m), 3.78-3.90 (4H, m), 4.31-4.45 (4H, m), 7.46 (1H, s), 7.55-7.70 (2H, m), 7.83-7.89 (2H, m), 9.20-9.30 (4H, m) .
(LC-MS: [M+Na]⁺/Rt(min)) = 521/0.51 measurement condition A

### Reference Example 6

### Di-tert-butyl 2,2'-[(2,2-dimethyl-3,4-dihydro-2H-benzo[H]chromene-5,6-diyl)bis{oxycarbonyliminoethane-2,1-diyl[(tert-butoxycarbonyl)imino] }]diacetate

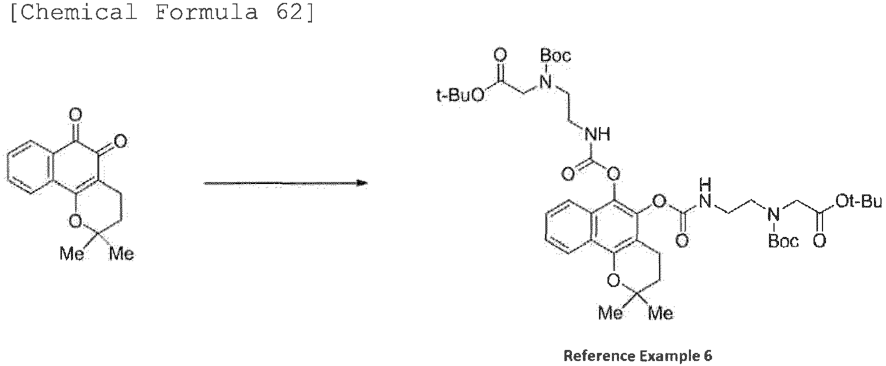

A suspension of 2,2-dimethyl-3,4,4a,10b-tetrahydro-2H-benzo[h]chromen-5,6-dione (200 mg), zinc (324 mg), sodium dithionite (862 mg), diisopropylethylamine (1.44 mL), and tetra-n-butylammonium bromide (26.6 mg) in N,N-dimethylformamide (20 mL) was stirred for 1 hour at room temperature under a nitrogen atmosphere. The reaction mixture was then cooled to 0°C, and a toluene solution of tert-butyl N-(tert-butoxycarbonyl)-N-(2-isocyanatoethyl)glycinate prepared from N-(tert-butoxycarbonyl)-N-(2-tert-butoxy-2-oxyethyl)-β-alanine (1.00 g) in the same manner as Reference Example 1 was added dropwise over 15 minutes. The mixture was stirred for another 1 hour at room temperature, and then ethyl acetate and aqueous saturated ammonium chloride solution were added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 1:1 to 1:4) to give Reference Example 6 (50 mg).
(LC-MS: [M+H]⁺/Rt(min)) = 845/1.44 measurement condition A

### Example 4

### 2,2'-[(2,2-Dimethyl-3,4-dihydro-2H-benzo[H]chromene-5,6-diyl)bis(oxycarbonyliminoethane-2,1-diylimino)]diacetic acid dihydrochloride

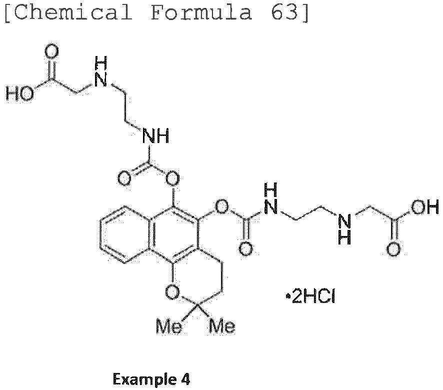

4 mol/L hydrochloric acid/dioxane solution (5.0 mL) was added to Reference Example 6 (50 mg) and stirred for 2 hours at 60°C. The reaction solution was concentrated, and the residue was washed with ethyl acetate to give Example 4 (20 mg) .
¹HNMR (400 MHz, DMSO-d6) δ 1.37 (6H, s), 1.85-1.98 (2H, m), 2.55-2.70 (2H, m), 3.10-3.30 (4H, m), 3.40-3.50 (4H, m), 3.90-4.00 (4H, m), 7.47-7.53 (2H, m), 7.75-7.78 (1H, m), 8.05-8.10 (1H, m), 8.22-8.30 (2H, m).
(LC-MS: [M+Na]⁺/Rt(min)) = 533/0.64 measurement condition A

### Reference Example 7

### 2-Acetyl-5-(methoxymethoxy)naphtho[2,3-b]furan-4,9-dione

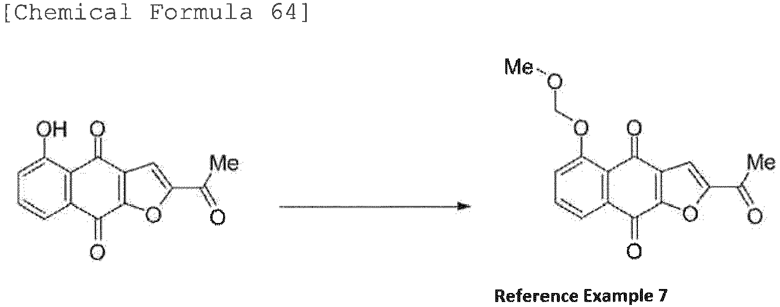

Chloromethyl methyl ether (0.71 mL) was added to a suspension of 2-acetyl-5-hydroxynaphtho[2,3-b]furan-4,9-dione (800 mg) and diisopropylethylamine (5.44 mL) in dichloroethane (50 mL) and the reaction mixture was stirred for 2 hours at 50°C under a nitrogen atmosphere. The reaction solution was then evaporated under reduced pressure. The residue was purified by silica gel column chromatography (hexane/ethyl acetate = 2:1 to 1:4) to give Reference Example 7 (500 mg).
(LC-MS: [M+H]⁺/Rt(min)) = 301/0.80 measurement condition A

### Reference Example 8

### 2-Acetyl-4-hydroxy-5-(methoxymethoxy)naphtho[2,3-b]furan-9-yl tert-butyl propane-1,3-diyl biscarbamate

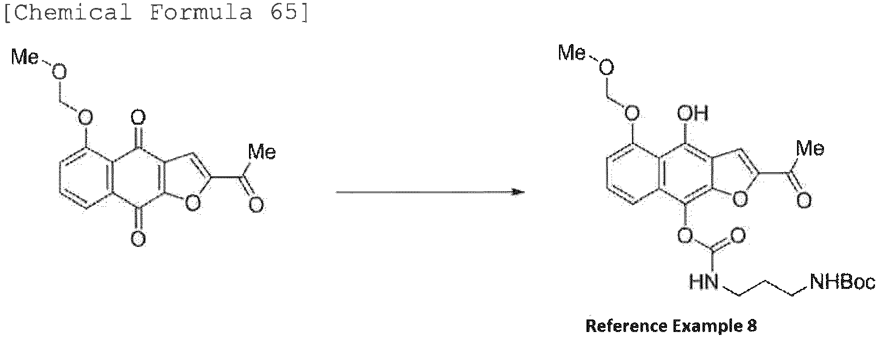

A suspension of Reference Example 7 (100 mg), zinc (131 mg), sodium dithionite (348 mg), diisopropylethylamine (0.58 mL), and tetra-n-butylammonium bromide (10.7 mg) in N,N-dimethylformamide (20 mL) was stirred for 1 hour at room temperature under a nitrogen atmosphere. The reaction mixture was then cooled to 0°C, and a toluene solution of tert-butyl(3-isocyanatopropyl)carbamate prepared by a method similar to Reference Example 1 from 4-[(tert-butoxycarbonyl)amino] butanoic acid (271 mg) was added dropwise over 15 minutes. The mixture was stirred for another 1 hour at room temperature, and then ethyl acetate and aqueous saturated ammonium chloride solution were added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 1:1 to 1:10) to give Reference Example 8 (98 mg).
LC-MS: [M+H]⁺/Rt(min)) = 503/1.11 measurement condition A

### Reference Example 9

### 2-Acetyl-4,5-dihydroxynaphtho[2,3-b]furan-9-yl (3-aminopropyl)carbamate hydrochloride

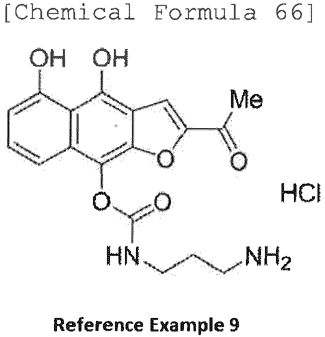

4 mol/L hydrochloric acid/dioxane solution (5.0 mL) was added to Reference Example 8 (98 mg) and stirred for 2 hours at 0°C. The reaction solution was concentrated, and the residue was washed with ethyl acetate to give Reference Example 9 (40 mg).
(LC-MS: [M+H]⁺/Rt(min)) = 359/0.48 measurement condition A

### Reference Example 10

### tert-Butyl 10-(4,5-dimethoxy-2-methyl-3,6-dioxocyclohexa-1,4-dien-1-yl)decayl carbonate

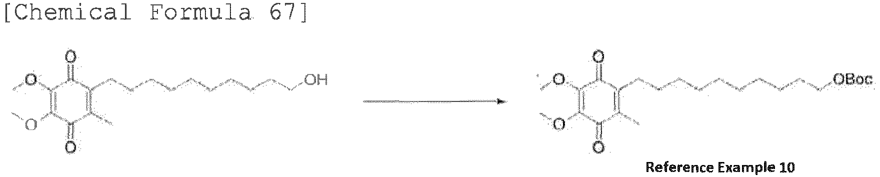

A THF solution (3 mL) of 2-(10-(hydroxydecyl)-5,6-dimethoxy-3-methylcyclohexa-2,5-dien-1,4-dione (100 mg), di-tert-butylcarbonate (97 mg), diisopropylethylamine (0.10 mL), and N,N-dimethylaminopyridine (1.8 mg) was stirred for 28 hours at room temperature under a nitrogen atmosphere. The reaction solution was concentrated, and the residue was purified by silica gel column chromatography (hexane/ethyl acetate = 6:1 to 1:3) to give Reference Example 10 (51 mg).
(LC-MS: [M+H]⁺/Rt(min)) = 439/1.40 measurement condition A

### Reference Example 11

### Dimethyl (2S,2'S)-4,4'-[(2-{10-[(tert-butoxycarbonyl)oxy]decayl}-5,6-dimethoxy-3-methylbenzene-1,4-diyl)bis(oxycarbonylimino)]bis{2-[(tert-butoxycarbonyl)amino]butanoate}

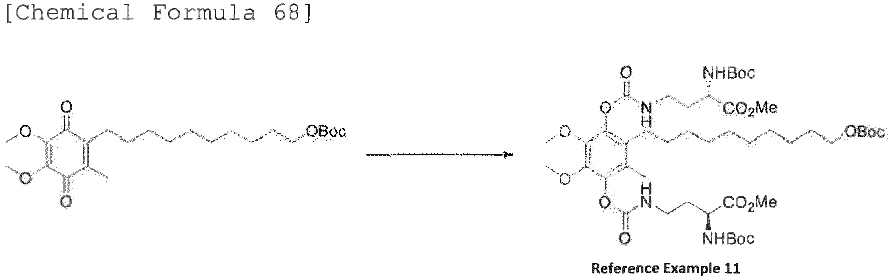

A suspension of Reference Example 10 (50 mg), zinc (119 mg), sodium dithionite (159 mg), diisopropylethylamine (0.20 mL), and tetra-n-butylammonium bromide (7.4 mg) in N,N-dimethylformamide (2.0 mL) was stirred for 1.5 hours at room temperature under a nitrogen atmosphere. The reaction mixture was then cooled to 0°C, and a toluene solution of methyl (3S)-3-[(tert-butoxycarbonyl)amino]-5-isocyanatopentanoate prepared by a method similar to Reference Example 1 from (4S)-4-[(tert-butoxycarbonyl)amino]-5-methoxy-5-oxopentanoic acid (0.12 g) was added dropwise over 5 minutes. The mixture was stirred for another 1.5 hours at 0°C, and then ethyl acetate and aqueous saturated ammonium chloride solution were added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 1:0 to 1:9) to give Reference Example 11 (57 mg).
(LC-MS: [M+Na]⁺/Rt(min)) = 979/1.36 measurement condition A

### Example 5

### Dimethyl (2S,2'S)-4,4'-[(2-{10-[(tert-butoxycarbonyl)oxy]decayl}-5,6-dimethoxy-3-methylbenzene-1,4-diyl)bis(oxycarbonylimino)]bis(2-aminobutanoate) dihydrochloride

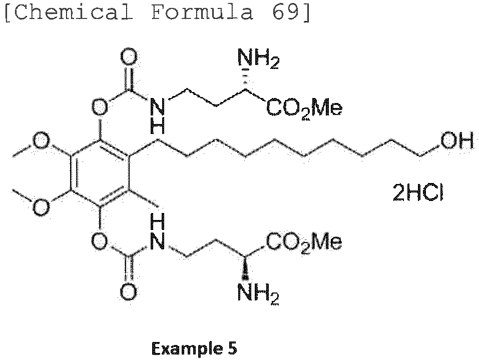

4 mol/L hydrochloric acid/dioxane solution (1.0 mL) was added to Reference Example 11 (36 mg), and the mixture was stirred for 30 minutes at 50°C. The reaction solution was concentrated, and the residue was washed with chloroform to give Example 5 (20 mg).
¹HNMR (400 MHz, DMSO-d6) δ1.25-1.41 (16H, m), 1.94-2.09 (4H, m), 2.02 (3H, s), 2.40-2.45 (2H, m), 3.20-3.26 (4H, m), 3.30-3.50 (2H, m), 3.73 (6H, s), 3.78 (6H, s), 4.08-4.14 (2H, m), 7.98 (2H, m), 8.48 (6H, br).
(LC-MS: [M+H]⁺/Rt(min)) = 657/0.61 measurement condition A

### Reference Example 12

### 4,7-diphenyl-1,3-benzodioxole-5,6-diyl bis({3-[(tert-butoxycarbonyl)amino]propyl}carbamate)

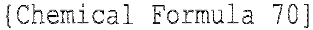

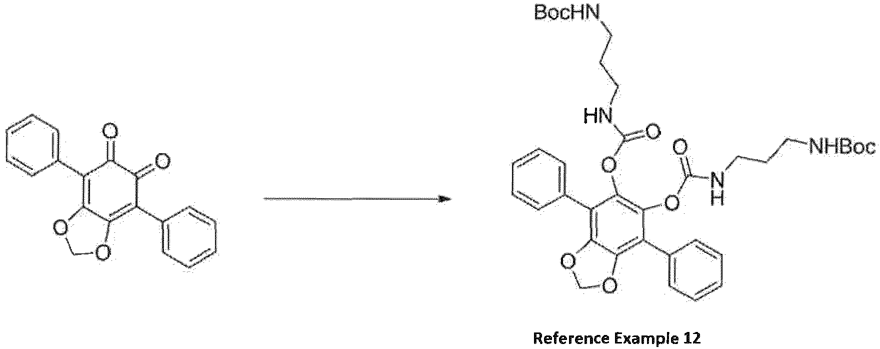

A suspension of 4,7-diphenyl-1,3-benzodioxol-5,6-dione (0.10 g), zinc (0.17 g), sodium dithionite (0.20 g), diisopropylethylamine (0.23 mL), and tetra-n-butylammonium bromide (11 mg) in N,N-dimethylformamide (3.0 mL) was stirred for 1 hour at room temperature under a nitrogen atmosphere. The reaction mixture was then cooled to 0°C, and a toluene solution of tert-butyl (3-isocyanatopropyl)carbamate prepared by a method similar to Reference Example 1 from 4-[(tert-butoxycarbonyl)amino]butanoic acid (271 mg) was added dropwise over 15 minutes. The mixture was stirred for another 30 minutes at 0°C, and then the reaction solution was filtered through celite. After diluting the filtrate with an aqueous 10% potassium hydrogen sulfate solution, the solution was extracted twice with ethyl acetate. The resulting organic layer was dried over anhydrous sodium sulfate, which was then filtered off, and the solvent was evaporated under reduced pressure. The residue was purified by silica gel column chromatography (chloroform/ethyl acetate = 1:0 to 1:1) to give Reference Example 12 (0.18 g).
(LC-MS: [M+H]⁺/Rt(min)) = 707/1.13 measurement condition A

### Example 6

### 4,7-Diphenyl-1,3-benzodioxole-5,6-diyl bis[(3-aminopropyl)carbamate] dihydrochloride

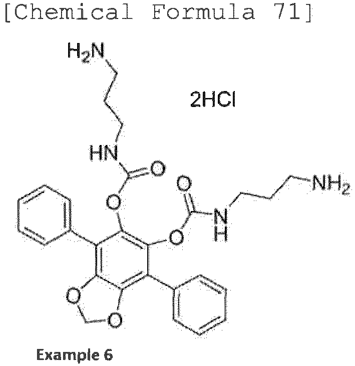

4 mol/L hydrochloric acid/dioxane solution (3.0 mL) was added to a chloroform solution (3.0 mL) of Reference Example 12 (0.18 g) and stirred for 1 hour at room temperature. The reaction solution was concentrated, and the residue was washed with chloroform to give Example 6 (62 mg).
¹HNMR (400 MHz, DMSO-d6) δ 1.64 (4H, m), 2.66 (4H, m), 3.02 (4H, m), 6.08 (2H, s), 7.38-7.43 (2H, m), 7.45-7.49 (8H, m), 7.76 (2H, m), 7.90 (6H, m).
(LC-MS: [M+2H]²⁺/Rt(min)) = 254/0.52 measurement condition A

### Reference Example 13

### Di-tert-butyl 2,2'-(1,1':4',1"-terphenyl-2',5'-diylbis{oxycarbonyliminoethane-2,1-diyl[(tert-butoxycarbonyl)imino]})diacetate

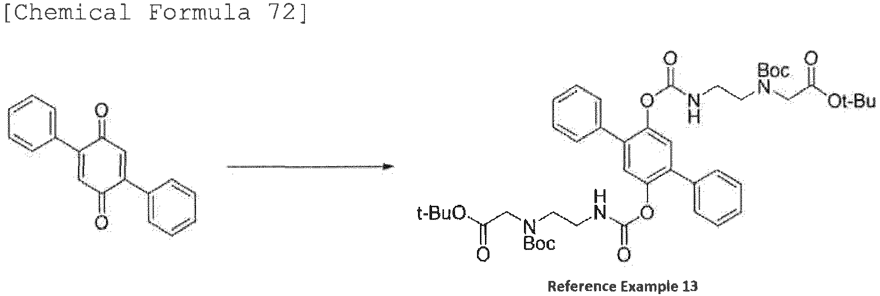

A suspension of 2,5-diphenylcyclohex-2,5-dien-1,4-dione (0.10 g), zinc (0.20 g), sodium dithionite (0.33 g), diisopropylethylamine (0.34 mL), and tetra-n-butylammonium bromide (25 mg) in N,N-dimethylformamide (4.5 mL) was stirred for 1 hour at room temperature under a nitrogen atmosphere. The reaction mixture was then cooled to 0°C, and a toluene solution of tert-butyl N-(tert-butoxycarbonyl)-N-(2-isocyanatoethyl)glycinate prepared by a method similar to Reference Example 1 from N-(tert-butoxycarbonyl)-N-(2-tert-butoxy-2-oxoethyl)-β-alanine (1.00 g) was added dropwise over 2 minutes. The mixture was stirred for another 1.5 hours at 0°C, and then ethyl acetate and aqueous saturated ammonium chloride solution were added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 9:1 to 1:1) to give Reference Example 13 (57 mg).
(LC-MS: [M+Na]⁺/Rt(min)) = 885/1.41 measurement condition A

### Example 7

### 2,2'-[1,1':4',1"-Terphenyl-2',5'-diylbis(oxycarbonyliminoethane-2,1-diylimino)]diacetic acid dihydrochloride

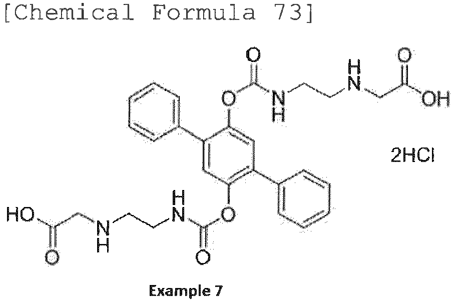

4 mol/L hydrochloric acid/dioxane solution (1.0 mL) was added to Reference Example 13 (74 mg), and the mixture was stirred for 3 hours at 50°C and then 2 hours at 70°C. The reaction solution was concentrated, and the residue was washed with chloroform to give Example 7 (41 mg).
¹HNMR (400 MHz, DMSO-d6) δ2.95 (4H, m), 3.14 (4H, m), 3.81 (4H, s), 7.32 (2H, s), 7.39-7.50 (10H, m), 7.93-7.98 (2H, m).
(LC-MS: [M+H]⁺/Rt(min)) = 551/0.45 measurement condition A

### Reference Example 14

### tert-Butyl (3S,4R)-4-[2-(2-chlorophenyl)-5,7-dihydroxy-4-oxo-4H-chromen-8-yl]-1-methylpiperidin-3-yl carbonate

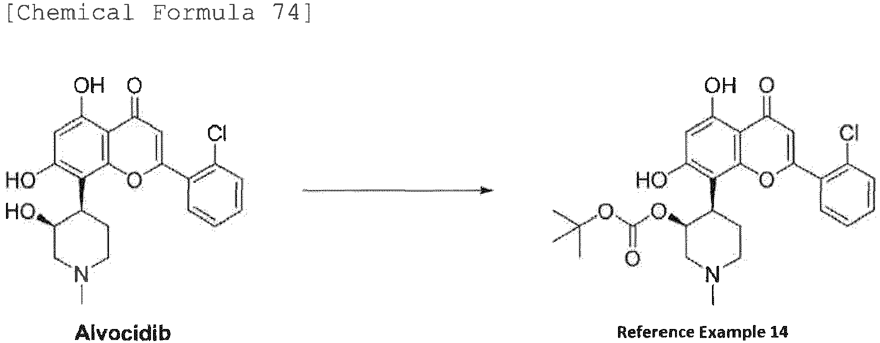

A methylene chloride (10.0 mL) solution of alvocidib (1.00 g), di-tert-butyl-dicarbonate (1.36 g), and triethylamine (1.74 mL) was stirred for 1 hour at room temperature under a nitrogen atmosphere. The mixture was then cooled to 0°C, and potassium carbonate (2.00 g) was added. The reaction mixture was stirred for 1 hour at room temperature. Ethyl acetate and aqueous saturated ammonium chloride solution were then added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 9:1 to 1:1) to give Reference Example 14 (1.20 g).
(LC-MS: [M+H]⁺/Rt(min)) = 502/0.754 measurement condition A

### Reference Example 15

### (3S,4R)-4-[7-{[{2-[(tert-Butoxycarbonyl)amino]ethyl}(methyl)carbamoyl]oxy}-2-(2-chlorophenyl)-5-hydroxy-4-oxo-4H-chromen-8-yl]-1-methylpiperidin-3-yl tert-butyl carbonate

### Reference Example 16

### (3S,4R)-4-[5-{[{2-[(tert-butoxycarbonyl)amino]ethyl}(methyl)carbamoyl]oxy}-2-(2-chlorophenyl)-7-hydroxy-4-oxo-4H-chromen-8-yl]-1-methylpiperidin-3-yl tert-butyl carbonate

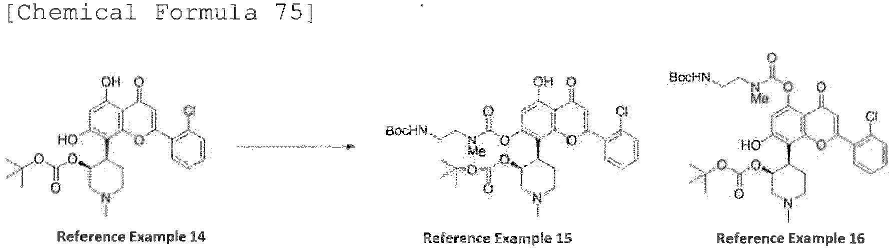

A chloroform (1.00 mL) solution of Reference Example 14 (118 mg) and triethylamine (0.20 mL) was stirred for 5 minutes at room temperature under a nitrogen atmosphere. The mixture was then cooled to 0°C, and 4-nitrophenyl chloroformate (120 mg) was added. The reaction mixture was stirred for 1 hour at room temperature. tert-Butyl 2-(methylamino) ethylcarbonate (443 mg) was then added at 0°C and stirred for 85 minutes at room temperature. An aqueous saturated ammonium chloride solution was added to the reaction solution, and then extracted twice with ethyl acetate. The resulting organic layer was dried over anhydrous sodium sulfate, which was then filtered off, and the solvent was evaporated under reduced pressure. The residue was purified by silica gel column chromatography (hexane/ethyl acetate = 5:1 to 3:2) to give Reference Example 15 (41.0 mg) and Reference Example 16 (32.0 mg).
Reference Example 15: (LC-MS: [M+H]⁺/Rt(min)) = 702/1.02 measurement condition A
Reference Example 16: (LC-MS: [M+H]⁺/Rt(min)) = 702/0.93 measurement condition A

### Example 8

### 2-(2-chlorophenyl)-5-hydroxy-8-[(3S,4R)-3-hydroxy-1-methylpiperidin-4-yl]-4-oxo-4H-chromen-7-yl (2-aminoethyl) methylcarbamate ditrifluoroacetate

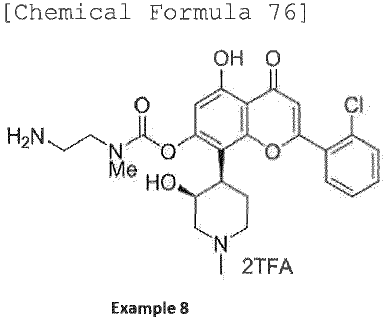

A methylene chloride (2.00 mL) solution of Reference Example 15 (41.0 mg) was stirred for 5 minutes at room temperature under a nitrogen atmosphere. Trifluoroacetic acid (0.20 mL) was added and the reaction mixture was then stirred for 3 hours at room temperature. The reaction solution was concentrated. The residue was washed with chloroform to give Example 8 (20.0 mg).
¹HNMR (400 MHz, CDCl3) δ 1.85-1.95 (1H, m), 2.74-3.22 (12H, m), 3.26-3.46 (2H, m), 3.53-3.68 (2H, m), 4.31 (1H, s), 6.52 (1H, s), 6.55 (1H, s), 7.41-7.49 (1H, m), 7.49-7.58 (3H, m), 7.73 (2H, m), 10.21 (1H, s).
(LC-MS: [M+H]⁺/Rt(min)) = 502/0.68 measurement condition A

### Example 9

### 2-(2-chlorophenyl)-7-hydroxy-8-[(3S,4R)-3-hydroxy-1-methylpiperidin-4-yl]-4-oxo-4H-chromen-5-yl (2-aminoethyl)methylcarbamate ditrifluoroacetate

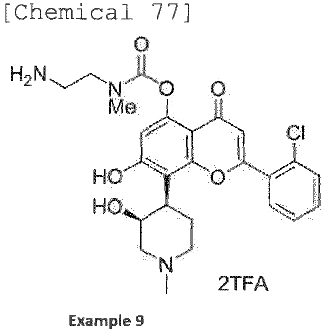

A methylene chloride (1.00 mL) solution of Reference Example 16 (32.0 mg) was stirred for 5 minutes at room temperature under a nitrogen atmosphere. Trifluoroacetic acid (0.10 mL) was added and the reaction mixture was then stirred for 3 hours at room temperature. The reaction solution was concentrated. The residue was washed with chloroform to give Example 9 (14.0 mg).
¹HNMR (400 MHz, DMSO-d6) δ 1.86-1.90 (1H, m), 2.68 (3H, s), 2.94-3.19 (6H, m), 3.51-3.55 (2H, m), 3.60-3.70 (1H, m), 4.15 (1H, s), 6.41 (1H, s), 6.69 (1H, s), 7.54-7.87 (4H, m), 9.30-9.40 (1H, m), 11.30-11.45 (1H, m).
(LC-MS: [M+H]⁺/Rt(min)) = 502/0.49 measurement condition A

### Reference Example 17

### (3S,4R)-4-[7-{[{2-[(tert-Butoxycarbonyl)(methyl)amino]ethyl}(methyl)carbamoyl]oxy}-2-(2-chlorophenyl)-5-hydroxy-4-oxo-4H-chromen-8-yl]-1-methylpiperidin-3-yl tert-butyl carbonate

### Reference Example 18

### (3S,4R)-4-[5-{[{2-[(tert-butoxycarbonyl)(methyl)amino]ethyl}(methyl)carbamoyl]oxy}-2-(2-chlorophenyl)-7-hydroxy-4-oxo-4H-chromen-8-yl]-1-methylpiperidin-3-yl tert-butyl carbonate

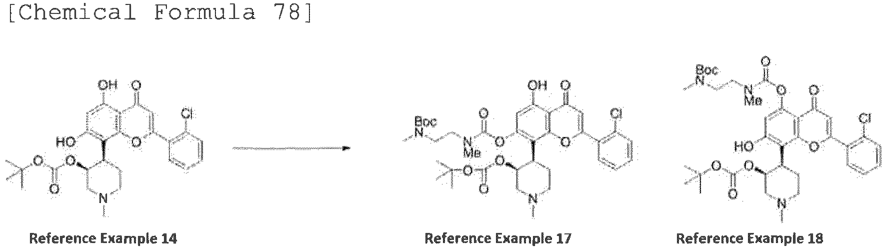

A chloroform (1.00 mL) solution of Reference Example 14 (118 mg) and triethylamine (0.20 mL) was stirred for 5 minutes at room temperature under a nitrogen atmosphere. The mixture was then cooled to 0°C, and 4-nitrophenyl chloroformate (120 mg) was added. The reaction mixture was stirred for 1 hour at room temperature. tert-Butyl 2-(methylamino) ethylcarbonate (443 mg) was then added at 0°C and stirred for 85 minutes at room temperature. An aqueous saturated ammonium chloride solution was added to the reaction solution, and then extracted twice with ethyl acetate. The resulting organic layer was dried over anhydrous sodium sulfate, which was then filtered off, and the solvent was evaporated under reduced pressure. The residue was purified by silica gel column chromatography (hexane/ethyl acetate = 5:1 to 3:2) to give Reference Example 17 (41.0 mg) and Reference Example 18 (32.0 mg).
Reference Example 17: (LC-MS: [M+H]⁺/Rt(min)) = 716/0.95 measurement condition A
Reference Example 18: (LC-MS: [M+H]⁺/Rt(min)) = 716/0.83 measurement condition A

### Example 10

### 2-(2-Chlorophenyl)-5-hydroxy-8-[(3S,4R)-3-hydroxy-1-methylpiperidin-4-yl]-4-oxo-4H-chromen-7-yl methyl[2-(methylamino)ethyl]carbamate ditrifluoroacetate

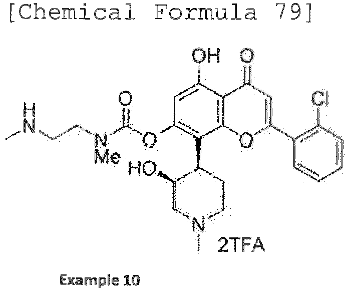

A methylene chloride (2.00 mL) solution of Reference Example 17 (41.0 mg) was stirred for 5 minutes at room temperature under a nitrogen atmosphere. Trifluoroacetic acid (0.20 mL) was added and the reaction mixture was then stirred for 3 hours at room temperature. The reaction solution was concentrated. The residue was washed with chloroform to give Example 10 (20.0 mg).
¹HNMR (400 MHz, DMSO-d6) δ 1.68-1.72 (1H, m), 2.26 (3H, s), 2.85-3.74 (24H, m), 4.95 (1H, s), 6.55 (1H, s), 6.62 (1H, m), 7.26-7.74 (4H, m), 12.66 (1H, s).
(LC-MS: [M+H]⁺/Rt(min)) = 516/0.60 measurement condition A

### Example 11

### 2-(2-Chlorophenyl)-7-hydroxy-8-[(3S,4R)-3-hydroxy-1-methylpiperidin-4-yl]-4-oxo-4H-chromen-5-yl methyl[2-(methylamino)ethyl]carbamate ditrifluoroacetate

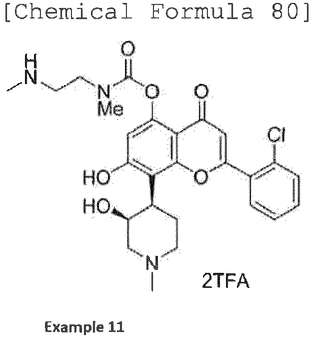

A methylene chloride (1.00 mL) solution of Reference Example 18 (32.0 mg) was stirred for 5 minutes at room temperature under a nitrogen atmosphere. Trifluoroacetic acid (0.10 mL) was added and the reaction mixture was then stirred for 3 hours at room temperature. The reaction solution was concentrated. The residue was washed with chloroform to give Example 11 (14.0 mg).
¹HNMR (400 MHz, DMSO-d6) δ 1.68-1.77 (1H, m), 2.75-2.77 (3H, m), 2.80-2.99 (2H, m), 2.85 (3H, s), 3.00-3.25 (2H, m), 3.08 (3H, s), 3.17 (3H, s), 3.31-3.41 (3H, m), 3.65-4.00 (5H, m), 4.37 (1H, s), 6.42 (1H, s), 6.72 (1H, m), 7.20-7.57 (4H, m).
(LC-MS: [M+H]⁺/Rt(min)) = 516/0.60 measurement condition A

### Reference Example 19

### Di-tert-butyl 2,2'-([(1R)-1,6,6-trimethyl-1,2,6,7,8,9-hexahydrophenanthro[1,2-b]furan-10,11-diyl]bis{oxycarbonylazanediylethane-2,1-diyl[(tert-butoxycarbonyl) azanediyl]})diacetate

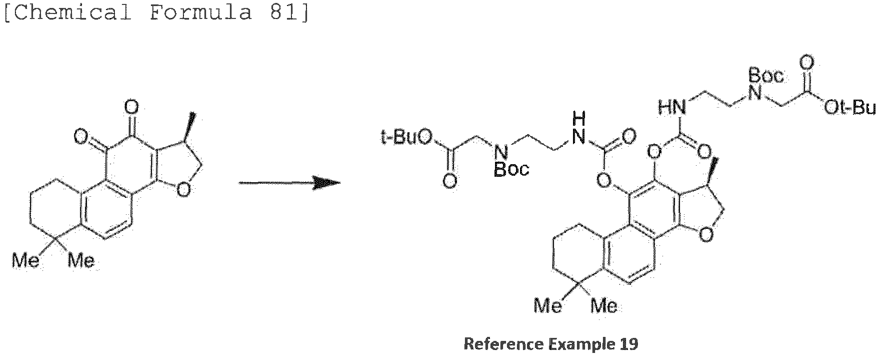

A suspension of (1R)-1,6,6-trimethyl-1,2,6,7,8,9-hexahydrophenanthro[1,2-b]furan-10,11-dione (270 mg), zinc (323 mg), sodium dithionite (860 mg), diisopropylethylamine (1.6 mL), and tetra-n-butylammonium bromide (29 mg) in N,N-dimethylformamide (10 mL) was stirred for 1 hours at room temperature under a nitrogen atmosphere. The reaction mixture was then cooled to 0°C, and a toluene solution of tert-butyl N-(tert-butoxycarbonyl)-N-(2-isocyanatoethyl)glycinate prepared from N-(tert-butoxycarbonyl)-N-(2-tert-butoxy-2-oxoethyl)-β-alanine (1.00 g), in a similar manner to Reference Example 1, was added dropwise over 15 minutes. The mixture was stirred for an addition 1 hour at room temperature, and then ethyl acetate and aqueous saturated ammonium chloride solution were added to the reaction solution. The resulting mixture was filtered through celite, and then the filtrate was partitioned between an organic layer and an aqueous layer. The aqueous layer was extracted twice with ethyl acetate. The resulting organic layer was washed once with water, and dried over anhydrous sodium sulfate, which was then filtered off, and the solvent was evaporated under reduced pressure. The residue was purified by silica gel column chromatography (hexane/ethyl acetate = 1:1 to 1:4) to give Reference Example 19 (820 mg).
(LC-MS: [M+Na]⁺/Rt(min)) = 921.45/1.60 measurement condition B

### Example 12

### 2,2'-{[(1R)-1,6,6-trimethyl-1,2,6,7,8,9-hexahydrophenanthro [1,2-b]furan-10,11-diyl]bis(oxycarbonylazanediylethane-2,1-diylazanediyl)}diacetic acid dihydrochloride

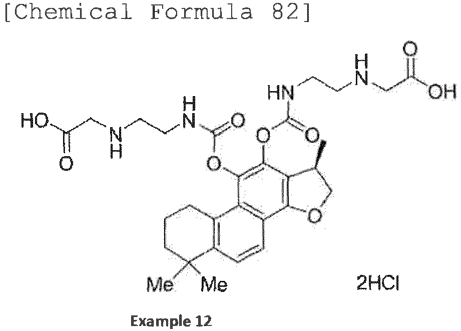

4 mol/L hydrochloric acid/dioxane solution (5 mL) of Reference Example 19 (152 mg) was stirred for 3 hours at 60°C under a nitrogen atmosphere. The reaction solution was concentrated, ethyl acetate (10 mL) was added to the residue, and the mixture was stirred for 1 hour at room temperature. The resulting solid was collected by filtration and washed with ethyl acetate to give Example 12 (111 mg).
¹HNMR (400 MHz, DMSO-d6) δ 1.25 (3H, d, J = 7.3 Hz), 1.28 (3H, s), 1.29 (3H, s), 1.61-1.62 (2H, m), 1.72-1.74 (2H, m), 3.09 (8H, t, J = 6.7 Hz), 3.66-3.74 (1H, m), 3.88 (4H, s), 4.28 (1H, dd, J = 9.2, 6.7 Hz), 4.84 (1H, t, J = 8.9 Hz), 7.49 (1H, d, J = 8.5 Hz), 7.65 (1H, d, J = 9.2 Hz), 8.15 (1H, dd, J = 11.3, 5.8 Hz), 8.10 (1H, dd, J = 14.0, 8.5 Hz).
(LC-MS: [M+2H]⁺²/Rt(min)) = 294/0.73 measurement condition A

### Reference Example 20

### Di-tert-butyl 2,2'-([(1R)-1,6,6-trimethyl-1,2,6,7,8,9-hexahydrophenanthro[1,2-b]furan-10,11-diyl]bis{oxycarbonylazanediylpropane-3,1-diyl[(tert-butoxycarbonyl)azanediyl]})diacetate

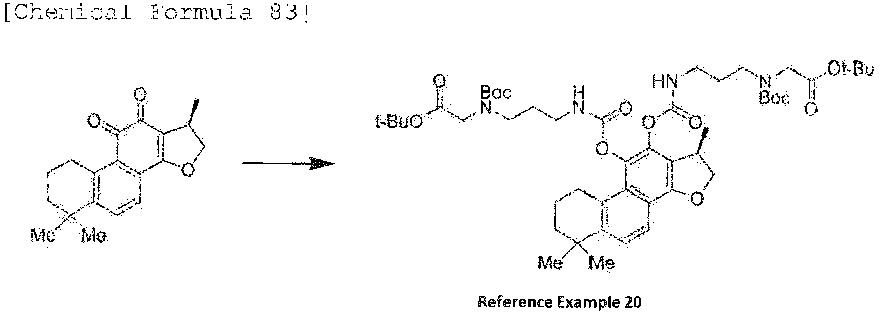

A toluene solution of tert-butyl N-(tert-butoxycarbonyl)-N-(3-isocyanatopropyl)glycinate obtained by the same reaction and processing as Reference Example 1 from known 4-[(tert-butoxycarbonyl)(2-tert-butoxy-2-oxoethyl)amino]butanoic acid (759 mg) and (1R)-1,6,6-trimethyl-1,2,6,7,8,9-hexahydrophenanthro[1,2-b]furan-10,11-dione (236 mg) was used as the raw material compound, which was subjected to the same reaction and processing as Reference Example 19 to give Reference Example 20 (739 mg).
(LC-MS: [M+Na]⁺/Rt(min)) = 949.46/1.65 measurement condition B

### Example 13

### 2,2'-{[(1R)-1,6,6-trimethyl-1,2,6,7,8,9-hexahydrophenanthro[1,2-b]furan-10,11-diyl]bis(oxycarbonylazanediylpropane-3,1-diylazanediyl)}diacetic acid dihydrochloride

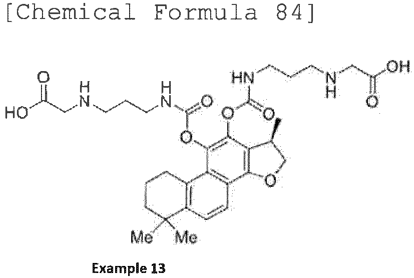

Reference Example 20 (121 mg) was used as the raw material compound and subjected to the same reaction and processing as Example 12 to give Example 13 (90 mg).
(LC-MS: [M+2H]⁺²/Rt(min)) = 308/0.82 measurement condition B

### Test Examples

Hereinafter, the chemical features and pharmacokinetics of the representative compounds of the invention are described while providing test results for the compounds, but the present invention is not limited to the Test Examples.

### Test Example 1. Solution stability test

The stability at each pH was evaluated for Example 1, Example 2, Example 4, Reference Example 9, Example 6, Example 8, Example 9, Example 10, and Example 11. The test compounds were incubated at 25°C at a concentration of 25 umol/L in a buffer prepared to have a pH of 2.0, 3.0, 5.0, 7.4, and 9.0. The compounds were measured by HPLC after 0, 3, 6, 9, 12, and 24 hours.

The buffers used at each pH are the following.
pH 2.0: 50 mmol/L glycine buffer
pH 3.0: 50 mmol/L citrate buffer
pH 5.0: 50 mmol/L citrate buffer
pH 7.4: 50 mmol/L phosphate buffer
pH 9.0: 50 mmol/L glycine buffer

The measurement conditions for HPLC are the following.
HPLC conditions
Column: Acquity UPLC BEH C18, 1.7 um, 50 x 2.1 mm
Column temperature: 40°C
Mobile phase:
A: 0.1% trifluoroacetic acid containing water
B: acetonitrile
A/B (min): 95/5(0) → 0/100 (3.5) → 0/100 (4) → 95/5 (4.01) → 95/5(5)
Flow rate: 0.8 mL/min
Detection: UV visible detector, measured wavelength 254 nm
Injected volume: 5 uL

The test results are shown in Tables 1, 2, 3, 4, 5, 6, 7, 8, 9, 9a, 9b, and 10.

It was confirmed that Example 1 is very stable at a pH of 2.0, and is sufficiently stable, although some degradation was observed, at a pH of 3.0. It was confirmed that the residual ratio after 9 hours was 0% at a pH of 5.0, and the residual ratio was already 0% immediately after starting the test at a pH of 7.4 and 9.0.

It was confirmed that Example 2 is very stable at a pH of 2.0, 3.0, and 5.0, and the residual ratio was 0% after 12 hours at a pH of 7.4, and the residual ratio was 0% after 3 hours at a pH of 9.0.

It was confirmed that Example 4 has sufficient stability at a pH of 2.0 and 3.0. It was confirmed that the residual ratio was 78% after 24 hours at a pH of 5.0, and the residual ratio had a value close to 0% after 3 hours at a pH of 7.4 and immediately after starting the test at a pH of 9.0.

It was confirmed that Reference Example 9 has sufficient stability, although some degradation was observed, at a pH of 2.0 and 3.0. It was confirmed that the residual ratio was 67% after 24 hours at a pH of 5.0, the residual ratio was 26% after 24 hours at a pH of 7.4, and the residual ratio was 0% after 6 hours at a pH of 9.0.

It was confirmed that Example 6 is very stable at a pH of 2.0, 3.0, and 5.0, and the residual ratio was 17% after 24 hours at a pH of 7.4, and the residual ratio was a value close to 0% after 3 hours at a pH of 9.0.

It was confirmed that Example 8 has sufficient stability at a pH of 2.0 and 3.0. It was confirmed that the residual ratio was 85% after 24 hours at a pH of 5.0, and the residual ratio was a value close to 0% after 3 hours at a pH of 7.4 and 9.0.

It was confirmed that Example 9 is stable at a pH of 2.0, 3.0, and 5.0, the residual ratio was 77% after 24 hours at a pH of 7.4, and the residual ratio was a value close to 0% after 12 hours at a pH of 9.0.

It was confirmed that Example 10 has sufficient stability at a pH of 2.0 and 3.0. It was confirmed that the residual ratio was 19% after 24 hours at a pH of 5.0, and the residual ratio was a value close to 0% immediately after starting the test at a pH of 7.4 and 9.0.

It was confirmed that Example 11 is stable at a pH of 2.0, 3.0, and 5.0, and the residual ratio was 8% after 24 hours at a pH of 7.4, and the residual ratio was a value close to 0% after 3 hours at a pH of 9.0.

It was confirmed that Example 12 is stable at a pH of 2.0 and 3.0, and the residual ratio was 93% after 24 hours at a pH of 5.0, and the residual ratio was 0% after 12 hours at a pH of 7.4.

It was confirmed that Example 13 is stable at a pH of 2.0, 3.0, and 5.0, and the residual ratio was 6% after 24 hours at a pH of 7.4.

It was confirmed from the above results that pH dependent and chemical degradation progressed for the series of compounds of the invention represented by Examples 1, Example 2, Example 4, Reference Example 9, Example 6, Example 8, Example 9, Example 10, Example 11, Example 12, and Example 13, which are prodrugs of ortho-quinones, para-quinones, or polyphenols.

It was also demonstrated by the test results that the series of compounds of the invention can be a prodrug for intravenous administration with sufficient stability by preparing the compounds as an administered liquid with a pH of 2.0 or 3.0. It is confirmed that compounds of the Examples and Reference Examples are converted to a corresponding active form after 24 hours in a buffer with a pH of 7.4.

### [Table 1]

**[Table 1] (Residual ratio% of Example 1)**

| Time (hr) | 0 | 3 | 6 | 9 | 12 | 24 |
|---|---|---|---|---|---|---|
| pH2. 0 | 100 | 100 | 103 | 102 | 102 | 98.6 |
| pH3. 0 | 99.9 | 97.6 | 95.3 | 95.6 | 90.9 | 86.9 |
| pH5. 0 | 85.1 | 6.6 | 3.9 | 0.0 | 0.0 | 0.0 |
| pH7. 4 | 0.0 | 0.0 | 0.0 | 0.0 | 0.0 | 0.0 |
| pH9. 0 | 0.0 | 0.0 | 0.0 | 0.0 | 0.0 | 0.0 |

### [Table 2]

**[Table 2] (Residual ratio% of Example 2)**

| Time (hr) | 0 | 3 | 6 | 9 | 12 | 24 |
|---|---|---|---|---|---|---|
| pH2.0 | 100 | 100 | 99. 8 | 99.7 | 102 | 100 |
| pH3. 0 | 101 | 101 | 101 | 100 | 100 | 101 |
| pH5.0 | 101 | 101 | 102 | 102 | 100 | 102 |
| pH7. 4 | 102 | 50.7 | 32.0 | 16.2 | 0.0 | 0.0 |
| pH9. 0 | 44.5 | 0.0 | 0.0 | 0.0 | 0.0 | 0.0 |

### [Table 3]

**[Table 3] (Residual ratio% of Example 4)**

| **Time** (hr) | 0 | 3 | 6 | 9 | 12 | 24 |
|---|---|---|---|---|---|---|
| pH2. 0 | 100 | 100 | 98.6 | 97.0 | 97.1 | 96.7 |
| pH3. 0 | 100 | 100 | 100 | 100 | 100 | 100 |
| pH5. 0 | 100 | 93.4 | 88.1 | 84.4 | 80.3 | 78.1 |
| pH7. 4 | 30.1 | 0.9 | 0.3 | 0.0 | 0.0 | 0.0 |
| pH9. 0 | 1.3 | 0.2 | 0.4 | 0.4 | 0.3 | 0.4 |

### [Table 4]

**[Table 4] (Residual ratio% of Reference Example 9)**

| Time (hr) | 0 | 3 | 6 | 9 | 12 | 24 |
|---|---|---|---|---|---|---|
| pH2.0 | 100 | 96.9 | 92.9 | 85.7 | 85.2 | N. D. |
| pH3. 0 | 101 | 100 | 100 | 99.8 | 99.5 | 98.4 |
| pH5. 0 | 102 | 104 | 99.7 | 93.8 | 87.4 | 67.1 |
| pH7. 4 | 103 | 83.0 | 67.1 | 52.7 | 41.1 | 25.5 |
| pH9.0 | 75.3 | 13.7 | 0.0 | 0.0 | 0.0 | 0.0 |

| | | | | | | |
|---|---|---|---|---|---|---|
| ***N. D. :Not Determined** | | | | | | |

### [Table 5]

**[Table 5] (Residual ratio% of Example 6)**

| Time (hr) | 0 | 3 | 6 | 9 | 12 | 24 |
|---|---|---|---|---|---|---|
| pH2. 0 | 100 | 100 | 100 | 100 | 100 | 100 |
| pH3. 0 | 100 | 100 | 100 | 100 | 100 | 100 |
| pH5. 0 | 100 | 100 | 99.8 | 99.6 | 99.5 | 99.5 |
| pH7. 4 | 96.8 | 65.8 | 45.2 | 31.4 | 22.2 | 16.5 |
| pH9. 0 | 10.4 | 0.4 | 0.7 | 0.7 | 0.9 | 0.9 |

### [Table 6]

**[Table 6] (Residual ratio% of Example 8)**

| Time (hr) | 0 | 3 | 6 | 9 | 12 | 24 |
|---|---|---|---|---|---|---|
| pH2.0 | 100 | 100 | 100 | 100 | 100 | 100 |
| pH3. 0 | 100 | 100 | 100 | 100 | 100 | 100 |
| pH5. 0 | 100 | 98.2 | 96.1 | 94.4 | 92.4 | 85.2 |
| pH7. 4 | 76.4 | 0.1 | 0.0 | 0.1 | 0.2 | 0.5 |
| pH9.0 | 9.7 | 0.2 | 0.2 | 0.1 | 0.1 | 0.0 |

### [Table 7]

**[Table 7] (Residual ratio% of Example 9)**

| Time (hr) | 0 | 3 | 6 | 9 | 12 | 24 |
|---|---|---|---|---|---|---|
| pH2.0 | 100 | 99.9 | 99.9 | 99.9 | 99.9 | 99.9 |
| pH3.0 | 99.9 | 99.9 | 99.9 | 99.9 | 99.9 | 99.9 |
| pH5. 0 | 100 | 100 | 99.9 | 99.8 | 99.7 | 99.5 |
| pH7. 4 | 99.9 | 96.7 | 93.6 | 90.6 | 87.7 | 76.8 |
| pH9. 0 | 98.4 | 36.2 | 12.3 | 4.1 | 1.3 | 0.0 |

### [Table 8]

**[Table 8] (Residual ratio% of Example 10)**

| Time (hr) | 0 | 3 | 6 | 9 | 12 | 24 |
|---|---|---|---|---|---|---|
| pH2.0 | 100 | 100 | 100 | 100 | 99.7 | 99.8 |
| pH3. 0 | 100 | 100 | 100 | 99.8 | 99.5 | 98. 2 |
| pH5. 0 | 98.1 | 80. 2 | 66.4 | 54.4 | 44.6 | 18.7 |
| pH7. 4 | 0.2 | 0.0 | 0.0 | 0.0 | 0.1 | 0.1 |
| pH9. 0 | 0.1 | 0.1 | 0.1 | 0.1 | 0.1 | 0.0 |

### [Table 9]

**[Table 9] (Residual ratio% of Example 11)**

| Time (hr) | 0 | 3 | 6 | 9 | 12 | 24 |
|---|---|---|---|---|---|---|
| pH2. 0 | 100 | 100 | 100 | 100 | 100 | 100 |
| pH3. 0 | 100 | 100 | 100 | 100 | 100 | 100 |
| pH5. 0 | 100 | 100 | 100 | 99. 9 | 99. 6 | 97.9 |
| pH7. 4 | 97.4 | 73.3 | 55.6 | 41.1 | 30.3 | 7.7 |
| pH9.0 | 19.9 | 0.2 | 0. 2 | 0.1 | 0.0 | 1.6 |

### [Table 9a]

**[Table 9a] (Residual ratio% of Example 12)**

| Time (hr) | 0 | 3 | 6 | 9 | 12 | 24 |
|---|---|---|---|---|---|---|
| pH2. 0 | 100 | 99.4 | 98.9 | 98.7 | 98.5 | 98.5 |
| pH3.0 | 100 | 99.2 | 98.9 | 98.6 | 98.4 | 98.3 |
| pH5. 0 | 100 | 99.1 | 98.1 | 97.1 | 96.4 | 93.3 |
| pH7. 4 | 100 | 9.8 | 1.7 | 1.4 | 0 | 0 |

### [Table 9b]

**[Table 9b] (Residual ratio% of Example 13)**

| Time (hr) | 0 | 3 | 6 | 9 | 12 | 24 |
|---|---|---|---|---|---|---|
| pH2. 0 | 100 | 99.7 | 99.4 | 99.3 | 99.1 | 100 |
| pH3. 0 | 100 | 100 | 100 | 99.8 | 99.6 | 100 |
| pH5. 0 | 100 | 99.4 | 99.2 | 99.0 | 98.6 | 98.4 |
| pH7. 4 | 100 | 61.8 | 42.6 | 28.5 | 19.1 | 6.1 |

### [Table 10]

**[Table 10] (Measurement value %)**

| Rate of generation of active form after 24 hours in buffer with pH of 7.4 | |
|---|---|
| Examples 1 | 92 |
| Example 2 | 7 |
| Example 4 | 37 |
| Reference Example 9 | 71 |
| Example 8 | 88 |
| Example 9 | 23 |
| Example 10 | 96 |
| Reference Example 11 | 91 |
| Example 12 | 11 |
| Example 13 | 5 |

The yield of the Example and Reference Example compounds and active forms is calculated with each of the peak area of the Example and Reference Example compounds at 0 minutes and the peak area upon dissolution of the corresponding active form at 100 umol/L as 100%. The yield is denoted in terms of %.

### Test Example 2. Solubility test

The solubility of the water-soluble prodrugs described in the Examples and the Reference Examples was measured. A water-soluble prodrug or a corresponding active form was added to each 50 mmol/L glycine buffer (pH 2.0, 3.0, or 5.0). Reference Example 9, Example 8, Example 9, Example 10, and Example 11 were shaken for 1 hour at room temperature, and other compounds were shaken for 24 hours at room temperature and then filtered through a membrane filter. The filtrate was measured by HPLC.

HPLC measurement conditions are the following.
HPLC condition
Column: Acquity UPLC BEH C18, 1.7 um, 50 x 2.1 mm
Column temperature: 40°C
Mobile phase:
A: 0.1% trifluoroacetic acid containing water
B: acetonitrile
A/B (min) : 95/5(0) → 0/100 (3.5) → 0/100 (4) → 95/5 (4.01) → 95/5(5)
Flow rate: 0.8 mL/min
Detection: UV visible detector, measured wavelength 254 nm
Injected volume: 1 uL or 2 uL

The test results are shown in Table 11. The series of prodrugs of the invention exhibited much better solubility than the corresponding active forms, revealing that they can be water-soluble prodrugs that are suitable for oral administration and intravenous administration.

### [Table 11]

**[Table 11]**

| Example number or compound name | pH in buffer | Solubility in buffer |
|---|---|---|
| Example 1 | 2.0 | > 10mg/mL |
| 2-acetylthieno[3,2]f][1] benzofuran-4,8-dione | 2.0 | 0.004mg/mL |
| Example 2 | 2.0 | > 10mg/mL |
| 2-methylnaphthalen-1,4-dione | 2.0 | 0.097mg/mL |
| Example 4 | 2.0 | >10mg/mL |
| 2,2-dimethyl-3,4,4a,10b-tetrahydro-2H-benzo[h] chromen-5,6-dione | 2.0 | 0.04mg/mL |
| Reference Example 9 | 3.0 | >10mg/mL |
| 2-acetyl-5-hydroxynaphtho[2,3-b] furan-4,9-dione | 3.0 | 0.001mg/mL |
| Example 6 | 2.0 | >10mg/mL |
| 4,7-diphenyl-1,3-benzodioxol-5,6-dione | 2.0 | 0.0003mg/mL |
| Example 8 | 5.0 | >10mg/mL |
| Example 9 | 5.0 | >10mg/mL |
| Example 10 | 5.0 | >10mg/mL |
| Example 11 | 5.0 | > 10mg/mL |
| Alvocidib | 5.0 | 0.68mg/mL |
| Example 12 | 2.0 | >10mg/mL |
| Example 13 | 2.0 | >10mg/mL |
| (1R)-1,6,6-trimethyl-1,2,6,7,8,9-hexahydrophenanthro[1,2-b] furan-10,11-dione | 2.0 | Below detection limit |

### Test Example 3. Evaluation of conversion rate to active form

The ratio of decrease of a test compound and the conversion ratio to a corresponding active form (2-acetylthieno[3,2-f][1]benzofuran-4,8-dione) in human plasma and CD1 mouse plasma were measured for Example 1.

The test compounds were incubated at 37°C at a concentration of 10 umol/L in human plasma (cat. no. 12250210, Cosmo Bio) and CD1 mouse plasma (cat. no. AP3054, KAC) and retrieved for a given time (0, 5, 10, 30, 60, and 120 minutes) and deproteinized, then analyzed by LC-MS. Acetonitrile comprising each of 10% quantity of 1 mol/L hydrochloric acid, 1% quantity of 46% citric acid, and phenytoin as an internal standard was used for deproteinization.

LC-MS measurement conditions were the following.
LC-MS condition
Column: Acquity UPLC BEH C18, 1.7 um, 50 x 2.1 mm
Column temperature: 40°C
Mobile phase:
A: 0.1% formic acid containing water
B: 0.1% formic acid containing acetonitrile
A/B (min) : 95/5 (0) → 5/95 (3) → 5/95 (5) → 95/5 (5.01) → 95/5 (9)
Flow rate: 0.4 mL/min
Detection: ESI (positive mode)
Injected volume: 10 uL

The results are shown in Table 12. It was confirmed that degradation of the compound of the invention represented by Example 1 quickly progressed under either human plasma or CD1 mouse plasma condition, and the interspecies difference was small. The results indicate that the test compound is a prodrug with a small interspecies difference, which is pH dependently converted to an active form through a route comprising chemical conversion, as confirmed in Test Example 1.

### [Table 12]

**[Table 12] (Measurement value %)**

| **Incubation time (minutes)** | | 0 | 5 | 10 | 30 | 60 | 120 |
|---|---|---|---|---|---|---|---|
| Human plasma | Example 1 | 100 | 0.0 | 0.0 | 0.0 | 0.0 | 0.0 |
| | 2-acetylthieno[3,2-f][1] benzofuran-4,8-dione | 0.0 | 36.4 | 51.6 | 83.4 | 89. 7 | 91.3 |
| CD1 mouse plasma | Example 1 | 100 | 0.11 | 0.01 | 0.01 | 0.01 | 0.08 |
| | 2-acetylthieno[3,2-f][1] benzofuran-4,8-dione | 0.0 | 21.1 | 36.7 | 70.8 | 73.8 | 71.4 |

The yield of the Examples compounds and active form 2-acetylthieno[3,2-f][1]benzofuran-4,8-dione is calculated with each of the peak area of the Example compounds at 0 minutes and the peak area upon dissolution of 2-acetylthieno[3,2-f][1]benzofuran-4,8-dione at 100 umol/L as 100%. The yield is denoted in terms of %.

In the same manner as Example 1, the conversion ratio to an active form was measured for each of the various test compounds in human plasma and CD1 mouse plasma, with incubation time as 120 minutes. The results are shown in Table 13. It was confirmed that the series of compounds of the invention was converted to an active form with a small interspecies difference in human plasma and CD1 mouse plasma. The results indicate that the series of test compounds of the invention are prodrugs with a small interspecies difference, which is pH dependently converted to an active form through a route comprising chemical conversion.

### [Table 13]

**[Table 13] (Measurement value %)**

| Incubation time (120 minutes) | Human plasma | CD1 mouse plasma |
|---|---|---|
| Example 2 | 6 | 3 |
| Example 4 | 45 | 40 |
| Reference Example 9 | 34 | 20 |
| Example 10 | 100 | 94 |
| Reference Example 11 | 100 | 100 |

The yield of the active forms is calculated with the each peak area upon dissolution of the generated active form at 100 umol/L as 100%. The yield is denoted in terms of %.

### Test Example 4. Pharmacokinetics test

The concentration of the active form (2-acetylthieno[3,2-f][1]benzofuran-4,8-dione) in plasma was measured for the water-soluble prodrug described in Example 1. The animal species, dosing method, preparation method of a standard solution, and the plasma concentration measurement method are the following.
Animals: 7-week old male CD1 (ICR) mice were used as the experimental animals.
Dosing method: the test sample was weighed and then dissolved in a 50 mmol/L glycine buffered saline (pH 2.0) as the administered solution. The body weight of mice was measured, and the prepared solution was intravenously administered.
Blood collection method: heparin was added to a blood collection tube, and blood was collected from mice. 1% quantity of 46% citric acid was added to the obtained blood, which was centrifuged to give the plasma. 10% quantity of 1 mol/L HCl was added to the collected plasma as the plasma sample. 10% quantity of 1 mol/L HCl added to blank plasma was used as the blank sample.
Preparation of standard solution: 1 mg of test sample was weighed and dissolved in 10 mL of acetonitrile using a measuring flask to prepare 100 ug/mL of standard solution.
Plasma concentration measurement: 100 ug/mL of standard solution was diluted with an acetonitrile solution to prepare a calibration curve sample with a concentration of interest. 20 uL of blank sample was added to 20 uL of calibration curve sample to prepare a plasma calibration curve sample. 20 uL of acetonitrile solution was added to 20 uL of administered plasma sample to prepare a sample for plasma analysis. 180 uL of internal standard containing acetonitrile (containing 1% quantity of 10 mol/L HCl) was added to each of the plasma calibration curve sample and sample for plasma analysis. Phenytoin was used as the internal standard, and the concentration was 200 nmol/L. 10 uL of each sample was analyzed by LC-MS. Calibration curves were created from the value obtained by dividing the peak area of the test sample by the peak area of an internal standard substance (peak ratio) in MS and the concentration of plasma calibration curve samples. The concentration in the sample was calculated from the peak ratio of each sample and calibration curves.

The test results are shown in Table 14. In the Table, "mean" indicates the mean and "S.E." indicates the standard error. It was confirmed that an active form is quickly generated by intravenous administration of the compound of Example 1.

The series of compounds of the invention can be expected to similarly generate active forms in humans and mice in view of the results of Test Example 1 in addition to this test result. Thus, expanded use of active forms, whose use through parenteral administration is limited, can be expected. Thus, the compounds are very useful.

### [Table 14]

**[Table 14]**

| Plasma | Concentration of compound of Example 1 (umol/L) | | | | |
|---|---|---|---|---|---|
| 2.0mg/kg (i.v.) | 1 | 2 | 3 | mean | S. E. |
| 0.083hr | 3.32 | 2.95 | 2.81 | 3. 03 | 0.15 |
| 0.5hr | 0.53 | 0. 35 | 0.46 | 0.45 | 0.05 |
| 1.0hr | 0.12 | 0.23 | 0.17 | 0.18 | 0.03 |
| 2.0hr | 0.06 | 0.06 | 0.08 | 0.07 | 0.01 |
| 6.0hr | 0.12 | 0.09 | 0.02 | 0.08 | 0.03 |

### [Industrial Applicability]

Pharmaceutical compositions comprising a chemically converted water-soluble prodrug of polyphenols, ortho-quinones, or para-quinones, which are hydrophobic agents, pharmaceutically acceptable salt thereof, or a hydrate or solvate thereof or a liquid formulation comprising them in an aqueous solution exhibit excellent solubility by introducing a carbamate chain or carbonate chain with a specific structure comprising a nucleophilic amine. Furthermore, clinically problematic interspecies and individual differences become small by quickly generating an active form by pH dependent and chemical conversion. Accordingly, the compound of the invention is extremely useful because use of an active form, which has problems in absorption through oral administration such that use through parenteral administration is limited due to the high crystallizability, can be expanded.

## Claims

1. A compound represented by formula (1) 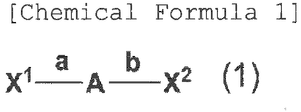 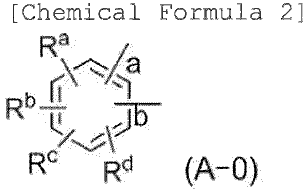 or a pharmaceutically acceptable salt thereof, wherein:
A represents A-0, a and b in the compound of formula (1) are symbols indicating that two bonds binding to A correspond to two bonds in A-0, respectively;
X¹, X², R^{a}, R^{b}, R^{c}, and R^{d} are present on any carbon atom in a random order on the aromatic ring of A-0;
X¹ and X² are the same or different, each independently a hydroxyl group or -O-C(=O)-Y-(C(R^{1A})(R^{1B}))ₙ-NH-R², wherein X¹ and X² are not simultaneously a hydroxyl group;
n is the same or different, each independently 2, 3, or 4;
Y is an oxygen atom or NR⁴;
R^{1A} and R^{1B} are the same or different, each independently a hydrogen atom, -CO₂R⁵, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
R² is a hydrogen atom, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
R^{a}, R^{b}, R^{c}, and R^{d} are the same or different, each independently a hydrogen atom, a hydroxyl group, a halogen atom, a cyano group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₃₋₁₀ cycloalkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 3- to 12-membered monocyclic or polycyclic heterocyclylcarbonyl group, a carboxyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 3- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 3-to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 3- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group, or
2 to 4 of R^{a}, R^{b}, R^{c}, and R^{d}, when adjacent to one another on the aromatic ring, together with 2 to 4 carbon atoms on the aromatic ring to which they are attached, may form 1 or 2 optionally substituted C₃₋₁₈ monocyclic or polycyclic hydrocarbon rings or optionally substituted 3- to 18-membered monocyclic or polycyclic heterocycles, wherein the C₃₋₁₈ monocyclic or polycyclic hydrocarbon ring or the 3- to 18-membered monocyclic or polycyclic heterocycle is fused to the aromatic ring to form a fused ring;
the C₃₋₁₈ monocyclic or polycyclic hydrocarbon ring or the 3- to 18-membered monocyclic or polycyclic heterocycle can have one or more substituents R^{e}, the one or more R^{e} being, each independently, a hydrogen atom, a hydroxyl group, a halogen atom, a cyano group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₃₋₁₀ cycloalkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 3- to 12-membered monocyclic or polycyclic heterocyclylcarbonyl group, a carboxyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 3- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 3-to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 3- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group;
R⁴ is a hydrogen atom, or a C₁₋₆ alkyl group optionally substituted with 1 to 2 substituents selected from the group consisting of a fluorine atom, a carboxyl group, a sulfinate group, a sulfonate group, a phosphate group, a C₆₋₁₀ aryl group, a C₁₋₆ alkoxy group, a C₃₋₈ cycloalkoxy group, -NR⁵R⁶, -CO₂R⁵, - CONR⁵R⁶, -SO₂R⁵, -SO₂NR⁵R⁶, -OCO₂R⁵, -OCONR⁵R⁶, and -NR⁵CO₂R⁶;
R⁵ and R⁶ are the same or different, each independently a hydrogen atom, or a C₁₋₆ alkyl group optionally substituted with 1 to 2 groups selected from the group consisting of a fluorine atom and a carboxyl group, or R⁵ and R⁶, together with the nitrogen atom to which they are attached, may form a 4- to 8-membered cyclic amino group;
wherein A is not 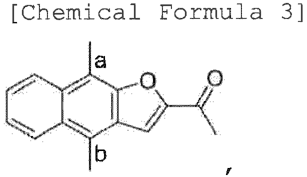 with the proviso that the compound is not the following compounds:
3-hydroxy-2-[(1R,6R)-3-methyl-6-(prop-1-en-2-yl)cyclohex-2-en-1-yl]-5-pentylphenyl (4-aminobutyl)carbamate (1);
2-[(1R,6R)-3-methyl-6-(prop-1-en-2-yl)cyclohex-2-en-1-yl]-5-pentylbenzene-1,3-diyl bis[(4-aminobutyl)carbamate] (2);
(3R)-8-hydroxy-3-[(2R,3S)-3-hydroxy-4-{(2R,4R,6S)-6-[(S)-{[(2S,3S)-2-hydroxy-3-methoxy-5-methylhex-5-enoyl]amino}(methoxy)methyl]-3,3,4-trimethyltetrahydro-2H-pyran-2-yl}butan-2-yl]-5-methyl-1-methylidene-3,4-dihydro-1H-isochromen-6-yl methyl[2-(methylamino)ethyl]carbamate (3);
3-hydroxy-2-methylphenyl (2-aminoethyl)carbamate (4);
3-hydroxy-2-methylphenyl (1-aminopropan-2-yl)carbamate (5);
3-hydroxyphenyl (2-aminoethyl)carbamate (6);
3-hydroxy-2-methylphenyl (2-aminoethyl)methylcarbamate (7);
3-hydroxyphenyl (1-aminopropan-2-yl)carbamate (8);
3-hydroxyphenyl (2-amino-2-methylpropyl)carbamate (9);
3-hydroxyphenyl [2-(methylamino)ethyl]carbamate (10);
3-hydroxyphenyl (1-amino-2-methylpropan-2-yl)carbamate (11);
3-hydroxyphenyl (3-aminopropyl)carbamate (12);
3-hydroxy-2-methylphenyl (2-aminopropyl)carbamate (13);
3-hydroxyphenyl (2-aminopropyl)carbamate (14);
3-hydroxy-2-methylphenyl [2-(methylamino)ethyl]carbamate (15) ;
3-hydroxyphenyl (2-aminoethyl)methylcarbamate (16); and
3-hydroxy-2-methylphenyl (3-aminopropyl)carbamate (17).

2. The compound or a pharmaceutically acceptable salt thereof according to claim 1, wherein
A represents A-1, A-2, A-3, A-4, A-5, A-6, A-7, A-8, A-9, A-10, A-11, A-12, A-13, A-14, A-15, A-16, or A-17: 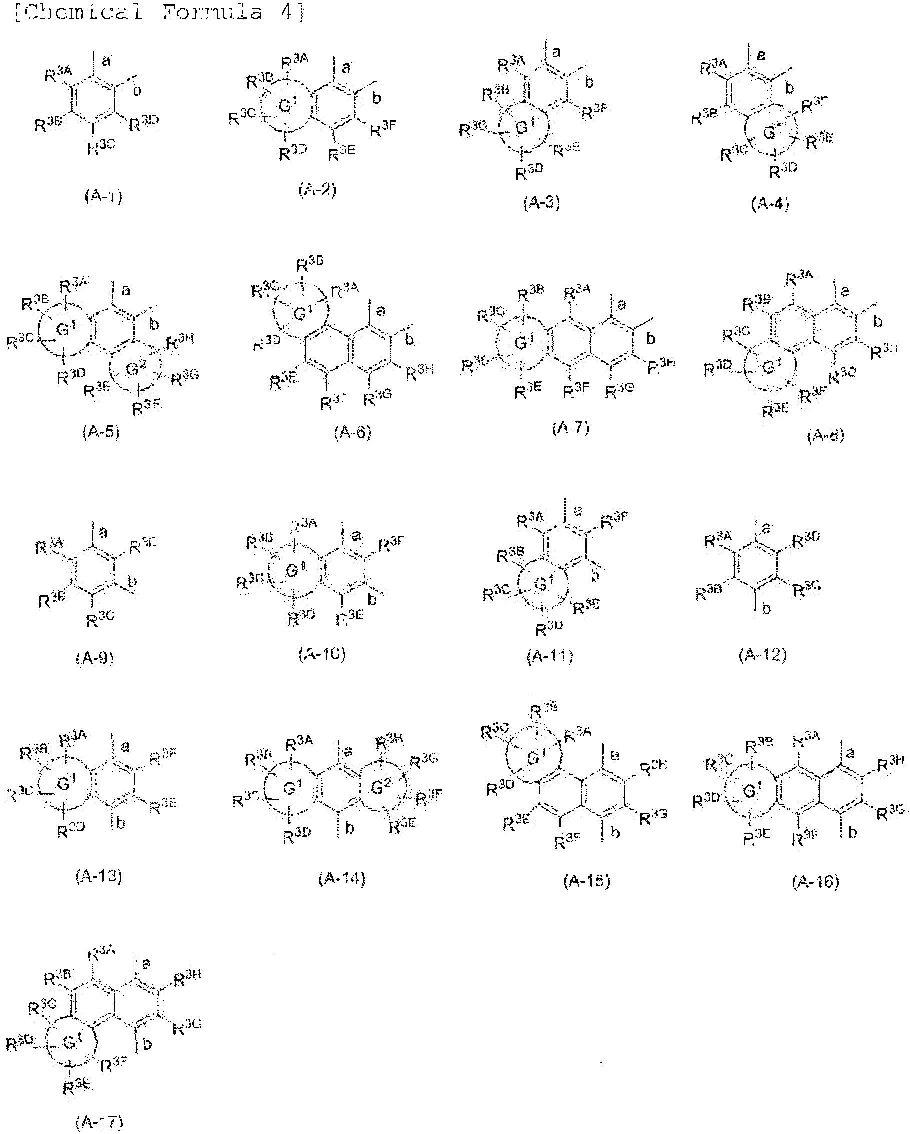
a and b in the compound of formula (1) are symbols indicating that two bonds binding to A correspond to two bonds in A-1 to A-17, respectively;
rings G¹ and G² are the same or different, each independently a benzene ring, or a 5- or 6-membered aromatic ring comprising, as a constituent atom, 1 to 3 heteroatoms consisting of O, S and N; and
R^{3A}, R^{3B}, R^{3C}, R^{3D}, R^{3E}, R^{3F}, R^{3G}, and R^{3H} are null, or the same or different, each independently a hydrogen atom, a hydroxyl group, a fluorine atom, a chlorine atom, a cyano group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₃₋₁₀ cycloalkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 5- to 12-membered monocyclic or polycyclic heterocyclylcarbonyl group, a carboxyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 5- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 5-to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 5- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form an optionally substituted C₃₋₁₀ cycloalkane or an optionally substituted 3-to 12-membered monocyclic or polycyclic heterocycle, wherein the C₃₋₁₀ cycloalkane or the 3- to 12-membered monocyclic or polycyclic heterocycle is fused to the aromatic ring to form a fused ring.

3. The compound or a pharmaceutically acceptable salt thereof according to claim 1 or 2, wherein an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₂₋₂₀ alkynyl group, an optionally substituted C₁₋₂₀ alkoxy group, an optionally substituted C₁₋₂₀ alkylcarbonyloxy group, an optionally substituted C₆₋₁₀ aryl group, an optionally substituted 5- to 10-membered heteroaryl group, an optionally substituted 3- to 12-membered monocyclic or polycyclic heterocyclic group, an optionally substituted C₁₋₂₀ alkylcarbonyl group, an optionally substituted C₃₋₁₀ cycloalkylcarbonyl group, an optionally substituted C₆₋₁₀ arylcarbonyl group, an optionally substituted 5- to 12-membered monocyclic or polycyclic heterocyclylcarbonyl group, an optionally substituted C₁₋₂₀ alkoxycarbonyl group, an optionally substituted C₃₋₁₀ cycloalkyloxycarbonyl group, an optionally substituted C₆₋₁₀ aryloxycarbonyl group, an optionally substituted 5- to 12-membered monocyclic or polycyclic heterocyclyloxycarbonyl group, an optionally substituted aminocarbonyl group, an optionally substituted C₁₋₂₀ alkylthio group, an optionally substituted C₃₋₁₀ cycloalkylthio group, an optionally substituted C₆₋₁₀ arylthio group, an optionally substituted 5- to 12-membered monocyclic or polycyclic heterocyclylthio group, a sulfinate group, an optionally substituted C₁₋₂₀ alkylsulfinyl group, an optionally substituted C₃₋₁₀ cycloalkylsulfinyl group, an optionally substituted C₆₋₁₀ arylsulfinyl group, an optionally substituted 5- to 12-membered monocyclic or polycyclic heterocyclylsulfinyl group, an optionally substituted aminosulfinyl group, a sulfonate group, an optionally substituted C₁₋₂₀ alkylsulfonyl group, an optionally substituted C₃₋₁₀ cycloalkylsulfonyl group, an optionally substituted C₆₋₁₀ arylsulfonyl group, an optionally substituted 5- to 12-membered monocyclic or polycyclic heterocyclylsulfonyl group, or an optionally substituted aminosulfonyl group in R^{3A}, R^{3B}, R^{3C}, R^{3D}, R^{3E}, R^{3F}, R^{3G}, and R^{3H} is each independently a group optionally substituted with the same or different 1 to 5 substituents selected from the group consisting of:
(1) a fluorine atom;
(2) a chlorine atom;
(3) a hydroxyl group;
(4) a carboxyl group;
(5) a sulfinate group;
(6) a sulfonate group;
(7) a phosphate group;
(8) an optionally substituted C₁₋₁₀ alkyl group;
(9) an optionally substituted C₃₋₁₀ cycloalkyl group;
(10) an optionally substituted C₆₋₁₀ aryl group;
(11) an optionally substituted 5- to 10-membered heteroaryl group;
(12) an optionally substituted C₁₋₁₀ alkoxy group;
(13) an optionally substituted C₃₋₁₀ cycloalkoxy group;
(14) an optionally substituted C₁₋₁₀ alkoxycarbonyl group;
(15) an optionally substituted C₁₋₁₀ alkylcarbonyl group;
(16) an optionally substituted 3- to 12-membered monocyclic or polycyclic heterocyclic group;
(17) an optionally substituted 3- to 12-membered cyclic amino group;
(18) -NR⁷R⁸;
(19) -CO₂R⁷;
(20) a guanidine group;
(21) -CONR⁷R⁸;
(22) -SO₂R⁷;
(23) -SO₂NR⁷R⁸;
(24) a cyano group;
(25) -OCO₂R⁷;
(26) -OCONR⁷R⁸;
(27) -NR⁷CO₂R⁸; and
(28) a triphenylphosphonium cation,
wherein the substituents in (8), (9), (10), (11), (12), (13), (14), (15), (16), and (17) are each independently a group optionally substituted with the same or different 1 to 5 substituents selected from the group consisting of:
(a) a fluorine atom;
(b) a chlorine atom;
(c) a hydroxyl group;
(d) a C₁₋₆ alkyl group;
(e) a C₁₋₆ alkoxy group;
(f) a cyano group;
(g) a carboxyl group;
(h) a sulfinate group;
(i) a sulfonate group;
(j) a phosphate group;
(k) a C₁₋₆ alkoxycarbonyl group;
(l) a C₁₋₆ alkylcarbonyl group;
(m) -NR⁷R⁸;
(n) -CO₂R⁷;
(o) a guanidine group;
(p) -CONR⁷R⁸;
(q) -SO₂R⁷;
(r) -SO₂NR⁷R⁸;
(s) a C₆₋₁₀ aryl group;
(t) a 5- to 10-membered heteroaryl group;
(u) a 3- to 12-membered cyclic amino group optionally substituted with 1 to 3 C₁₋₆ alkyl groups; and
(v) a 3- to 12-membered monocyclic or polycyclic heterocyclic group optionally substituted with 1 to 3 C₁₋₆ alkyl groups,
wherein R⁷ and R⁸ are the same or different, each independently a hydrogen atom, or a C₁₋₆ alkyl group optionally substituted with 1 to 2 groups selected from the group consisting of a fluorine atom and a carboxyl group, or R⁷ and R⁸, together with the nitrogen atom to which they are attached, may form a 4- to 8-membered cyclic amino group.

4. The compound or a pharmaceutically acceptable salt thereof according to any one of claims 1 to 3, wherein
R^{3A}, R^{3B}, R^{3C}, R^{3D}, R^{3E}, R^{3F}, R^{3G}, and R^{3H} are null, or the same or different, each independently a hydrogen atom, a hydroxyl group, an optionally substituted amino group, an optionally substituted 3- to 12-membered cyclic amino group, an optionally substituted C₁₋₃₀ alkyl group, an optionally substituted C₃₋₁₀ cycloalkyl group, an optionally substituted C₂₋₃₀ alkenyl group, an optionally substituted C₁₋₂₀ alkoxy group, an optionally substituted C₁₋₂₀ alkylcarbonyloxy group, an optionally substituted 3- to 12-membered monocyclic or polycyclic heterocyclic group, an optionally substituted C₆₋₁₀ aryl group, an optionally substituted C₁₋₂₀ alkylcarbonyl group, or a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form an optionally substituted C₃₋₁₀ cycloalkane or an optionally substituted 3-to 12-membered monocyclic or polycyclic heterocycle, wherein the C₃₋₁₀ cycloalkane or the 3- to 12-membered monocyclic or polycyclic heterocycle is fused to the aromatic ring to form a fused ring.

5. The compound or a pharmaceutically acceptable salt thereof according to any one of claims 1 to 4, wherein R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) an optionally substituted 3- to 12-membered cyclic amino group, wherein the cyclic amino group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, and a 5- to 10-membered heteroaryl group;
(4) an optionally substituted C₁₋₃₀ alkyl group, wherein the alkyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(5) an optionally substituted C₃₋₁₀ cycloalkyl group, wherein the cycloalkyl group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(6) an optionally substituted C₂₋₃₀ alkenyl group, wherein the alkenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
(7) an optionally substituted C₁₋₂₀ alkoxy group, wherein the alkoxy group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, and a C₁₋₁₀ alkoxycarbonyl group;
(8) an optionally substituted C₁₋₂₀ alkylcarbonyloxy group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(9) an optionally substituted 3- to 12-membered monocyclic or polycyclic heterocyclic group, wherein the heterocyclic group is optionally substituted with 1 to 3 groups selected from the group consisting of a chlorine atom, a hydroxyl group, a carboxyl group, a C₁₋₁₀ alkyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(10) an optionally substituted phenyl group, wherein the phenyl group is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation;
(11) an optionally substituted C₁₋₂₀ alkylcarbonyl group, wherein the alkyl is optionally substituted with 1 to 3 groups selected from the group consisting of a chlorine atom, a hydroxyl group, a carboxyl group, a C₃₋₁₀ cycloalkyl group, an optionally chloro-substituted C₆₋₁₀ aryl group, a 5- to 10-membered heteroaryl group, a C₁₋₁₀ alkoxycarbonyl group, and a triphenylphosphonium cation; or
(12) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₃₋₁₀ cycloalkane, wherein the cycloalkane is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation; or
(2') an optionally substituted 3- to 12-membered monocyclic or polycyclic heterocycle, wherein the heterocycle is optionally substituted with 1 to 3 groups selected from the group consisting of a chlorine atom, a hydroxyl group, a carboxyl group, a C₁₋₆ alkyl group, a C₃₋₁₀ cycloalkyl group, an optionally chloro-substituted C₆₋₁₀ aryl group, a 5- to 10-membered heteroaryl group, a C₁₋₆ alkoxycarbonyl group, and a triphenylphosphonium cation;
wherein the C₃₋₁₀ cycloalkane or the 3- to 12-membered monocyclic or polycyclic heterocycle is fused to the aromatic ring to form a fused ring.

6. The compound or a pharmaceutically acceptable salt thereof according to any one of claims 1 to 5, wherein R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) an optionally substituted C₁₋₃₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
(5) an optionally substituted C₂₋₃₀ alkenyl group, wherein the alkenyl group is optionally substituted with 1 to 3 groups selected from the group consisting of a carboxyl group and a pyridyl group;
(6) a C₁₋₁₂ alkoxy group;
(7) a C₁₋₁₂ alkylcarbonyloxy group;
(8) a 5- to 8-membered monocyclic or polycyclic heterocyclic group, wherein the heterocyclic group is optionally substituted with 1 to 3 groups selected from the group consisting of a hydroxyl group and a C₁₋₁₀ alkyl group;
(9) a phenyl group;
(10) a C₁₋₁₂ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₅₋₈ cycloalkane, wherein the cycloalkane is optionally substituted with 1 to 3 groups selected from the group consisting of a C₁₋₆ alkyl group and an optionally chloro-substituted C₆₋₁₀ aryl group; or
(2') an optionally substituted 5- to 8-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group,
wherein the C₃₋₁₀ cycloalkane or the 3- to 12-membered monocyclic or polycyclic heterocycle is fused to the aromatic ring to form a fused ring.

7. The compound or a pharmaceutically acceptable salt thereof according to any one of claims 2 to 6, wherein A is A-1, A-2, A-3, A-4, A-9, A-12, A-13, A-14, A-15, A-16, or A-17.

8. The compound or a pharmaceutically acceptable salt thereof according to any one of claims 2 to 7, wherein A is A-1, A-2, A-4, A-9, A-12, A-13, or A-14.

9. The compound or a pharmaceutically acceptable salt thereof according to any one of claims 1 to 8, wherein R^{1A} and R^{1B} are the same or different, each independently a hydrogen atom, -CO₂R⁵, or a C₁₋₆ alkyl group optionally substituted with 1 to 2 substituents selected from the group consisting of a carboxyl group, -CO₂R⁵, -CONR⁵R⁶, -SO₂R⁵, -SO₂NR⁵R⁶, -OCO₂R⁵, - OCONR⁵R⁶, and -NR⁵CO₂R⁶.

10. The compound or a pharmaceutically acceptable salt thereof according to any one of claims 2 to 9, wherein rings G¹ and G² are the same or different, each independently a benzene ring, a furan ring, or a thiophene ring.

11. The compound or a pharmaceutically acceptable salt thereof according to any one of claims 1 to 10, wherein R² is a hydrogen atom, or a C₁₋₆ alkyl group optionally substituted with 1 to 2 substituents selected from the group consisting of a carboxyl group, -CO₂R⁵, -CONR⁵R⁶, -SO₂R⁵, -SO₂NR⁵R⁶, -OCO₂R⁵, -OCONR⁵R⁶, and -NR⁵CO₂R⁶.

12. The compound or a pharmaceutically acceptable salt thereof according to any one of claims 1 to 11, wherein R⁴ is a hydrogen atom, or a C₁₋₆ alkyl group optionally substituted with 1 to 2 substituents selected from the group consisting of a carboxyl group, a C₁₋₆ alkoxy group, -NR⁵R⁶, -CO₂R⁵, - CONR⁵R⁶, -OCO₂R⁵, -OCONR⁵R⁶, and -NR⁵CO₂R⁶.

13. The compound or a pharmaceutically acceptable salt thereof according to any one of claims 1 to 12, wherein R⁴ is a hydrogen atom or a C₁₋₆ alkyl group.

14. The compound or a pharmaceutically acceptable salt thereof according to any one of claims 1 to 13, wherein R⁵ and R⁶ are the same or different, each independently a hydrogen atom, or a C₁₋₆ alkyl group optionally substituted with 1 to 2 carboxyl groups, or R⁵ and R⁶, together with the nitrogen atom to which they are attached, may form a 4- to 8-membered cyclic amino group.

15. The compound or a pharmaceutically acceptable salt thereof according to claim 2, wherein
A is A-1, A-2, A-4, A-9, A-12, A-13, or A-14;
X¹ and X² are the same or different, each independently a hydroxyl group, or -O-C(=O)-Y-(C(R^{1A})(R^{1B}))ₙ-NH-R², wherein X¹ and X² are not simultaneously a hydroxyl group;
n is the same or different, each independently 2, 3, or 4;
Y is an oxygen atom or NR⁴;
rings G¹ and G² are the same or different, each independently a benzene ring, a furan ring, or a thiophene ring;
R^{1A} and R^{1B} are the same or different, each independently a hydrogen atom, -CO₂R⁵, or a C₁₋₆ alkyl group optionally substituted with 1 to 2 substituents selected from the group consisting of a carboxyl group, -CO₂R⁵, -CONR⁵R⁶, and -SO₂NR⁵R⁶;
R² is a hydrogen atom, or a C₁₋₆ alkyl group optionally substituted with 1 to 2 substituents selected from the group consisting of a carboxyl group, -CO₂R⁵, -CONR⁵R⁶, -SO₂R⁵, - SO₂NR⁵R⁶, -OCO₂R⁵, -OCONR⁵R⁶, and -NR⁵CO₂R⁶;
R^{3A}, R^{3B}, R^{3C}, R^{3D}, R^{3E}, R^{3F}, R^{3G}, and R^{3H} are null, or the same or different, each independently:
(1) a hydrogen atom,
(2) a hydroxyl group,
(3) an optionally substituted C₁₋₂₀ alkyl group, wherein the alkyl group is optionally substituted with 1 to 3 groups selected from the group consisting of a hydroxyl group, a carboxyl group, an optionally chloro-substituted phenyl group, a pyridyl group, a C₁₋₆ alkoxycarbonyl group, and a triphenylphosphonium cation;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group;
(5) an optionally substituted C₂₋₂₀ alkenyl group, wherein the alkenyl group is optionally substituted with 1 to 3 groups selected from the group consisting of a carboxyl group and a pyridyl group;
(6) a C₁₋₁₂ alkoxy group;
(7) a C₁₋₁₂ alkylcarbonyloxy group;
(8) a 5- to 6-membered monocyclic or polycyclic heterocyclic group, wherein the heterocyclic group is optionally substituted with 1 to 3 groups selected from the group consisting of a hydroxyl group and a C₁₋₁₀ alkyl group;
(9) a phenyl group;
(10) a C₁₋₁₂ alkylcarbonyl group; or
(11) a carboxyl group, or
R^{3A} and R^{3B}, R^{3B} and R^{3C}, R^{3C} and R^{3D}, R^{3D} and R^{3E}, R^{3E} and R^{3F}, R^{3F} and R^{3G}, or R^{3G} and R^{3H}, when adjacent to each other on an aromatic ring, together with 2 carbon atoms on the aromatic ring to which they are attached, may form:
(1') an optionally substituted C₅₋₆ cycloalkane, wherein the cycloalkane is optionally substituted with 1 to 3 groups selected from the group consisting of a C₁₋₆ alkyl group and an optionally chloro-substituted C₆₋₁₀ aryl group; or
(2') an optionally substituted 5- to 6-membered monocyclic or polycyclic heterocycle, wherein the heterocycle is optionally substituted with 1 to 3 groups selected from the group consisting of a C₁₋₆ alkyl group and an optionally chloro-substituted C₆₋₁₀ aryl group,
wherein the C₃₋₁₀ cycloalkane or the 3- to 12-membered monocyclic or polycyclic heterocycle is fused to the aromatic ring to form a fused ring;
R⁴ is a hydrogen atom or a C₁₋₄ alkyl group; and
R⁵ and R⁶ are the same or different, each independently a hydrogen atom or a C₁₋₆ alkyl group, or R⁵ and R⁶, together with the nitrogen atom to which they are attached, may form a 4- to 8-membered cyclic amino group.

16. The compound or a pharmaceutically acceptable salt thereof according to any one of claims 1 to 15, wherein R^{1A} and R^{1B} are the same or different, each independently a hydrogen atom, -CO₂R⁵, or a C₁₋₆ alkyl group optionally substituted with 1 to 2 substituents selected from the group consisting of a carboxyl group and -CO₂R⁵.

17. The compound or a pharmaceutically acceptable salt thereof according to any one of claims 1 to 16, wherein R^{1A} and R^{1B} are hydrogen atoms.

18. The compound or a pharmaceutically acceptable salt thereof according to any one of claims 1 to 17, wherein R² is a hydrogen atom, or a C₁₋₆ alkyl group optionally substituted with 1 to 2 substituents selected from the group consisting of a carboxyl group, -CO₂R⁵, -CONR⁵R⁶, and -SO₂NR⁵R⁶.

19. The compound or a pharmaceutically acceptable salt thereof according to any one of claims 1 to 18, wherein R² is a hydrogen atom, or a C₁₋₆ alkyl group optionally substituted with 1 to 2 carboxyl groups.

20. The compound or a pharmaceutically acceptable salt thereof according to any one of claims 1 to 19, wherein Y is an oxygen atom.

21. The compound or a pharmaceutically acceptable salt thereof according to any one of claims 1 to 19, wherein Y is NR⁴.

22. The compound or a pharmaceutically acceptable salt thereof according to any one of claims 1 to 21, wherein R⁴ is a C₁₋₄ alkyl group.

23. The compound or a pharmaceutically acceptable salt thereof according to any one of claims 1 to 21, wherein R⁴ is a hydrogen atom.

24. The compound or a pharmaceutically acceptable salt thereof according to any one of claims 1 to 23, wherein R⁵ and R⁶ are the same or different, each independently a hydrogen atom or a C₁₋₆ alkyl group.

25. The compound or a pharmaceutically acceptable salt thereof according to any one of claims 1 to 24, wherein X¹ and X² are -O-C(=O)-Y-(C(R^{1A})(R^{1B}))ₙ-NH-R².

26. The compound or a pharmaceutically acceptable salt thereof according to any one of claims 1 to 24, wherein one of X¹ and X² is -O-C(=O)-Y-(C(R^{1A})(R^{1B}))ₙ-NH-R², and the other is a hydroxyl group.

27. The compound or a pharmaceutically acceptable salt thereof according to any one of claims 1 to 26, wherein n is 2.

28. The compound or a pharmaceutically acceptable salt thereof according to any one of claims 1 to 26, wherein n is 3.

29. The compound or a pharmaceutically acceptable salt thereof according to any one of claims 2 to 28, wherein
A is A-13;
ring G¹ is a benzene ring; and
R^{3A}, R^{3B}, R^{3C}, R^{3D}, R^{3E}, and R^{3F} are the same or different, each independently:
(1) a hydrogen atom;
(2) a hydroxyl group;
(3) a C₁₋₂₀ alkyl group;
(4) an optionally substituted C₃₋₁₀ cycloalkyl group, wherein the cycloalkyl group is optionally substituted with 1 to 3 groups selected from the group consisting of a C₃₋₁₀ cycloalkyl group and an optionally chloro-substituted phenyl group; or
(5) a C₂₋₂₀ alkenyl group.

30. The compound or a pharmaceutically acceptable salt thereof according to any one of claims 2 to 28, wherein
A is A-12; and
R^{3A}, R^{3B}, R^{3C}, and R^{3D} are the same or different, each independently:
(1) a hydrogen atom;
(2) an optionally substituted C₁₋₁₂ alkyl group, wherein the alkyl group is optionally substituted with 1 to 3 groups selected from the group consisting of a hydroxyl group, a carboxyl group, a phenyl group, a pyridyl group, a C₁₋₆ alkoxycarbonyl group, and a triphenylphosphonium cation;
(3) an optionally substituted C₂₋₂₀ alkenyl group, wherein the alkenyl group is optionally substituted with 1 to 3 groups selected from the group consisting of a carboxyl group and a pyridyl group;
(4) a C₁₋₆ alkoxy group; or
(5) a phenyl group.

31. The compound or a pharmaceutically acceptable salt thereof according to any one of claims 2 to 28, wherein
A is A-14;
rings G¹ and G² are the same or different, each independently a benzene ring, a furan ring, or a thiophene ring; and
R^{3A}, R^{3B}, R^{3C}, R^{3D}, R^{3E}, R^{3F}, R^{3G}, and R^{3H} are null, or the same or different, each independently:
(1) a hydrogen atom;
(2) a hydroxyl group;
(3) a C₁₋₆ alkylcarbonyloxy group;
(4) a C₁₋₆ alkylcarbonyl group; or
(5) a carboxyl group.

32. The compound or a pharmaceutically acceptable salt thereof according to any one of claims 2 to 28, wherein
A is A-2 or A-4;
ring G¹ is a benzene ring; and
R^{3A}, R^{3B}, R^{3C}, R^{3D}, R^{3E}, and R^{3F} are the same or different, each independently:
(1) a hydrogen atom;
(2) a C₁₋₆ alkyl group; or
(3) a C₁₋₆ alkoxy group, or,
R^{3A} and R^{3B}, R^{3B} and R^{3C}, R^{3C} and R^{3D}, R^{3D} and R^{3E}, or R^{3E} and R^{3F}, when adjacent to each other on an aromatic ring, together with 2 carbon atoms on the aromatic ring to which they are attached, may form:
(1') cyclohexane optionally substituted with 1 to 3 C₁₋₆ alkyl groups;
(2') tetrahydrofuran optionally substituted with 1 to 3 C₁₋₆ alkyl groups; or
(3') tetrahydropyran optionally substituted with 1 to 3 C₁₋₆ alkyl groups,
wherein the cyclohexane, the tetrahydrofuran, or the tetrahydropyran is fused to the aromatic ring to form a structure comprising

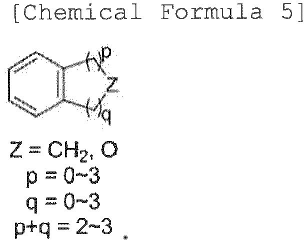

33. The compound or a pharmaceutically acceptable salt thereof according to any one of claims 2 to 28, wherein
A is A-9; and
R^{3A}, R^{3B}, R^{3C}, and R^{3D} are the same or different, each independently:
(1) a hydrogen atom; or
(2) a 3- to 12-membered monocyclic or polycyclic heterocyclic group, wherein the heterocyclic group is optionally substituted with 1 to 3 groups selected from the group consisting of a hydroxyl group and a C₁₋₁₀ alkyl group, or
R^{3A} and R^{3B} or R^{3B} and R^{3C}, together with 2 carbon atoms on the aromatic ring to which they are attached, may form a dihydropyranone ring optionally substituted with an optionally chloro-substituted C₆₋₁₀ phenyl group, wherein the dihydropyranone ring is fused to the aromatic ring to form a structure comprising

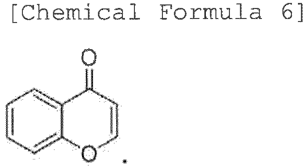

34. The compound or a pharmaceutically acceptable salt thereof according to claim 1 or 2, wherein
A is the following structure: 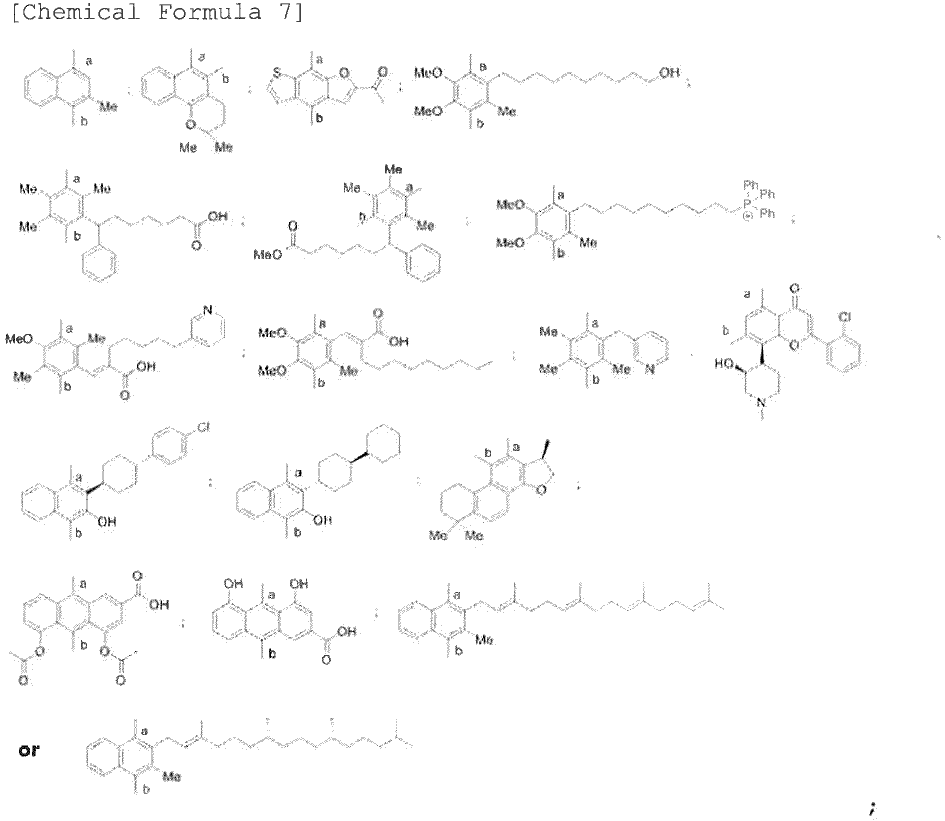
X¹ and X² are the same or different, each independently a hydroxyl group or -O-C(=O)-Y-(C(R^{1A})(R^{1B}))ₙ-NH-R², wherein X¹ and X² are not simultaneously a hydroxyl group;
n is the same or different, each independently 2, 3, or 4;
Y is an oxygen atom or NR⁴;
R^{1A} and R^{1B} are a hydrogen atom or -CO₂R⁵;
R² is a hydrogen atom, or a C₁₋₆ alkyl group optionally substituted with 1 to 2 carboxyl groups;
R⁴ is a hydrogen atom or a C₁₋₄ alkyl group; and
R⁵ is a hydrogen atom, or a C₁₋₆ alkyl group optionally substituted with 1 to 2 groups selected from the group consisting of a fluorine atom and a carboxyl group.

35. The compound or a pharmaceutically acceptable salt thereof according to claim 34, wherein A is the following structure:

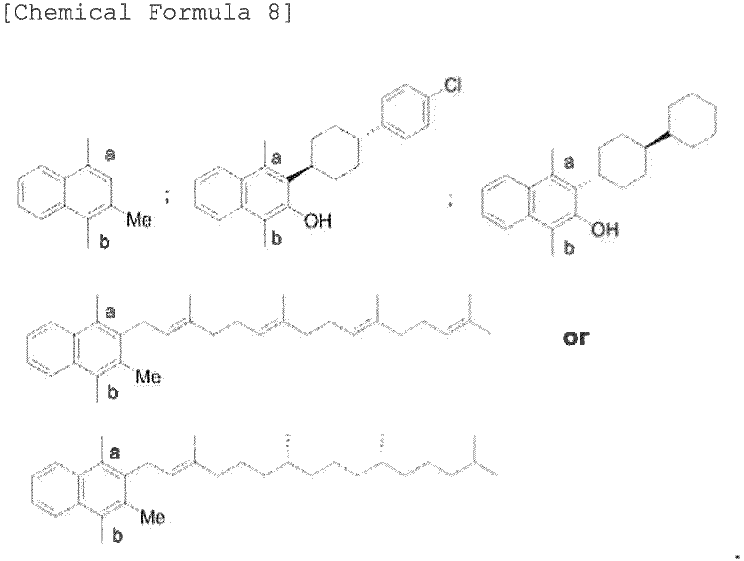

36. The compound or a pharmaceutically acceptable salt thereof according to claim 34, wherein A is the following structure:

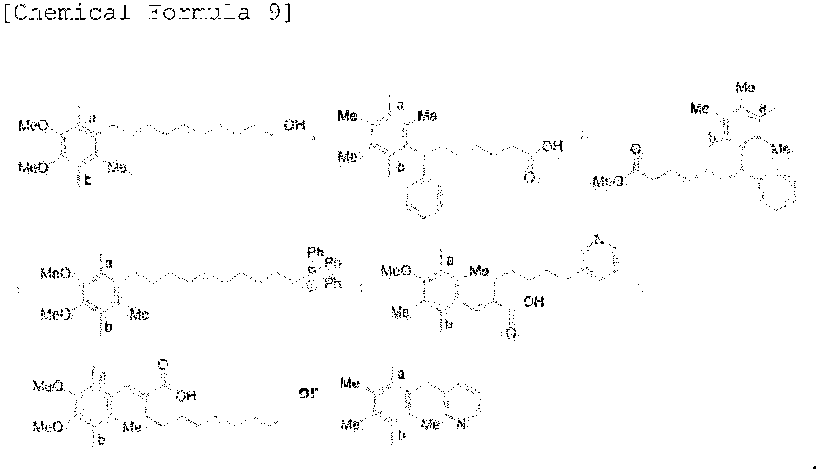

37. The compound or a pharmaceutically acceptable salt thereof according to claim 34, wherein A is the following structure:

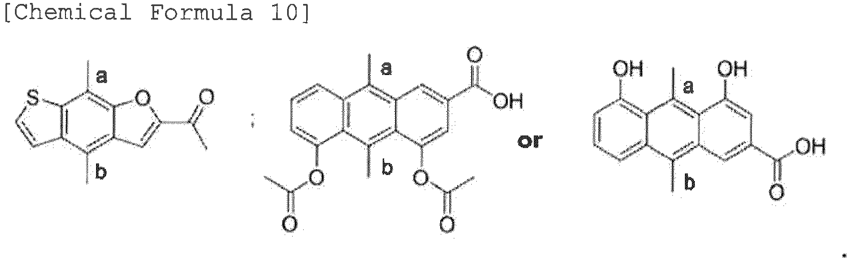

38. The compound or a pharmaceutically acceptable salt thereof according to claim 34, wherein A is the following structure:

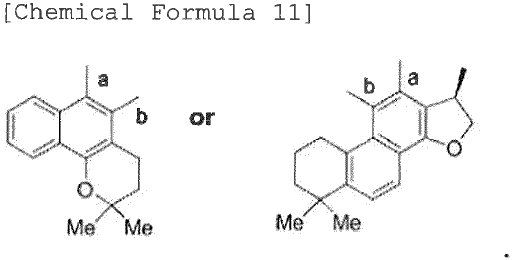

39. The compound or a pharmaceutically acceptable salt thereof according to claim 34, wherein A is the following structure:

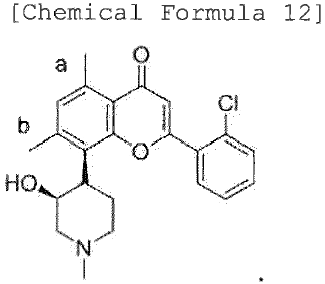

40. A pharmaceutical composition comprising the compound or a pharmaceutically acceptable salt thereof according to any one of claims 1 to 39.

41. A therapeutic agent or a preventive agent for cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes, comprising the compound or a pharmaceutically acceptable salt thereof according to any one of claims 1 to 39 as an active ingredient, or the pharmaceutical composition according to claim 40.

42. A method for treating and/or preventing cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes, **characterized by** administering, to a patient in need thereof, a therapeutically and/or preventively effective amount of the compound or a pharmaceutically acceptable salt thereof according to any one of claims 1 to 39 or the pharmaceutical composition according to claim 40.

43. Use of the compound or a pharmaceutically acceptable salt thereof according to any one of claims 1 to 39 or the pharmaceutical composition according to claim 40 for the manufacture of a therapeutic agent and/or a preventive agent for cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes.

44. The compound or a pharmaceutically acceptable salt thereof according to any one of claims 1 to 39 or the pharmaceutical composition according to claim 40 for use in the treatment and/or prevention of cancer, allergy, dementia, muscular dystrophy, demyelinating disease, protozoal infection, heart failure, hypertension, liver disease, bullous disease, thrombus, hemorrhage, vitamin deficiency, osteoporosis, obesity, central nervous system disease, arthritis, kidney disease, inflammation, and diabetes.
